# EUROPEAN PATENT SPECIFICATION

(11) **EP 1 443 329 B1**
(45) Date of publication and mention of the grant of the patent: **15.09.2010**
(21) Application number: 04010960.5
(22) Date of filing: 22.09.1994
(51) Int. Cl.: G01N 35/02, G01N 33/53

(54) **A method for simultaneously performing a plurality of assays**
Verfahren zum gleichzeitigen Durchführen von mehreren Assays
Méthode d'effectuer simultanément plusieurs essais biologiques

(30) Priority: 24.09.1993 US 126411
(43) Date of publication of application: 04.08.2004
(62) Divisional of application: 02001046.8
(73) Proprietor: ABBOTT LABORATORIES, Abbott Park IL 60064-3500 (US)
(72) Inventor: Clark, Frederick L., Plano TX 75023 (US); Clemens, John M., Wadsworth IL 60083 (US); Hance, Robert B., Evanston IL 60202 (US); Hendrick, Kendall B., Southlake TX 76092 (US); Tayi, Apparao, Grayslake IL 60030 (US); Kanewske, William J. III, Dallas TX 75208 (US); Lagocki, Peter A., Plano, TX 75075 (US); Martin, Richard R., Irving TX 75063 (US); McDowell, Douglas D., Wildwood IL 60030 (US); Merriam, Richard A., Dallas TX 75218 (US); Moore, Larry W., Plano TX 75075 (US); Oleksak, Carl M., Fort Worth TX 76118 (US); Pennington, Charles D., Lake Zurich IL 60061 (US); Raymoure, William J., Lake Bluff IL 60044 (US); Rumbaugh, William D., Carrollton TX 75006 (US); Schmidt, Linda S., Colorado Springs, CO 80918 (US); Schrier, Paul R., Carollton TX 75007 (US); Smith, Jane B., Vernon Hills IL 60061 (US); Spronk, Adrian M., Lindenhurst IL 60046 (US); Walker, Edna S., Chicago IL 60618 (US); Vaught, James A., Euless TX 76039 (US); Vickstrom, Richard L., Algonquin IL 60102 (US); Walker, Donny Ray, Coppell TX 75019 (US); Watkins, William E. III, Cedar Hill TX 75104 (US); Winter, Gary E., Hanover Park IL 60103 (US); Wohlford, Robert A., Irving TX 75062 (US); Clift, Gilbert, Mesquite TX 75150 (US); Cloonan, Kevin M., Round Lake IL 60073 (US); Mitchell, James E., Lake Barrington IL 60010 (US); Stanton, Alyn K., Barrington IL 60010 (US); Yost, David A., Poolesville MD 20837 (US); Hills, David B., Plano TX 75025 (US)
(74) Representative: Modiano, Micaela Nadia

(56) References cited:
- EP-A- 0 517 477
- WO-A-93/23732
- US-A- 4 678 752
- HIROSHI MITSUMAKI ET AL: "SMALL SIZED AUTOMATED BLOOD ANALYZER CAPABLE OF QUICK RESPONSE TO MULTIPLE APPLICATIONS" HITACHI REVIEW, HITACHI LTD. TOKYO, JP, vol. 41, no. 4, 1 September 1992 (1992-09-01), pages 161-166, XP000334655 ISSN: 0018-277X
- CORKAN L A ET AL: "EXPERIMENT MANAGER SOFTWARE FOR AN AUTOMATED CHEMISTRY WORKSTATION, INCLUDING A SCHEDULER FOR PARALLEL EXPERIMENTATION" LABORATORY AUTOMATION & INFORMATION MANAGEMENT, ELSEVIER SCIENCE PUBLISHERS BV., AMSTERDAM, NL, vol. 17, no. 1, 1 October 1992 (1992-10-01), pages 47-74, XP000321830 ISSN: 1381-141X

## Description

HIROSHI MITSUMAKI ET AL: "SMALL SIZED AUTOMATED BLOOD ANALYZER CAPABLE OF QUICK RESPONSE TO MULTIPLE APPLICATIONS" HITACHI REVIEW, HITACHI LTD. TOKYO, JP, vol, 41, no. 4, 1 September 1992 (1992-09-01), pages 161-166, XP000334655 ISSN: 0018-277X teaches an analyzer wherein the reaction time may be shortened a priori for minimizing data report time.

### Field of the Invention

The present invention relates to a method for simultaneously performing at least two assays as defined in independent claim 1.

Further advantageous aspects of the invention are set out in the dependent claims.

### Background of the Invention

### The Automated Analytical System

Although various known clinical analyzers for chemical, immunochemical and biological testing of samples are available, clinical technology is rapidly changing due to increasing demands in the clinical laboratory to provide new levels of service. These new levels of service must be more cost effective to decrease the operating expenditures such as labor cost and the like, and must provide shorter turnaround time of test results to reduce the patient's length of stay in the hospital as well as improve efficiency of outpatient treatment. Modernization of analytical apparatus and procedures demands consolidation of work stations to meet the growing challenge placed on clinical laboratories.

Generally, analysis of a test sample involves the reaction of test samples with one or more reagents with respect to one or more analytes wherein it is frequently desired that the analysis be performed on a selective basis with respect to each test sample. However, due to the high demands placed on clinical laboratories regarding not only volume throughput but also the number and frequency of various analyses, there is a need to provide an automated analysis system which is capable of combining accurate analytical results, multiple test menu versatility, low reagent and fluids loss and consumption, and of great benefit and importance, continuous and high throughput.

The present automated clinical analysis systems provide much improved accuracy of analytical results in comparison with accuracies of earlier systems. In the present systems, analysis of a test sample typically involves forming a reaction mixture comprising the test sample and one or more reagents, and the reaction mixture is then analyzed by an apparatus for one or more characteristics of the test sample. Reliance on automated clinical analyzers has improved the efficiency of the laboratory procedures, inasmuch as the technician has fewer tasks to perform. Automated clinical analyzers provide results much more rapidly while frequently avoiding operator or technician error, thus placing emphasis on accuracy and repeatability of a variety of tests. Automated clinical analyzers presently available for routine laboratory tests include a transport or conveyor system designed to transport containers of sample liquids between various operating stations. For example, a reaction tube or cuvette containing a test sample may pass through a reagent filling station, mixing station, reaction forming station, detection stations, analysis stations, and the like. Such present transport systems are, however, not flexible in that transport is in one direction and the reaction tubes or cuvettes, once inserted into the apparatus, must pass through without access before analysis occurs. Even further, the present transport systems allow only batch-like operation in that once the system is initially loaded, testing may only be performed on the initially loaded samples during a single operation cycle; alternative or additional samples can not be loaded during the operation cycle to allow continuing operations for extended periods.

As for multiple test menu versatility, some of the presently available automated clinical analyzers, such as automated immunoassay analyzers like the Abbott IMx^{®} analyzer and the Abbott TDx^{®} analyzer (Abbott Laboratories, Abbott Park, Illinois, USA), utilize procedures involving a variety of different assay steps. These present systems have typically relied on detection and measurement of optical changes in a reaction mixture during the assay process. For example, a number of well-known techniques using single or multi-wavelength fluorescence include fluorescent polarization immunoassays (FPIA) employing homogeneous immunoassay techniques, microparticle enzyme immunoassays (MEIA) employing heterogeneous immunoassay techniques, and the like. The MEIA technology, such as that used on the Abbott IMx^{®} analyzer, is used for high and low molecular weight analytes requiring greater sensitivity, and FPIA technology, such as that used on the Abbott TDx^{®} analyzer, is used primarily for lower molecular weight analytes. A front surface fluorometer is used in these systems to quantify a fluorescent product generated in the MEIA assays, while a fluorescence polarization optical system is used to quantify the degree of tracer binding to antibody in the FPIA assays. The test samples are automatically processed in certain of these systems, such as the Abbott IMx^{®} analyzer and Abbott TDx^{®} analyzer, by a robotic arm with a pipetting probe and a rotating carousel which positions the samples for processing. These systems are typically compact table-top analyzers which offer fully automated, walk-away immunoassay testing capabilities for both routine and specialized immunoassays. These nonisotopic methods eliminate radioactivity disposal problems and increase reagent shelf life while meeting the diverse requirements of a multitude of different assays. Though these presently available automated clinical analyzers provide a degree of improved multiple test menu versatility in comparison to earlier systems and practices, the present analyzers remain limited in that these systems are one direction only systems, or batch analyzers, which permit the analysis of multiple samples and provide for access to the test samples for the formation of subsequent reaction mixtures, but permit only one type of analysis at a time. It would, thus, be an improvement to provide a random access analyzer which allows for analysis of multiple test samples for multiple analytes. It would be an even further improvement if such a random access analyzer allowed for continuous operations; that is, if additional or alternative samples could be loaded for analysis during analysis operations by the system, without interruption of the analysis operations.

With respect to reagent and fluids consumption and loss in present automated clinical analyzers, a common feature of those analyzers is the inclusion of various reagents within the apparatus itself or placed near the apparatus for pipetting purposes. In these systems, liquid reagents, in bulk form, are selected for the various types of tests which are to be performed on the test sample, and are stored in or near the apparatus. Reagent delivery units, such as pumps and the like, along with valves, control and pipette mechanisms, are included in the present automated analyzers so that different reagents can be mixed according to the type of test to be performed. In certain of these present analyzers, for example, the Abbott Imx^{®} analyzer previously mentioned, all the steps required for analysis of test samples are automatically performed and those steps include numerous checks of the subsystems to insure that assays are run to completion with valid results. In the Abbott IMx^{®} in particular, quantification of the fluorescence intensity in the MEIA method and polarization in the FPIA method, as well as the final data reduction, are fully automated on the analyzer and results are printed by the analyzer and may be accessed through suitable means for automatic data collection by a laboratory computer. These various aspects of the present automated clinical analyzers, like the Abbott IMx^{®}, help limit reagent and fluids consumption and loss, as well as provide other advantages. Even with those advantages, however, improvement in reagent and fluids consumption and loss in an analysis system would be desirable. Even further, such improvement in consumption and loss by these, combined with benefits of continuous operations, accuracy of results, and test menu versatility would be a significant improvement in the art.

With respect to continuous and high throughput in automated analytical systems, the prior systems have been unable to provide these desirable characteristics. In the prior automated analytical systems, the systems are initially loaded with a plurality of test samples. The samples are then each tested during a full cycle of testing by the systems. Though the number of samples which may be initially loaded in these systems is fairly large, it is not possible to load additional test samples in these systems at the same time the systems are testing the initial load. Additional samples may only be loaded after testing of the prior sample load is complete. In order to increase throughput in these systems then, it would be advantageous to provide an automated analytical system which allowed for loading of additional samples at any time, even while the system is testing other samples. It would be an even further advantage if such a system could provide accurate results, multiple test menu versatility, and low reagent and fluids loss and consumption while at the same time allowing continuous access to and testing of samples. The prior systems have been unable to provide these advantages. The present automated continuous and random access system provides, all these advantages. In addition to those advantages, the present invention also provides additional improvements directed to specific aspects, parts, and operations of these systems.

### Specific Aspects, Parts, and Operations

Other benefits and advantages, in addition to those previously described (i.e., accurate analytical results, multiple test menu versatility, low reagent and fluids consumption and loss, and continuous and high throughput), directed to specific aspects, parts, and operations of automated clinical analyzers would also be improvements in the art.

### A. Detection Systems:

For example, an improved analyzer might incorporate capability to perform both homogeneous and heterogeneous assays. Automated analytical apparatus for performing homogeneous assays, the detection of precipitate formed by reaction between antigens and antibodies in a test sample-cell to form light scattering centers, and methods and apparatus for detecting immunological agglutination reactions are known in the art. Such apparatus and methods include, for example, the steps of measuring light absorption of the liquid medium with antibody before and after the antigen-antibody reaction by using light which is absorbable by the antibody, and calculating the difference of the absorptions. In this way, the presence or absence of agglutination is detected based on the fact that the agglutination reaction reduces the concentration of antibody, which affects the light absorption of the liquid medium. As is typical of methods and apparatus for performing homogeneous assays, these procedures do not require separation of a solid phase from the reaction mixture for further analysis. Heterogeneous assays are also known through the use of a sample analyzer for quantitating relatively small amounts of clinically significant compounds in a liquid test sample by focusing a light source onto the sample so that, for example, fluorescent particles in the sample cause fluorescent conditions, the intensity of which is the function of the intensity of the light beam and the concentration of fluorescent particles in the sample. A detector senses photons forming the fluorescent emissions of the particles when excited by the light beam. The introduction of a solid phase material into the sample requires subsequent separation of the solid phase from the reaction mixture for further analysis and before the fluorescent emissions can be detected and measured. It would be advantageous in an automated clinical analyzer to incorporate apparatus and methods for performing, selectively on the same sample, various homogeneous and heterogeneous assays concurrently in a random access fashion. Such apparatus and methods could provide for the analysis of a plurality of liquid samples wherein each sample is analyzed with respect to at least one analyte utilizing both homogeneous and heterogeneous assay techniques.

### B. Syringe Bubble Flusher:

Another possible benefit and advantage directed to specific aspects, parts, and operations of automated clinical analyzers involves the precision and accuracy of the fluidics within the automated analytical instrument. That precision and accuracy during assay procedures is closely related to the precision and accuracy with which fluids can be aspirated and dispensed by the instrument. Although a syringe or similar device within the instrument can provide aspirating and dispensing steps, performance of prior syringes is often severely degraded by the presence of air bubbles in.the syringe. Existing construction and designs of such syringes have failed to provide efficient means of removing such bubbles. For example, various relatively ineffective and cumbersome manual techniques and manipulations, such as abruptly tapping the syringe, and the like, have been used to flush bubbles out of the syringe. Thus, there remains a need, and it would be, an improvement in the art, to provide an automated clinical analyzer with a fluidics system which includes a syringe or similar device to provide precise and accurate aspirations, dispensing, and bubble flushing steps while avoiding problems previously encountered in automatic flushing of bubbles completely from the fluidics system.

### C. Reaction Vessel and Loader:

It would also be advantageous in an automated analytical instrument to provide for ease of loading and handling of test samples and reagents. Such loading and handling can be problematic, in particular, when test samples and reagents are contained in containers of varying shape and size and must be supplied to the instrument. The present invention provides improved ease of loading and handling because of the particular containers used by the system for handling test samples and reagents, and further provides for ease of manipulation of those samples and reagents and reaction processes. In addition, the invention provides for disposable containers for those samples and reagents and other advantages, such as storage free from contaminants.

### D. Reagent Cap Actuator:

Even other benefits and advantages directed to specific aspects, parts, and operations of automated clinical analyzers are possible. Prior methods for reducing evaporation of costly reagents from system containers have utilized manual operations to cap reagent containers, as well as use of various other reclosing container caps which are held open during liquid access cycle and then allowed to reseal by removing the opening force. It would be an improvement in automated clinical analyzers to provide apparatus and methods wherein computer-controlled robotic devices replace the need for manual intervention in these regards. Those devices could be beneficial and advantageous if capable of minimizing reagent evaporation.

### E. Cartridge, Feeder and Carton:

Further benefits and advantages of specific aspects, parts, and operations of automated clinical analyzers are also desirable. The diagnostics industry has heretofore routinely utilized several types of systems which each require hand loading of cartridges, reagent packs, and sample containers into batch and semi-automatic instruments. In an automated, continuous and random access analytical system, manual hand loading of such items is further complicated when dealing with volume and reliability concerns. It would clearly be an improvement, then, to incorporate in these automated clinical analyzers an automatic feeder, capable of feeding tubular parts, such as cartridges, and orientating the cartridges with an open end up. An automatic cartridge feeder hopper of multiple cartridges could save substantial operator time and error since multiple cartridges could be loaded into the hopper feeder system directly from the cartridge packaging systems, eliminating individual hand feeding of the cartridges, and assuring reliability within the automated diagnostic system. Moreover, it would be advantageous to provide in these systems various handling and loading means to facilitate the handling and loading of reaction vessels which are utilized with systems.

### F. Sample Container Segment:

The invention even further provides advantageous mechanisms for loading particular containers. These mechanisms are of great importance because small, nonuniform containers are necessary in certain instances. Where many of these containers must be employed, it becomes hard, if not impossible, for an operator to maintain adequate supply to the instrument if loading and positioning of the containers is largely manual. The present invention provides improved containers and loading mechanisms to allow easy and continuous loading and processing capability.

### G. Optics Control System:

A further aspect in which improvement would be beneficial is optics and optic controls utilized by automated analytical systems. Measurements and other operations performed by these systems often employ characterization of relative optical properties. Because accuracy of these measurements and operations depend upon aspects of the optical system, the optical system must function in a reliable manner, particularly in an automated and continuous system in which a multitude of simultaneous operations are being performed in addition to the optical measurements.

### H. Smart Wash:

In automated analytical instruments, one particular area of concern has been carryover by or cross contamination of the samples, regiments, and other fluids. Typically, some form of wash is employed in these devices to wash apparatus between steps when switching between different fluid substances. The approach former employed has often been to maximize the amount of wash to account for the worst case scenario of potential contamination. It would be an improvement to provide a variable wash mechanism that operates according to the degree of potential contamination to insure no contamination, but yet conserves wash fluids and other resources.

### I. Chemiluminescent Test:

Chemiluminescent testing is generally known. In fact, chemiluminescent testing has been employed in certain automated analytical systems. It appears, however, that chemiluminescent testing has not been employed in a continuous and random access system. It would be an advantage to provide a continuous and random access analytical system suitable for chemiluminescent testing.

### J. Sample segment container:

The often differing sizes and configurations of containers employed with automated analytical systems presents special problems. Also, keeping track of those containers and their contents has required significant resources. It would be advantageous to provide means in an automated analytical system for handling and identifying containers.

### K. Liquid Level Sensor:

Yet another aspect of automated clinical analyzers which could be advantageously improved is the liquids measurement systems. In particular, a liquid level sense mechanism employed for sensing fluid levels in various sample containers is desired in those analyzers. The present invention provides a new fluid level sensing system to allow for liquids measurement. Such system is an improvement in the art.

The present invention, an automated continuous and random.access analytical system and components thereof, provides for the primary capabilities missing from the prior art automated clinical analyzers; that is, the present system combines accurate analytical result capability, multiple test menu versatility, low reagent and fluids loss and consumption, and continuous and high throughput. Because the present invention provides those capabilities, the invention is a significant improvement in the art. In addition to those capabilities, however, the present invention provides other benefits and advantages over the prior systems. Those benefits and advantages, in certain instances being directed to specific aspects, parts, and operations in an automated continuous and random access analytical system, provide much improvement in that particular system, as well as in numerous and varied possible applications.

HIROSHI MITSUMAKI ET AL. "Small Sized Automated Blood Analyzer Capable of Quick Response to Multiple Applications" Hitachi Review, Hitachi LTD., TOKYO, JP, vol. 41, no. 4, 1 September 1992 (1992-09-01), pages 161-166, XP000334655 ISSN 0018-277X describes a method for simultaneously performing at least two assays, said assays comprising a plurality of activities, for a plurality of liquid samples on a continuous analytical system, said method comprising the steps of: combining an aliquot of a first liquid sample with at least one reagent in a first reaction container to form a first assay reaction mixture for said first liquid sample; combining an aliquot of a second liquid sample with at least one reagent in a second reaction container to form a second assay reaction mixture for said second liquid sample; incubating said first and said second assay reaction mixtures at least one time; performing activities associated with each assay other than said combining and said incubating on the first and second assay reaction mixtures to complete each assay, said other activities including analyzing the incubated assay reaction mixtures; and scheduling the steps of said combining, said incubating, and said performing activities other than said combining and said incubating associated with each of the assays according to a predetermined protocol, said protocol specifying: (a) what activities are to be performed for a given assay; (b) an order in which said activities of (a) are to be performed; (c) at least one reaction period between said activities of (a), (d) how said activities of (a) are to be performed; and (e) duration of said activities of (a).

### Summary of the Invention

The present invention provides for a method for simultaneously performing at least two assays as defined in claim 1. Further advantageous features of the invention are set out in the dependent claims.

Additional advantages and novel features of the invention will be set forth in part in the description which follows, and will become apparent to those skilled in the art upon examination of the following or may be learned by practice of the invention. The objects and advantages of the invention may be obtained by means of the exemplary combinations more particularly pointed out in the following specification and appended claims, including all equivalents thereof.

### Brief Description of the Drawings

FIG. 1 is an isometric view of the automated analytical system illustrating the system cabinetry, exposed front end carousel, computer screen and keyboard.
FIG. 2 is an isometric view of the automated analytical system apparatus frame and cabinet.
FIG. 3 is a top plan view in section of the lower cabinet of FIGS. 1 and 2 illustrating water and/or buffer supply station as well as liquid and solid waster containers of the automated analytical system.
FIG. 4A is a top plan view of the automated analytical system in section with component covers removed to show the automated analytical system apparatus in detail and relative position.
FIG. 4B is a front elevational view of the automated analytical system in isolation and partial section of elements of the front end carousel.
FIG. 5 is a top view in isolation and partial section of drive and guide elements of the front end carousel of the automated analytical system being removed.
FIG. 6 is a cross-sectional side view of a process carousel of the automated analytical system in isolation with two reaction vessels in place, one of which is in position for an FPIA read.
FIG. 7 is an isometric view of the probe, probe arm and pipettor of the automated analytical system in isolation.
FIG. 8 is a schematic side view of the probe arm wiring and sensor means of the automated analytical system.
FIG. 9A is a sectional side view of the transfer element of the automated analytical system engaging a reaction vessel for transfer from the main carousel into the transfer station.
FIG. 9B is a perspective side elevational view of the transfer station of the automated analytical system.
FIG. 10 is a block diagram showing the sequence of activities to be performed in a first assay.
FIG. 11 is a block diagram showing the sequence of activities to be performed in a second assay.
FIG. 12 is a block diagram showing an incubation period between two activities as comprising a nominal incubation period and a variable incubation window.
FIG. 13 is a set of six block diagrams each showing a different combination of activities and incubation periods reflecting the rules of a flexible protocol technology.
FIG. 14 is a block diagram showing the timing protocol for a pipetting activity.
FIG. 15 is a top plan view of the automated analytical system in section with component covers removed to show the automated analytical apparatus in detail and relative position inclusive of a chemiluminescent reader for a magnetic particle capture technology and a chemiluminescent reader for membrane particle capture technology.
FIG. 16 is a cross sectional view of a detection head of the detection device for chemiluminescent detection.
FIG. 17 is a cross sectional view in section of the detection device light pipe positioned over a chemiluminescent particle capture container with light shield in place.
FIG. 18 is a simplified block diagram of the preferred embodiment of the liquid level sensing device utilized in connection with an automated analytical system.
FIG. 19 is a more detailed block diagram of the liquid level sensing system of FIG. 18.
FIG. 20 is a simplified schematic diagram illustrating the current flow in the fluid level sensing system.
FIG. 21 is an illustration of the geometries between the probe, its electromagnetic field, a liquid sample container, and the antenna when the probe is in air.
FIG. 22 is an illustration of the geometries between the probe, its electromagnetic field, a liquid sample container, and the antenna when the probe contacts liquid.
FIG. 23 is an illustration of the geometries between the probe, its electromagnetic field, a liquid sample container, and the antenna when the probe has contacted liquid and the distance from the probe/liquid combination to the antenna is too great to trigger a detection.
FIG. 24 is an illustration of a sensing sleeve which functions to channel the electrical signal from the probe/liquid combination to the vicinity of the receiving antenna.
FIG. 25 is a graphical representation of system noise level versus signal frequency.
FIG. 26 is a cross-sectional side elevational view of an automatic bubble flushing syringe of the automated analytical system.
FIG. 27 is a sectional end view in isolation of the piston and bore of the automatic bubble flushing syringe of FIG. 26.
FIG. 28 is a sectional side view in isolation of the syringe bore end portion of the automatic bubble flushing syringe with the reciprocating piston near the end of travel toward the bore end portion and a phantom position within the bore illustrating the piston withdrawal to the outward extension;
FIGS. 29 and 30 represent a perspective side elevational view and partial end view of a reagent pack and reagent pack cover means for use with the automated analytical system.
FIG. 31 is a top view of a reagent pack having the reagent containers covered.
FIG. 32 taken along section A-A of FIG. 31 presents a side view in section taken along the line A-A of FIG. 31 illustrating a cover means in various open and closed positions.
FIG. 33 is an isometric view of an open reagent vessel capping means.
FIG. 34 is a perspective side elevational view of a reagent container lid opening and closing station with the reagent containers in the reagent pack having the lids opened.
FIG. 35 presents a different perspective side elevation view from that of FIG. 34 wherein the reagent containers of the reagent pack are below elements of the opening and closing station with the reagent pack lids being closed.
FIG. 36 is a, perspective view of a test sample container segment assembly.
FIG. 37 is a bottom view of the test sample container segment assembly of FIG. 36.
FIG. 38 is a cross sectional view in isolation of the sample carousel with a mounted test sample container segment assembly also in cross section.
FIG. 39 is a cross sectional view of a modified sample cup with skirts.
FIG. 40 is a perspective view of a short test sample Vacutainer^{®} tube segment assembly.
FIG. 41 is a top cross sectional view of the short test sample Vacutainer^{®} tube segment assembly taken along the line A-A of FIG. 40.
FIG. 42 is a bottom view of the short test sample Vacutainer^{®} tube segment assembly of FIG. 40.
FIG. 43 is a cross sectional view of a long test sample cup adaptor with tube in place.
FIG. 44 is a cross sectional view of a short test sample cup adaptor with tube in place.
FIGS. 45A and 45B represent a top plan view of a reaction vessel and a side view of the redaction vessel for use with the automated analytical system, respectively, with reaction vessel compartments labeled where appropriate for FPIA processing.
FIG. 45C present an end view of the reaction vessel of FIG. 45B.
FIG. 46 is an isometric view in section of the reaction vessel loading device illustrating the device holding to vessels and means for mounting other vessels.
FIG. 47 is a top view of the reaction vessel loading device presented in an arc which matches the radius of the reaction vessel carousel, the loading device having mounted thereon ten reaction vessels.
FIG. 48 is an isometric view in section of the reaction vessel loading device illustrating the loader mounted with two reaction vessels and means for mounting other reaction vessels.
FIG. 49 is a top view of the reaction vessel loading device, the reaction vessel loading device having arced linear dimensions which match the radius of the reaction vessel carousel, the loader having mounted thereon two reaction vessels and the capability of mounting eight additional reaction vessels.
FIG. 50 is a schematic view illustrating the system control environment airflow and temperature control of the automated analytical system.
FIG. 51 is an elevational, cross-sectional view of the process carousel as disposed in the controlled environmental zone and holding a plurality of reaction vessels.
FIG. 52 is a perspective view of a heater assembly for liquid temperature control.
FIG. 53 is a cross-sectional view through the heater assembly of FIG. 52 showing the heater element within the block.
FIG. 54 is a partial cross-sectional view of the heater assembly of FIG. 52 showing liquid tubing, for example, a tubing coil within the heater assembly.
FIG. 55 is a side elevational view in partial section of a MEIA cartridge for use with the automated analytical system.
FIG. 56 is a side elevational view in section of a MEIA cartridge feeder of the automated analytical system.
FIG. 57 is a side sectional view in isolation of the MEIA cartridge feeder-cartridge orientation pin mechanism of the cartridge feeder of FIG. 56.
FIG. 58 is a side cross-sectional view in isolation of a split open cartridge carton shown in various open positions in phantom as engaged in cooperation with a cartridge hopper containing multiple cartridges.
FIG. 59A is an isometric view of the cartridge carton, taken form the lower side of the cartridge carton.
FIG. 59B is a partial, isometric view of the cartridge carton, illustrating the operation of the tab opening.
FIG. 60 is an isometric view of the cartridge carton, taken from the upper side of the cartridge carton.
FIG. 61 is an isometric view of another embodiment of a free standing cartridge hopper showing the cartridge hopper in a detached mode suitable for loading cartridges from a cartridge carton.
FIG. 62 is a schematic of the FPIA optics system of the automated analytical system.
FIG. 63 is a schematic of the MEIA optics system of the automated analytical system.
FIG. 64 is a box diagram of the optics control system of the automated analytical system.
FIG. 65 is a pictorial time graph of the FPIA reader sequence of the automated analytical system.
FIG. 66 is a pictorial time graph of the MEIA read sequence of the automated analytical system.

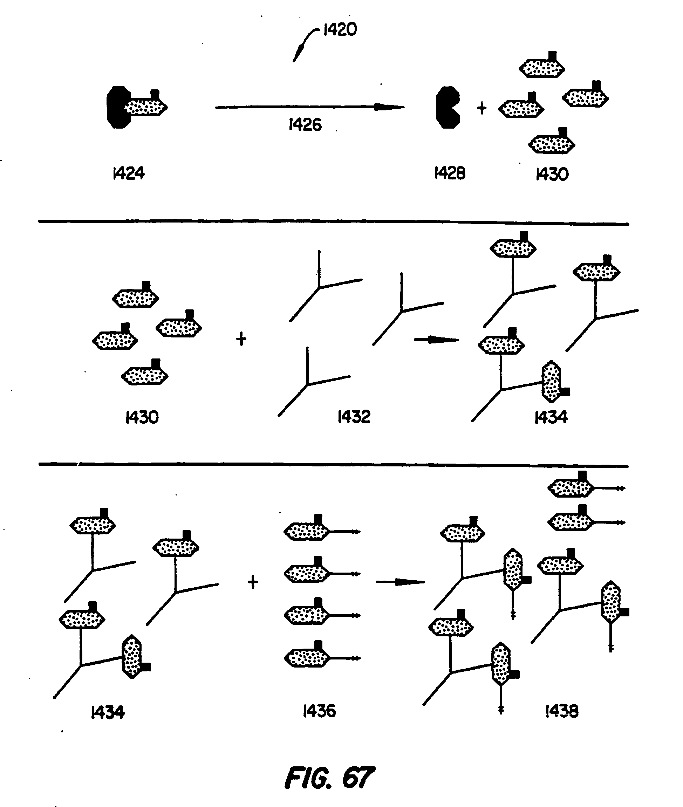

FIG. 67 is a schematic reaction sequence of a FPIA for T4 performed on the automated analytical system.

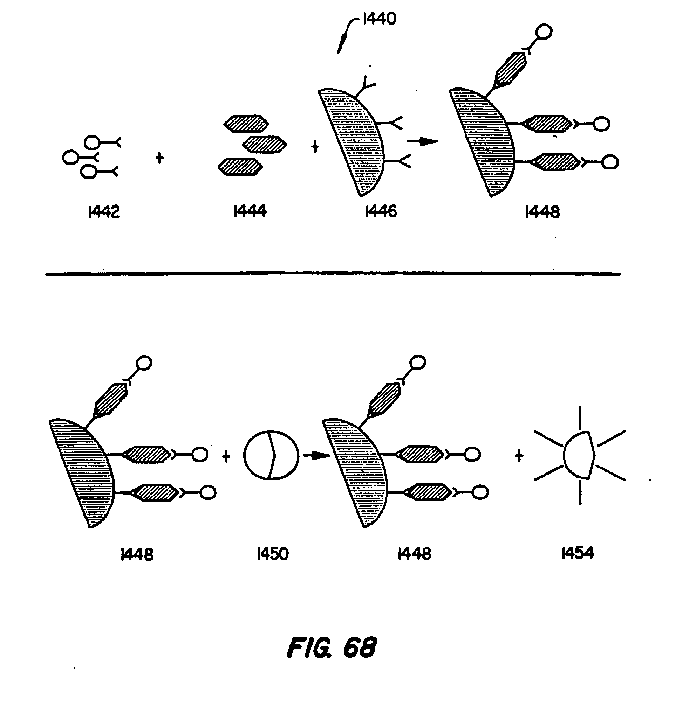

FIG. 68 is a schematic reaction sequence of a one-step sandwich MEIA performed on the automated analytical system.

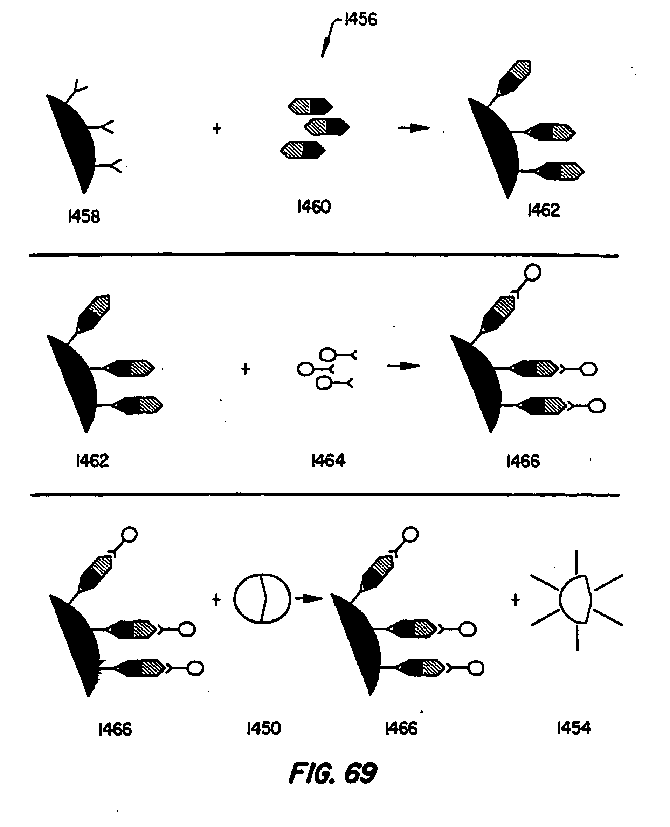

FIG. 69 is a schematic reaction sequence of a two-step sandwich MEIA performed on the automated analytical system.

### Detailed Description of the Invention

The following description is divided into separate sections with headings to more clearly describe the invention, but should not be considered as limiting the scope of the invention.

### DEFINITIONS

The following definitions are applicable to the present invention:

The term "test sample", as used herein, refers to a material suspected of containing the analyte. The test sample can be used directly as obtained from the source or following a pretreatment to modify the character of the sample. The test sample can be derived from any biological source, such as a physiological fluid, including, blood, saliva, ocular lens fluid, cerebral spinal fluid, sweat, urine, milk, ascites fluid, raucous, synovial fluid, peritoneal fluid, amniotic fluid or the like. The test sample can be pretreated prior to use, such as preparing plasma from blood, diluting viscous fluids, or the mike; methods of treatment can involve filtration, distillation, concentration, inactivation of interfering components, and the addition of reagents. Besides physiological fluids, other liquid samples can be used such as water, food products and the like for the performance of environmental or food production assays. In addition, a solid material suspected of containing the analyte can be used as the test sample. In some instances it may be beneficial to modify a solid test sample to form a liquid medium or to release the analyte.

The term "analyte" or "analyte of interest", as used herein, refers to the compound or composition to be detected or measured and which has at least one epitope or binding site. The analyte can be any substance for which there exists a naturally occurring binding member or for which a binding member can be prepared. Analytes include, but are not limited to, toxins, organic compounds, proteins, peptides, microorganisms, amino acids, nucleic acids, hormones, steroids, vitamins, drugs (including those administered for therapeutic purposes as well as those administered for illicit purposes), virus particles and metabolites of or antibodies to any of the above substances. The term "analyte" also includes any antigenic substances, haptens, antibodies, macromolecules and combinations thereof.

The term "analyte-analog", as used herein, refers to a substance which cross-reacts with an analyte-specific binding member, although it may do so to a greater or lesser extent than does the analyte itself. The analyte-analog can include a modified analyte as well as a fragmented or synthetic portion of the analyte molecule, so long as the analyte-analog has at least one epitopic site in common with the analyte of interest. An example of an analyte-analog is a synthetic peptide sequence which duplicates at least one epitope of the whole-molecule analyte so that the analyte-analog can bind to an analyte-specific binding member.

The term "binding member", as used herein, refers to a member of a binding pair, i.e., two different molecules wherein one of the molecules specifically binds to the second molecule through chemical or physical means. In addition to antigen and antibody binding pair members, other binding pairs include, as examples without limitation, biotin and avidin, carbohydrates and lectins, complementary nucleotide sequences, complementary peptide sequences, effector and receptor molecules, enzyme cofactors and enzymes, enzyme inhibitors and enzymes, a peptide sequence and an antibody specific for the sequence or the entire protein, polymeric acids and bases, dyes and protein binders, peptides and specific protein binders (e.g., ribonuclease, S-peptide and ribonuclease S-protein), and the like. Furthermore, binding pairs can include members that are analogs of the original binding member, for example, an analyte-analog or a binding member made by recombinant techniques or molecular engineering. If the binding member is an immunoreactant it can be, for example, a monoclonal or polyclonal antibody, a recombinant protein or recombinant antibody, a chimeric antibody, a mixture(s) or fragment (s) of the foregoing, as well as a preparation of such antibodies, peptides and nucleotides for which suitability for use as binding members is well known to those skilled in the art.

The term "detectable moiety", as used herein, refers to any compound or conventional detectable chemical group having a detectable physical or chemical property and which can be used to label a binding member to form a conjugate therewith. Such detectable chemical group can be, but is not intended to be limited to, enzymatically active groups such as enzymes, enzyme substrates, prosthetic groups or coenzymes; spin labels; fluorescers and fluorogens; chromophores and chromogens; luminescers such as chemiluminescers and bioluminescers; specifically bindable ligands such as biotin and avidin; electroactive species; radioisotopes; toxins; drugs; haptens; DNA; RNA; polysaccharides; polypeptides; liposomes; colored particles and colored microparticles; and the like.

The term "continuous access", as used herein, refers to the ability to add additional test samples or reagents to the automated analytical system of the present invention without the interruption of assays which are being performed by the automated analytical system of the present invention at the time of such addition.

The term "random access", as used herein, refers to the ability of the automated analytical system of the present invention to simultaneously perform more than one scheduled assay in any order in which such plurality of scheduled assays are presented into the automated analytical system of the present invention.

The term "simultaneous", as used herein, refers to the ability of the automated analytical system of the present invention to independently perform two or more scheduled assays at the same time.

The term "kitting", as used herein, refers to the ability of the automated analytical system of the present invention to create a unit dose disposable by separately transferring test samples and reagents to a reaction vessel without initiation of an assay reaction sequence.

The term "quat" refers to a polycationic material solution for assays which use these materials which are not an antibody or antigen to capture the analyte from the sample on the' matrix of, for example, MEIA cartridge. In the system, quat is dispensed to the matrix during test processing, prior to the transfer of the reaction mixture from the reaction vessel.

### DETECTION SYSTEM

The automated analytical system of the present invention is capable of performing various assays employing various detection systems known in the art and include, but are not intended to be limited to, spectrophotometric absorbance assay such as end-point reaction analysis and rate of reaction analysis, turbidimetric assays, nephelometric assays, radiative energy attenuation assays (such as those described in U. S. Patent No. 4, 496, 293 and U.S. Patent No. 4, 743, 561), ion capture assays, colorimetric assays, fluorometric assays, electrochemical detection systems, potentiometric detection systems, amperometric detection systems, and immunoassays. Immunoassays include, but are not intended to be limited to, heterogeneous immunoassay such as competitive immunoassays, sandwich immunoassays, immunometric immunoassays, and the like, where the amount of a detectable moiety employed therein can be measured and correlated to the amount of analyte present in a test sample.

Generally, in a spectrophotometric assay, such as those performed on the Abbott Spectrum clinical analyzer and the Abbott Spectrum Series II clinical analyzer (Abbott Laboratories, Abbott Park, IL, USA) the interaction in an assay solution between the analyte to be determined and a reagent system specific for the analyte produces a detectable change in the transmittive properties of the assay solution. The change in the transmittive properties refers to the amount of light absorbed or scattered by an assay solution within a particular wavelength band when a beam of light of known intensity is passed through the assay solution. The change in the transmittive properties of an assay solution is measured by passing monochromic light having a known intensity though the assay solution and determining the ratio of the intensity of the transmitted or scattered light to the intensity of the incident light. Nearly all analytes either absorb energy of a specific wavelength or interact in an assay solution with a particular reagent system to produce a detectable change in the transmittive properties of the assay solution, characteristics which have resulted in the development of numerous specific spectrophotometric assays.

Spectrophotometric assays which rely upon the measurement of the change in the transmittive properties of an assay solution as a measure of an analyte in the assay solution include, for example, assays wherein there is a change in the color of the assay when there is a change in the turbidity of the assay solution, that is, turbidimetric or nephelometric assays.

In a colorimetric assay, the change in the transmittive properties of an assay solution is generally referred to as the absorbance of the assay solution and is dependent upon the change in the color of the assay solution due to the interaction of the analyte to be determined and reagent system specific for the analyte. The absorbance of the assay solution is related to the concentration of the analyte in the assay solution. A colorimetric assay utilizes a chromogenic reagent system capable of interacting in an assay solution with the particular analyte of interest, to produce a detectable change in the transmittive properties, specifically the color, of the assay solution. Numerous chromogenic reagent systems useful in the determination of specific analytes have been developed and are commercially available.

The principle of turbidimetric assays is to determine the amount of light scattered or blocked by particulate matter as light passes though an assay solution. In a turbidimetric assay, the analyte of interest interacts with a reagent system specific for the analyte to form a suspension of particles in the assay solution. As a beam of light having a known intensity is passed through an assay solution, the suspension of particles formed by the interaction of the analyte reagent system blocks or scatters the incident light, thereby reducing the intensity of the light transmitted through the assay solution. The change of the transmittive properties in a turbidimetric assay refers to the decrease in the intensity of the light transmitted through an assay solution, is related to the amount of incident light that is scattered or blocked by the suspension of particles, and depends upon the number of particles present and the cross-sectional area of such particles.

A nephelometric assay is similar to a turbidimetric assay in that the analyte of interest interacts with a reagent system specific for the ligand to form a suspension of particles in the assay solution. In a nephelometric assay, the change in the transmittive properties of the assay solution is also related to the amount of incident light scattered or blocked by the suspension of particles, but unlike a turbidimetric assay wherein the intensity of the light transmitted through the assay solution is measured, the scattered or blocked light is measured at an angle to the light incident to the assay solution. Therefore, in a nephelometric assay the change in the transmittive properties refers to the difference in intensities of light incident to the assay solution and light scattered at an angle to the incident light. Turbidimetric and nephelometric assays are utilized in the analysis of blood, urine, spinal fluid, and the like, for the determination of analytes such as proteins wherein there is no comparable colorimetric assay due to the lack of an effective chromogenic reagent system. Yoe and Klimman, Photoelectric Chemical Analysis, Vol. II: Nephelometry, Wiley & Sons, Inc., New York, 1929, describe various nephelometric assays various reagents and reagent systems which can be employed for performing spectrophotometric assays on the automated analytical systems of the present invention include, but are not intended to be limited to, those for the simultaneous determination of glucose and urea, such as described in U. S. Patent No. 5,037,738. The simultaneous determination of calcium and phosphorous; the simultaneous determination of cholesterol and triglycerides; determining isoenzymes; determining blood ammonia levels, and the like, can be performed on the apparatus and by the methods of the present invention.

Typically in a fluorometric assay, an analyte in an assay solution is chemically or immunologically transformed into a fluorescent complex or conjugate thereby producing a detectable change in the fluorescent properties of the assay solution. The change in the fluorescent properties of the assay solution is measured by exciting the fluorescent complex or conjugate properties produced with monochromatic light of a wavelength within the excitation wavelength band of the fluorescer, and measuring the intensity of the emitted light at a wavelength within the emission wavelength band of the fluorescer. The fluorescent intensity of the emitted light is related to the concentration of the analyte However, the intensity of the fluorescence emitted by the assay solution may be inhibited when the ligand to be determined complexes with nonfluorescent interferences such as protein or phosphates present in the sample, or when the sample containing the ligand to be determined has sufficient color so as to act as a filter and thereby reduce the intensity of the emitted fluorescence. It is well recognized that in order to maximize the sensitivity and specificity of a fluorometric assay, these inhibiting factors, if present, must be overcome either by removal of the nonfluorescent interferences or color producing material prior to the analysis, or by compensating for the presence of such factors using an internal standard added to a second aliquot of sample and carrying out the entire assay procedure using the aliquot containing the internal standard.

### ASSAY FORMATS

Generally, homogeneous and heterogeneous immunoassays depend upon the ability of a first binding member of a binding member pair to specifically bind to a second binding member of a binding member pair wherein a conjugate, comprising one of such binding members labeled with a detectable moiety, is employed to determine the extent of such binding. For example, where such binding pair members are an analyte and an antibody to such analyte, the extent of binding is determined by the amount of the detectable moiety present in the conjugate, which either has or has not participated in a binding reaction with the analyte, wherein the amount of the detectable moiety detected and measured can be correlated to the amount of analyte present in the test sample.

Homogeneous immunoassays typically are performed in a competitive immunoassay format involving a competition between an analyte from a test sample and a tracer for a limited number of receptor binding sites on an antibody to the analyte. The tracer comprises the analyte or analog thereof labeled with a detectable moiety wherein the concentration of analyte in the test sample determines the amount of the tracer that will specifically bind to the antibody. The amount of the tracer-antibody conjugate produced by such binding may be quantitatively measured and is inversely proportional to the amount of analyte present in the test sample. For example, fluorescent polarization techniques for making such determination, such as in fluorescent polarization immunoassays as described herein, are based on the principle that a fluorescently labeled compound when excited by linearly polarized light will emit fluorescence having a degree of polarization inversely related to its rate of rotation. When a molecule such as a tracer-antibody conjugate having a fluorescent label is excited with a linearly polarized fluorescent molecule it is constrained from rotating between the time light is absorbed and emitted. When a "free" tracer molecule (i.e., unbound to an antibody) is excited by linearly polarized light, its rotation is much faster than the corresponding tracer-antibody conjugate and the molecules are more randomly orientated, therefore, the emitted light is polarized. Accordingly, when plane polarized light is passed through a solution containing the aforementioned reagents, a fluorescent polarization response is detected and correlated to the amount of analyte present in the test sample.

Various fluorescent compounds which can be employed for performing fluorescent polarization assays on the automated analytical system of the present invention include, but are not intended to be limited to, aminofluoresceins, such as described in U. S. Patent No. 4, 510, 251 and U. S. Patent No. 4, 614, 823, triazinylaminofluoresceins, such as described in U.S. Patent No. 4, 420, 568 and U.S. Patent No. 4, 593, 089, carboxyfluoresceins, such as described in U.S. Patent No. 4,668,640, and the like.

Heterogenous immunoassays typically involve a labeled reagent or tracer comprising an analyte, an analog of the analyte, or an antibody thereto, labeled with a detectable moiety, to form a free species and a bound species. In order to correlate the amount of tracer in one of such species to the amount of analyte present in the test sample, the free species must first be separated from the bound species, which can be accomplished according to methods known in the art employing solid phase materials for the direct immobilization of one of the binding participants in the binding reaction, such as the antibody, analyte or analog of the analyte, wherein one of the binding participants is immobilized on a solid phase material, such as a test tube, beads, particles, microparticles or the matrix of a fibrous material, and the like, according to methods known in the art.

Heterogenous immunoassays can be performed in a competitive immunoassay format as described above wherein, for example, the antibody can be immobilized to a solid phase material whereby upon separation, the amount of the tracer which is bound to such solid phase material can be detected and correlated to the amount of analyte present in the test sample. Another form of a heterogeneous immunoassay employing a solid phase material is referred to as a sandwich immunoassay, which involves contacting a test sample containing, for example, an antigen with a protein such as an antibody or another substance capable of binding the antigen, and which is immobilized on a solid phase material. The solid phase material typically is treated with a second antigen or antibody which has been labeled with a detectable moiety. The second antigen or antibody then becomes bound to the corresponding antigen or antibody on the solid phase material and, following one or more washing steps to remove any unbound material, an indicator material such as a chromogenic substance which reacts with the detectable moiety (e.g., where the detectable moiety is an enzyme, a substrate for such enzyme is added) to produce a color change. The color change is then detected and correlated to the amount of antigen or antibody present in the test sample.

For example, a heterogeneous immunoassay which can be performed by the automated analytical system of the present invention, in either a competitive or sandwich immunoassay format, is a microparticle capture enzyme immunoassay, such as that described in Clinical Chemistry, Volume 34, No. 9, pages 1726-1732 (1988), employing microparticles as the solid phase material.

In addition, the use of sucrose in microparticle diluent has been found to achieve neutral density of the microparticles. The methodology entails the determination of the optimum sucrose concentration which will eliminate the settling of microparticles. The sucrose concentration required to achieve neutral density is assay specific and microparticle lot specific. The principal involves dissolving sucrose in solution to increase the density of the diluent. When the density of the diluent and microparticles are equivalent, the microparticles will be in a suspended state. Density neutralization can also be achieved by using other materials such as metrizamide and/or metrizoic acid.

Separation of the bound and free species is accomplished by capture of the microparticles on a glass fiber matrix of a simple cartridge (herein, the "MEIA cartridge"), a process that relies on the high affinity of glass fibers for the microparticles, wherein the microparticles adhere to the surface of the fibers irreversibly, and nonspecifically bound material can be effectively removed by washing the matrix. The matrix also provides a precisely located mechanical support for the microparticles during the optical quantification phase of the assay protocol as described herein.

When performing a sandwich immunoassay, microparticles coated with antibody to the analyte in the test sample are incubated with the test sample containing the analyte of interest to form a capture complex with the analyte from the test sample. A conjugate comprising antibody to the analyte labeled with a detectable moiety, preferably an enzyme, is then incubated with the capture complex to form the second of a sandwich complex. When performing a competitive immunoassay, microparticles coated with antibody to the analyte in the test sample are incubated with the test sample containing the analyte of interest and a conjugate comprising the analyte or analog thereof labeled with a detectable moiety, preferably an enzyme. Removal of unbound conjugate is accomplished with the glass fiber matrix of the MEIA cartridge and, where the detectable moiety is an enzyme, a substrate for the enzyme capable of providing a detectable signal is added and the signal provided thereby is measured and correlated to the amount of analyte present in the test sample. Preferably, the enzyme-substrate system employed by the competitive and sandwich MEIA formats is alkaline phosphatase and 4-methylumbelliferyl phosphate (MUP), although other enzyme-substrate systems known in the art can be employed as well.

The MEIA cartridge which is employed by the automated analytical system of the present invention comprises a reaction well for retaining and immobilizing microparticle-analyte complexes. The reaction well has an entrance port and means for holding a quantity of sample and assay reaction mixtures positioned over a fibrous matrix which retains and immobilizes microparticle-analyte complexes as described above. The fibrous matrix is composed of fibers having an average spatial separation greater than the average diameter of the microparticles. Preferably, the average fiber spatial separation is greater than 10 microns.

The reaction well further comprises an absorbent material positioned below the fibrous matrix to enhance the flow of sample and assay reaction mixtures through the fibrous matrix. Preferably, the absorbent material is a fibrous material whose fibers predominantly lie in a plane perpendicular to the lower surface of the fibrous matrix. The absorbent material is in fluid communication with the fibrous matrix. Generally, the absorbent material is in physical contact with the lower surface of the fibrous matrix. The interior of the reaction well, therefore, is generally sized or contains positioning means to maintain the fluid communication between the absorbent material and the fibrous matrix. Preferably, a spike located at the bottom of the reaction well can be used to force the absorbent material into contact with the lower surface of the fibrous matrix. Additionally, it is preferable to vent to the atmosphere the gases displaced in the absorbent material by the liquids absorbed therein during the performance of an immunoassay.

According to the immunoassay methodologies described above, standard solutions of the analyte of known concentrations covering the clinical concentration range are typically prepared and assayed as is the test sample to be assayed. This blank assay provides a series of signal measurements corresponding to the known concentrations from which a standard curve is drawn. The optical signal corresponding to the unknown sample is correlated in a concentration value through interpretation from the blank or standard curve.

### ANALYTICAL SYSTEM METHOD

Automated analytical methodology for effecting analysis of a plurality of test samples according to the present invention is achieved by introducing reagent packs, test sample container and reaction vessels onto concentric carousels of a main carousel. The test sample container can be a test tube, cuvette, vacutainer tube, and the like, for holding a test sample. The reagent packs and test sample containers are identified and aligned respectively with a reaction vessel for transfer and kitting of the reaction vessel by transfer of test sample and specific reagents from the reagent pack for preparation of a predetermined test. The reaction vessel containing the test sample and one or more reagents is transferred to a process carousel wherein controlled environment conditions exist for incubation once the sample has been appropriately mixed with various -reagents to form a reaction mixture. When all assay processing steps have been completed, the reaction mixture is identified and transferred to at least, for example, one of a fluorescent polarization immunoassay reader or a microparticle enzyme immunoassay cartridge positioned on a separate cartridge wheel or carousel for further preparation before reading. The processed test samples are read and the readings are calculated with the resulting data being recorded and/or printed.

The methodology of the automated immunoassay analytical system is achieved through the use of a self-contained, fully automated, continuous and random access instrument comprising a main carousel assembly consisting of the reagent pack carousel, a reaction vessel carousel and a test sample container carousel concentrically and independently rotatable. The main carousel assembly is provided with a transfer pipette operated by a boom arm for transferring and kitting test sample and reagents into the reaction vessel automatically following a predetermined test schedule. The main carousel assembly is provided with bar code readers for reagent packs and test sample containers and has the capability of aligning the reagent pack carousel and test sample container carousel and a reaction vessel for pipette transfer operations. Once the assay to be performed is scheduled, the reaction vessel carousel, the reagent pack carousel and the test sample container carousel are rotated until the reaction vessel, a reagent pack and a test sample container, respectively, are determined to be in the transfer pipette access position. The transfer pipette then transfers the test sample from the test sample container and, depending upon the assay to be performed, the reagents from the reagent pack are transferred to the reaction vessel. The reaction vessel carousel is then rotated to a transfer station position which contacts the reaction vessel with a transfer mechanism and pulls the reaction vessel into the transfer station. The reaction vessel is then loaded onto the process carousel by the transfer mechanism.

When performing a fluorescent polarization immunoassay (FPIA) with the automated analytical system of the present invention as described in more detail below, various pipetting activities are performed by a second transfer pipette apparatus which is in service for the process carousel, and the process carousel is rotated so that the reaction vessel, when properly pipetted with, for example, FPIA reagents, is at the read station of the FPIA processing stations and the FPIA determination on reading, is made on the reaction vessel. The process carousel is then rotated so that the read reaction vessel is at the transfer station. The reaction vessel is again contacted and transferred by the transfer station. The transfer station is rotated and pushes the reaction vessel into a release container opening.

For a microparticle enzyme immunoassay (MEIA) performed with the automated analytical system of the present invention as described in more detail below, after the various pipetting activities for the MEIA, which can be completed at the main carousel assembly, the reaction vessel is transferred to the process carousel as described in the FPIA process. Pipetting can also be accomplished in the process carousel or jointly between the two carousels. To complete the MEIA, the reaction mixture is transferred from the reaction vessel to a matrix of an MEIA cartridge on a cartridge carousel with the second transfer pipette. The matrix is washed with a buffer and a substrate, such as MUP (defined earlier), or other suitable substrate known in the art. The cartridge carousel is then rotated so that the MEIA cartridge is positioned at an MEIA processing assembly and the MEIA determination is made. The MEIA reaction vessel is ejected into the waste container as described for the FPIA reaction vessel. The MEIA cartridge is independently ejected from the cartridge wheel by an ejector at an appropriate ejector station into a waste container.

Preferably, two distinct analytical technologies as described above, FPIA and MEIA, are incorporated into the automated analytical system of the present invention; however, more than two distinct analytical technologies can be incorporated into the inventive system. These methods are complimentary and share a commonality of apparatus and procedural steps, with the FPIA generally being the method of choice for analytes of low molecular weight and MEIA for molecules such as protein hormones, antibodies or analytes of low molecular weight requiring higher sensitivity. The two technologies share system components including the operator control panel, pipetting boom assemblies, fluidics systems, air and liquid reagent heaters, printers, bar code reader and stepper motors. Such commonality of use of system components allows for a compact instrument despite the dual FPIA and MEIA capability.

The FPIA optic systems (such as described in U.S. Patent No. 4,269,511 can utilize a polarizing filter which is an electrically switched liquid crystal, maintaining a compact size and avoiding complex and potentially unreliable moving parts. When performing FPIA assays utilizing the automated analytical system of the present invention, the FPIA reagent packs will typically include a tracer comprising the analyte or analog thereof, coupled to a detectable moiety, an antibody specific to that analyte, and a specimen pretreatment reagent. In a preferred FPIA format, the analyte being determined competes with the tracer for a limited number of binding sites on the antibodies specific to the portion or portions of the analyte and tracer. The detectable moiety component of the tracer is preferably a fluorescent moiety selected from the group consisting of fluoresceins, aminofluoresceins, carboxyfluoresceins, fluoresceinamines, and the like, more preferably carboxymethyl-aminomethyl-fluorescein, carboxyethylaminomethyl-carboxyfluorescein, 6-carboxyfluorescein, 5-carboxyfluorescein, succinylanimomethyl-fluorescein, thiourea-aminofluorescein, methoxytrianolylaminofluorescein, aminofluorescein, and the like.

In another embodiment, the FPIA format utilizes a unique, round, plastic, reaction cuvette suitable for fluorescence polarization and absorbance assay technologies which require no orientation other than top-to-bottom. This plastic reaction cuvette has physical characteristics of low birefringence throughout the optical read region as well as stringent dimensional tolerances which allow reproducible absorbance readings. Bifringence is defined as the degree of retardation of the extraordinary ray as it passes through a material. The greater the degree of retardation, the greater will be the level of birefringence. Retardation of the extra-ordinary ray is dependent on the magnitude and direction of the induced stress. Therefore, passing a ray of linearly polarized light through a material with induced stress will result in depolarization of the ray. In order for a cuvette to be utilized for fluorescence polarization measurements, it is important that the cuvette be prepared under conditions which yield minimum stress. The geometry of the cuvette' has been designed to utilize the inherent fluidics of automated medical diagnostic instrumentation to minimize the hydrophobic effect of plastic.

MEIA results can be determined by quantifying the rate of fluorescence developed when fluorogenic substrate is converted by the action of an enzyme labeled conjugate. For example, when performing either a competitive MEIA or sandwich MEIA, the specifically bound alkaline phosphatase on the microparticles is detected by addition of the fluorogenic substrate MUP to the matrix. The alkaline phosphatase catalyzes hydrolysis of the MUP to inorganic phosphate and fluorescent 4-methylumbelliferone (4-MU). The liberated 4-mu is detected by the MEIA optics assembly front surface fluorometer which is designed to detect fluorescence of low concentrations of 4-MU without interference by fluorescence of 4-MUP at a wavelength of 367 nm. A system of lenses and optical filters focus filtered light (wavelength = 365 nm) from a mercury arc lamp on to the surface of the matrix and focus emitted fluorescence from 4-MU (wavelength = 448 nm) on to a photo multiplier tube. Like the FPIA optics assembly, the MEIA optics system is compact and has no moving parts. About five percent of the excitation light is detected by a photodiode, allowing normalization of the fluorescence data and generation of a control signal used by the lamp power supply to maintain the intensity of the excitation light within five percent over the useful life of the lamp. The MEIA post-processor uses linear regression analysis to convert the data from multiple successive determinations of 4-MU fluorescence to a rate which is proportional to the concentration of alkaline phosphatase conjugate specifically bound to the microparticles.

MEIA formats can be run with a multi-position MEIA auxiliary carousel and process carousel as well as a MEIA reagent pack containing microparticle reagent, an alkaline phosphatase conjugate and, in some cases, a dilute buffer specific for the assay being performed. Because the microparticles tend not to settle out of suspension during the course of the assay, they can readily be pipetted. The effective surface area of polystyrene latex microparticles is several fold greater than that of a large diameter polystyrene bead (e.g., one quarter inch (ca. 6 mm) beads) commonly used in commercial immunoassays. Because of this large surface area and the very small diffusion distance between analyte and the capture molecules on the surface of the microparticles, the capture phase employed in many of the MEIA methods being performed reaches equilibrium within several minutes, allowing for a full carousel of test samples to be completed in a very short time frame.

Unlike an FPIA, the heterogeneous immunoassays, such as a MEIA, require a separation step as described above. In particular, after incubation of the microparticles with a test sample, the microparticles are separated from the reaction mixture by transfer to the matrix contained in the MEIA cartridge as described above. The matrix provides a precisely located mechanical support for the microparticles during the subsequent optical read phase-of the assay. This precisely located mechanical support, i. e. the cartridge, is fit into the auxiliary carousel at a predetermined spacing from the reader apparatus by camming means.

### ANALYTICAL SYSTEM APPARATUS

The automated immunoassay analytical system according to the present invention (hereinafter the "analytical system" or "system") is both continuous and random access. The following description of the analytical system includes a general description of sufficient scope for those skilled in the relevant arts, followed by more detailed descriptions of critical components and subsystems unique to the system. The drawings do not illustrate all of the mechanical and electrical elements for driving and controlling the various components of the system, because the structure and operation of such omitted elements are known to those of ordinary skill in the art who having knowledge of the information provided herein would understand the operation of the system and the various components and related processes utilized for treating samples and determining analytical results.

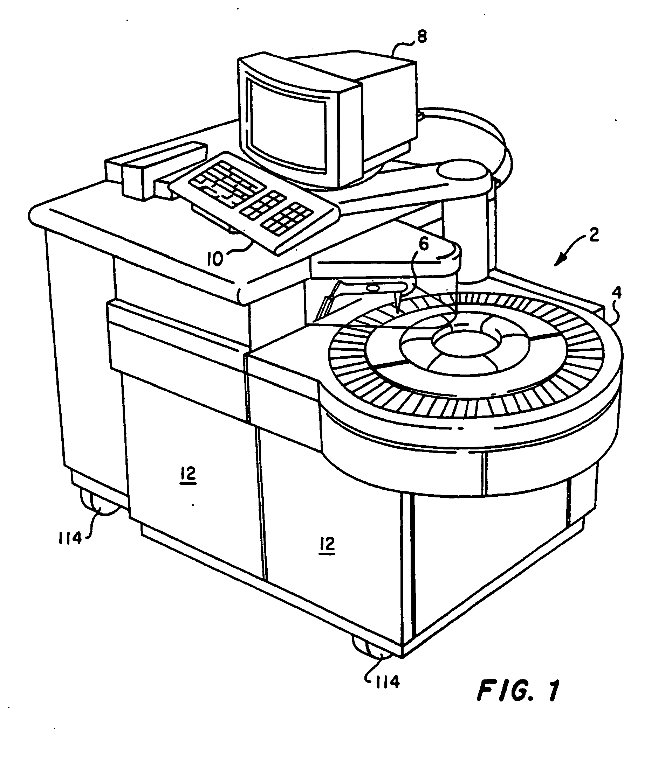

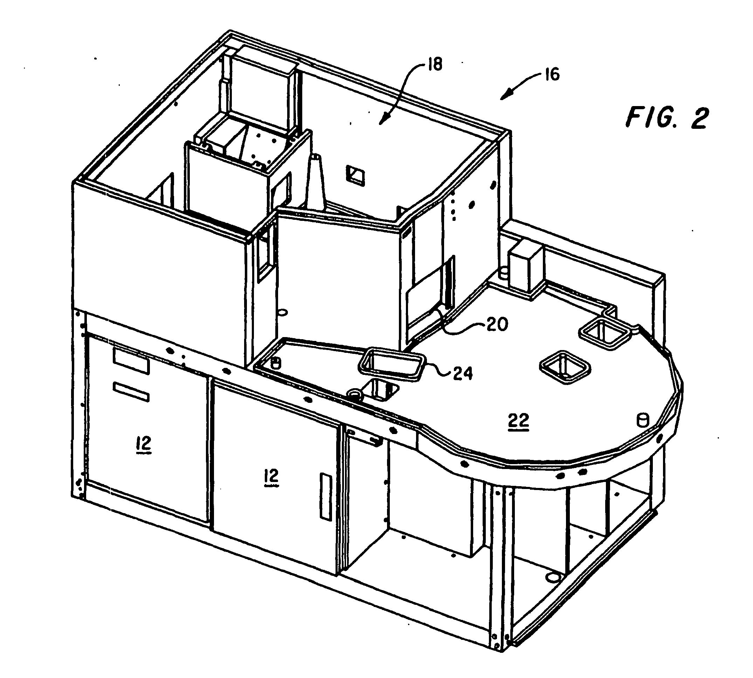

Referring to the drawings, FIGS. 1 and 2 present isometric views of the apparatus for the automatic immunoassay analytical system of the present invention. The system apparatus as it appears in FIG. 1 presents the system apparatus as used by the technician, with FIG. 2 illustrating an isometric view of the frame and cabinetry with component parts removed. The system apparatus of the present invention is identified generally as 2 in FIG. 1. The system apparatus 2 has an exposed front end carousel 4 which is serviced by a first transfer pipette mechanism 6 for kitting scheduled tests along with samples into a reaction vessel. The system provides a computer screen 8 and computer keyboard 10 along with access panels 12 for accessing storage and waste compartments. The system apparatus 2 is provided with rollers 14 for movement of the system apparatus within a laboratory complex as required. The freedom of movement of the system apparatus through rollers 14 is allowed since the system is fully self-contained but for power requirements.

Referring to FIG. 2, the system apparatus 2 cabinet frame 16 is illustrated with substantially all functioning components of the system apparatus removed. A controlled environment zone 18 is a closed unit during operation with light shielding and rigid control of airflow as well as temperature as opposed to the open front end carousel 4. The front end carousel 4 communicates with the controlled environment zone 18 through a transfer port 20. The front end carousel 4 is mounted to an aluminum base plate which rests on a support platform 22 and the first transfer pipette mechanism is mounted on means 24.

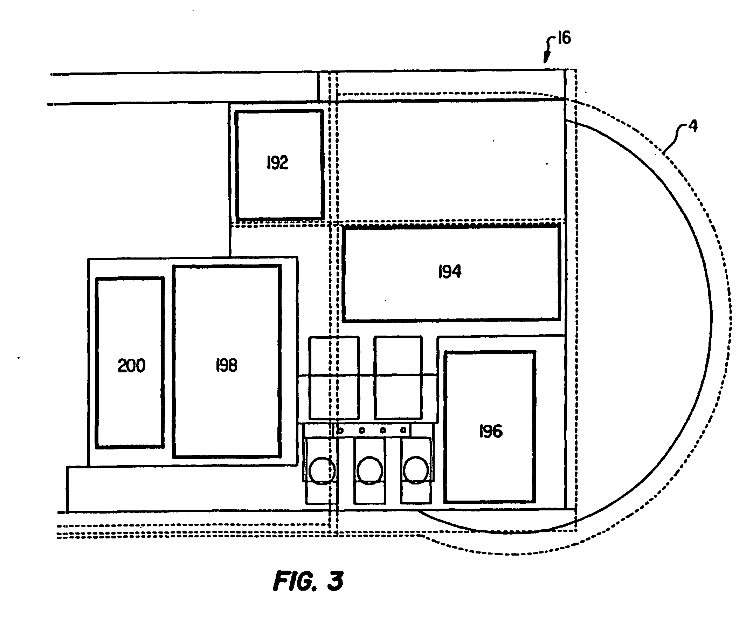

Referring to FIG. 3, the top plan view of the system apparatus 2 shows a portion of the cabinet frame 16 and the front end carousel 4 in partial phantom. This portion of the cabinet 16 also supports a power supply 192, a supply bottle 196, a solid waste container 198, and a liquid waste container 200. The supply bottle 196 provides buffers for the tests being performed, while the containers 198 and 200 provide storage for the processed waste material.

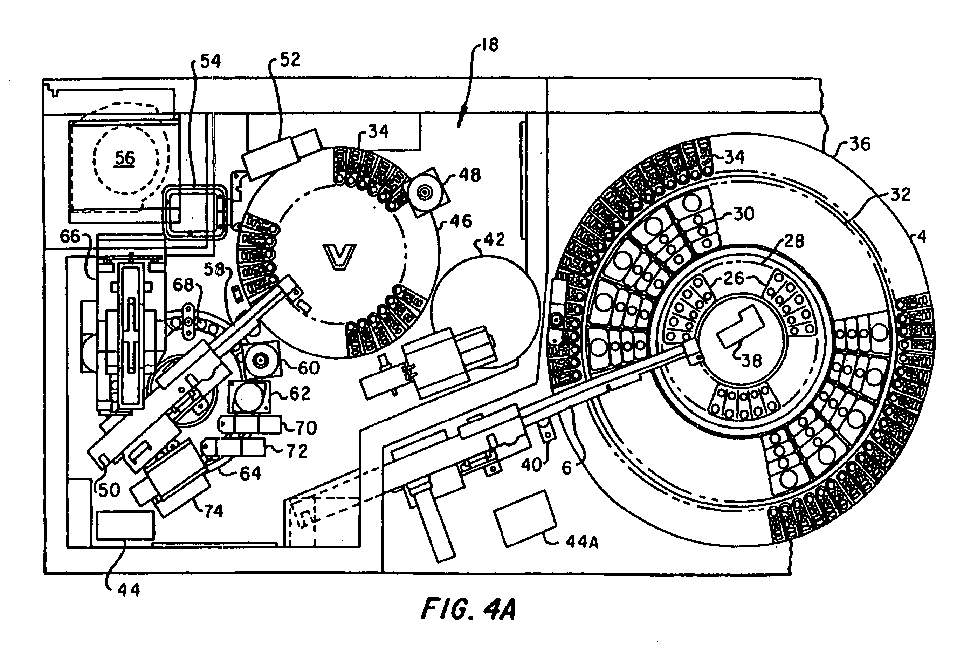

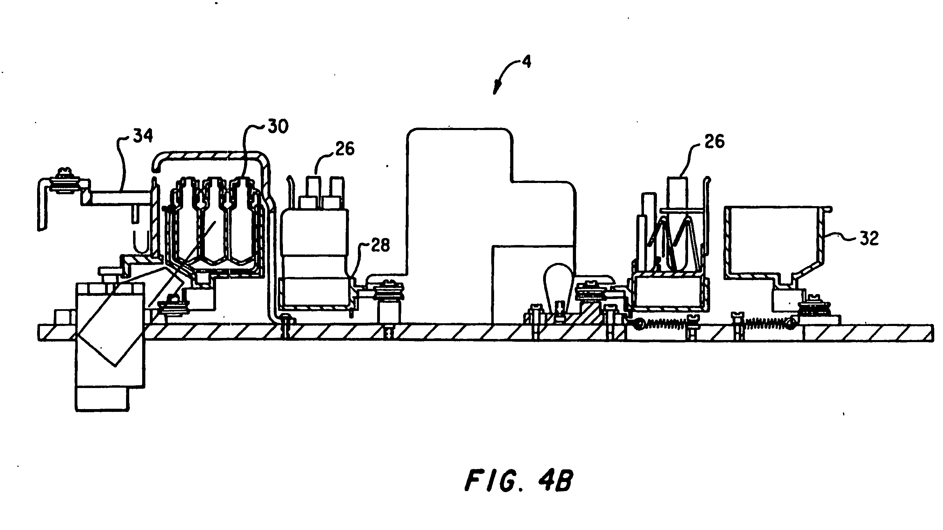

Referring to FIGS. 4A and 4B, components of the system apparatus are shown in more detail with relative positioning to further illustrate the process flow of the system apparatus. For example, sample cups 26 are mounted on a sample cup carousel 28 which is concentrically fitted within the front end carousel 4 along with reagent pack carousel 32 and reaction vessel carousel 36. The reagent pack carousel 32 is concentrically fitted between the sample cup carousel 28 and the reaction vessel carousel 36. The reagent pack carousel carries reagent packs 30 and the reaction vessel carousel 36 carries reaction vessels 34. The front end carousel 4 inclusive of the three front end carousels, the sample cup carousel 28, reagent pack carousel 32 and reaction vessel carousel 36 can by example contain the following capacities. The sample cup carousel 28 can hold 60 blood collection tubes, such as Vacutainer^{®} blood collection tubes, or 90 sample cups which are injection molded as one piece and can be provided with standalone base mounts. Standalone base mounts are suitable for technician handling and pipetting of samples into the sample cups. The reagent pack carousel 32 provides for 20 different reagent packs 30. The reaction vessel carousel 36 provides 90 reaction vessels 34.

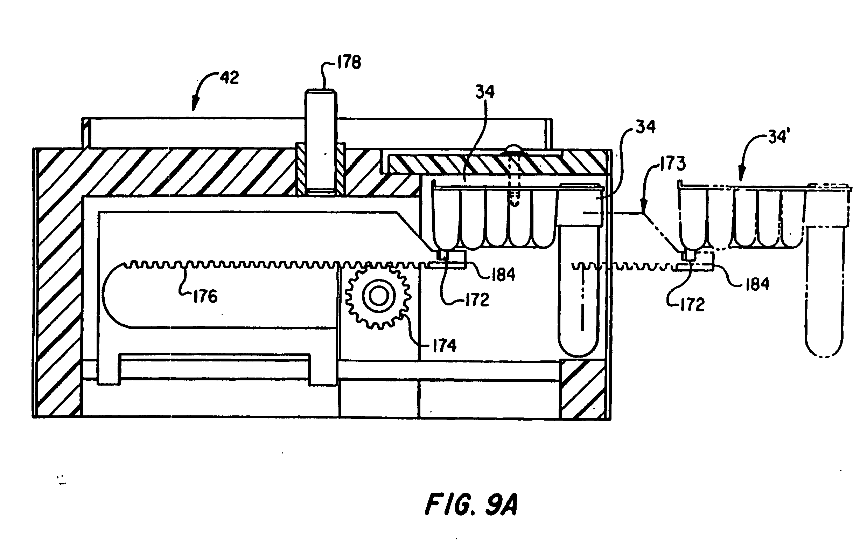

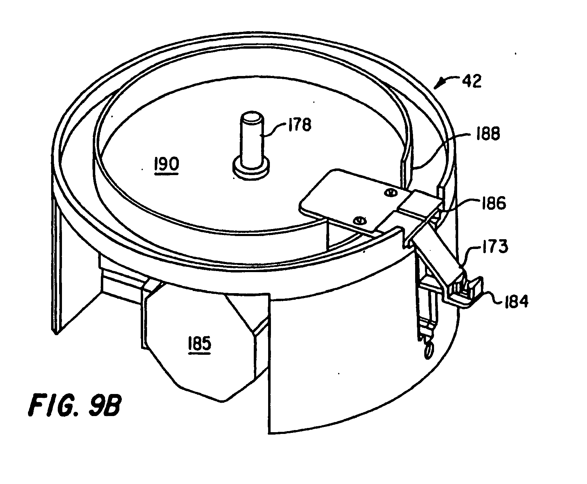

The front end carousel 4 has an operable bar code reader 38 for automatically identifying reagent pack carousel 32 and sample carousel 28. A wash cup 40 is provided for the first transfer pipette mechanism 6 for washing as required between transfer of various sample and reagents. The first transfer pipette mechanism 6 is utilized in kitting the various reagent pack liquid materials and sample into a reaction vessel 34. The reagents and the sample are properly kitted through means of the first transfer pipette mechanism 6 inclusive of pump means. The various carousels are rotated and aligned for kitting at the pipetting station. The kitted reaction vessel 34 is positioned by reaction vessel carousel 36 into the proper position for transfer to the transfer station 42. The reaction vessel 34 is transferred to the transfer station 42 through transfer means described below in more detail below (FIG. 9) wherein the transfer station 42 is then rotated to move the reaction vessel onto process carousel 46.

As shown, the process carousel 46 is driven by a stepper motor 48 and is serviced by a second transfer pipette mechanism 50. The process carousel 46 supported by three wheels for height control and control of any radial movement caused by irregularly shaped carousel elements. Both the FPIA and MEIA procedures utilize the system apparatus commonly up through and including the process carousel 46. The process carousel 46 includes FPIA processing 52 and FPIA processing lamp 54 for direct reading of FPIA analysis of kitted, pipetted and properly reacted reagents sample from the reaction vessel 34. The process carousel 46 holds, for example, 36 reaction vessels 34 and has a carousel diameter of about 12.5 inches (320 mm). The process carousel 46 moves the reaction vessel 34 between the transfer station 42, the second transfer pipettor mechanism 50, the point of pipetting, and the FPIA reader processing 52. The controlled environment zone 18, which includes the transfer station 42 and process carousel 46, provides FPIA processing with air circulation under temperature control by cabinet air circulation fan 56. A wash cup 58 for the second transfer pipette mechanism 50 is provided. The second transfer pipette 50 is utilized for adding reagents (pipetting) under conditions of incubation and timing to the sample in the FPIA test schedule reaction vessel 34 for FPIA processing.

MEIA processing can also utilize the second transfer pipette 50 for adding reagents to the sample before the reaction mix is added to MEIA cartridges 68 which are mounted on the auxiliary carousel 64, also referred to as the cartridge wheel carousel. The MEIA reagent mixed sample is transferred to the MEIA cartridge 68 by the second transfer pipette 50. The second transfer pipette 50 moves the pipette probe between the wells in the reaction vessel 34 on the process carousel 46 to the MEIA cartridge 68 on the auxiliary carousel 64 and to the wash cup 58. A rack-and-pinion drive through two axis stepper motor drives achieves precision drive on both the R and Z axis. Travel, for example, on the Z axis can be about 3 inches (75 mm) and on the R axis about 4. 5 to 5.0 inches (115-130 mm). The auxiliary carousel 64 holds, for example, 32 MEIA cartridges 68 and has a diameter of about 9.5

inches (141 mm). The auxiliary carousel 64 moves the MEIA cartridges 68 between various stations including the second transfer pipettor mechanism pipette point, the MUP dispense station 72, the MEIA washstation 70 and the MEIA reader 74 and the MEIA cartridge ejection point 62. The auxiliary carousel 64 is stepper motor driven and is carried by three wheels with one wheel located at the Z axis height control at the cartridge insertion point, the second wheel at the pipette point, and the third wheel at the MEIA reader in order to maintain the auxiliary carousel 64 within desired geometric relationships to these various functions.

MEIA cartridges 66 are loaded into a cartridge hopper 590 which feeds the MEIA cartridges 68 into the auxiliary carousel 64. The automatic feeding of the MEIA cartridges 68 is provided with a proper height adjustment of the cartridge 68 into the auxiliary carousel 64 as required by MEIA reading. The cartridge hopper 590 feeds individual cartridges 68 to the auxiliary carousel 64 and changes the axis of orientation of the cartridge 68 from horizontal to vertical by automatic means. Removal of the MEIA cartridges 68 is achieved through the use of an ejector 62 which operates through an ejection rod and forces the MEIA cartridge 68 from the auxiliary carousel 64 which is dropped into the solid waste container 200 (FIG. 3). The auxiliary carousel 64 is further serviced by a MEIA buffer heater and dispenser 70, MUP heater and dispenser probe 72, and MEIA reader 74. The MEIA cartridges .68 are removed from the auxiliary carousel 64 by a cartridge ejector 62 after the MEIA read has been completed.

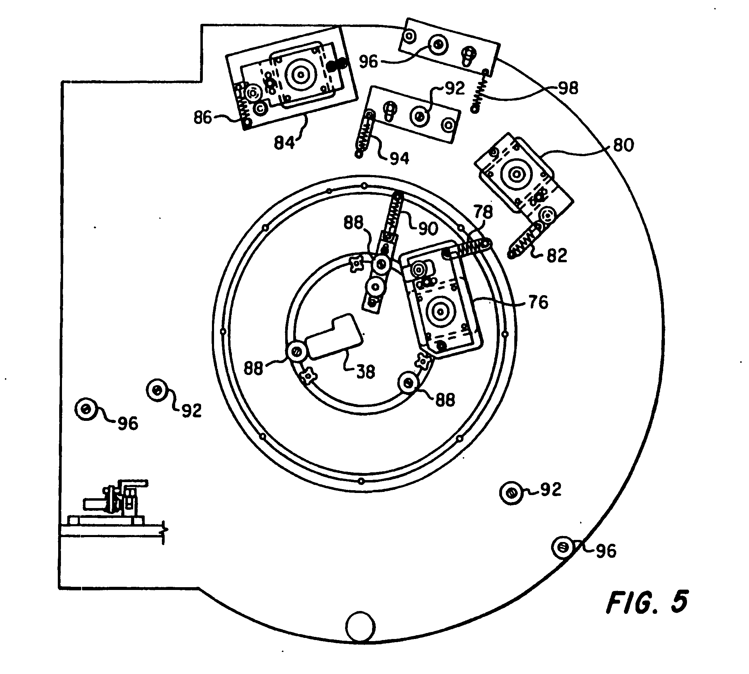

FIG. 5 provides a top view in isolation and partial section of elements of the drive and guide systems of the main carousel 4 with the various carousels removed. In FIG. 5 a sample cup carousel stepper motor 76 is shown mounted with mounting spring 78. The reagent pack carousel motor 80 is also shown with a mounting spring 82. The reaction vessel carousel motor 84 and mounting spring 86 are positioned to the exterior of the two inner carousels, i.e. the sample cups carousel 28 and the reagent pack carousel 32. Roller guides 88 are provided for the sample cup carousel 28 and a tensioning spring 90. The reagent pack carousel is provided with roller guides 92 and tensioning means 94. The reaction vessel roller guides 96 are also provided with spring elements 98, the purposes of the guide and these various spring elements being to maintain very finite tracking of the concentric carousels when motivated by the individual stepper motors.

Motion control for the system 2 is performed by 22 stepper motors of various sizes, some of which are identified herein. The specifications and operation of the stepper motors are described generally as follows, such description being sufficient for one skilled in the art. All the stepper motors are permanent magnet motors with 200 full steps per shaft revolution which is equivalent to 1.8 degrees revolution per step. A single stepping motor control system comprises the following:
(1) A step motor connected to a mechanism to move the mechanism as required.
(2) A driver which applies voltages to the step motor causing it to move in response to 3 control signals from control electronics, i. e. , an "Indexer".
(3) An Indexer which comprises electronics for controlling the motor by the driver. It determines move profiles, which include direction of rotation, number of steps to move and acceleration and velocity parameters.
(4) A home sensor is used for each step motor. The home sensor is used as a position reference by the Indexer and can also be used by the Indexer to check for errors.
(5) Encoders are used by the rotary devices, the carousels and transfer mechanism to verify correct movement. At the end of a move, the encoder count is checked to validate that the motor moved to the correct position.

The system microprocessor (CPU) is used to determine the distance, velocity and acceleration of a motor movement of the steppers. It transfers the information to the Indexer which then controls the movement. At the end of the movement, the Indexer then signals the system microprocessor (CPU) that the movement is complete. The system microprocessor (CPU) then checks the encoders to validate the movement if a rotary mechanism was being moved and checks the Indexer to verify it had detected no errors.

There are three indexer boards in each system 2. Each board is identical and can control up to eight stepper motors. Each board utilizes one slave microprocessor to provide the eight indexer functions on each board. Such a combination of functions is referred to as an "8-axis" indexer. Two of the indexer axes are not used. The indexer boards communicate to the system microprocessor (CPU) over a backplane VME bus. The Indexers have addresses that are modified by jumpers before installation into the system. This is the mechanism that allows otherwise identical boards to reside in the same system backplane VME bus. Each board is connected via the VME backplane bus to one cable per board that carries the indexer signals to the drivers. The Indexer provides a variety of movement profiles. Many of the step motor movements require that the speed of the motor be increased in a controlled fashion until the final velocity is reached. At some point in the movement, the speed must then be decreased in a controlled fashion. This process is called a "velocity profile" and can be done linearly, sinusoidally, or parabolically. The type of velocity profile executed is determined by the system microprocessor (CPU). The Indexers are available from vendors as "off the shelf" 8-axis indexers.

There are three PC boards used to provide the 22 separate motor drive circuits. Two of the boards are identical and referred to as the "stepper Drive" boards. Each of the Stepper Drive boards comprises eight functionally identical stepper driver circuits. They differ only in the current levels applied to each stepper motor. The current is controlled by a separate resistor in each driver circuit. The third board is called a "power I/O" board because it contains seven motor driver circuits and eight solenoid driver circuits. A single driver receives the following three inputs from an Indexer which controls its outputs to the step motor:
(1) Step input - for each step pulse input, the step motor will be moved one step,
(2) Direction input - constant level signal which controls the direction of motor rotation,
(3) Power Hi input - logic level input which causes the driver to apply maximum power to the step motor during movement. When Power Hi is not asserted, a lower power level is applied to the step motor to reduce heat and to reduce system power consumption when the motor is not being moved.

Each driver circuit has a pair of current setting resistors to set motor current level for Power High and to set a different motor current level when Power High is not asserted. There are two pairs of current setting resistors for each driver circuit. Additionally, each board has logic used to identify the position of the board in the card cage. There are two pins in the power backplane connectors which are used to encode each connector slot for the three boards that drive motors. By grounding or leaving unconnected, four combinations of two pins are possible. The board logic decodes the connector position and, through FET switches, each driver circuit than connects the correct pair of current setting resistors. The board output is only enabled if the Stepper Drive is plugged into one of the two connectors allocated for Stepper Drive boards. Each stepper drive circuit is known in the industry and available from most circuit vendors. The circuit is known as a "Bipolar chopper half-step driver". Although there are 200 "full steps" per shaft revolution, the motor can be driven in such a way as to cause the shaft to stop midway between the "full step" position. It can of course also stop at the "full step" positions, which provides a total of 400 steps per shaft revolution. This adds to the resolution of the moving mechanism and aids in reducing motor induced vibration.

The Power I/O board includes the seven stepper drive circuits and eight solenoid drivers as indicated above. Six of the stepper driver circuits are identical in function to those of the Stepper Drive boards. The seventh is functionally the same except that it is provided with less heat sink and therefore is limited to driving lower power motors. This circuit is used to drive the small ejector 62. There is only one pair of current setting resistors per driver circuit since there is only one Power I/O per system. Position decoding logic on the Power I/O enables outputs only when it is plugged into the connector designated for the Power I/O board.

The Home Sensors are fed into the Indexers and the Encoder circuits are fed into a VME general purpose board which provides counters for counting the encoder pulses and which also makes the counters available to the system microprocessor (CPU). At the beginning of a move, the system microprocessor (CPU) sets the appropriate encoder counter to zero. It then commands an Indexer to move the corresponding stepper motor the required number of steps. At the end of the move the system microprocessor (CPU) checks the encoder counter to verify that the motor did move the correct number of steps. There is a "window" of acceptability, such that the counter can be off by a few counts. If the counter is off by more than the permissible number of counts, an error is declared by the system microprocessor (CPU) and appropriate action is then taken by the system microprocessor (CPU).

The Power I/O board provides "chopper drives" to control various solenoid valves in the system. The system microprocessor (CPU) sets a bit of one of the digital I/O boards to energize a valve. The bit is optically coupled to the Power I/O solenoid drive circuit. The solenoid drive circuit then provides a 36V turn on voltage for approximately 300msec, after which the voltage is lowered to about 27 volts to reduce power dissipation and solenoid temperature rise. The lower voltage is achieved by applying the 36V in a chopped fashion such that the time average is about 27 volts, although the actual waveform is comprised only of 36V and ground signal levels. This is also known as pulse width modulation.

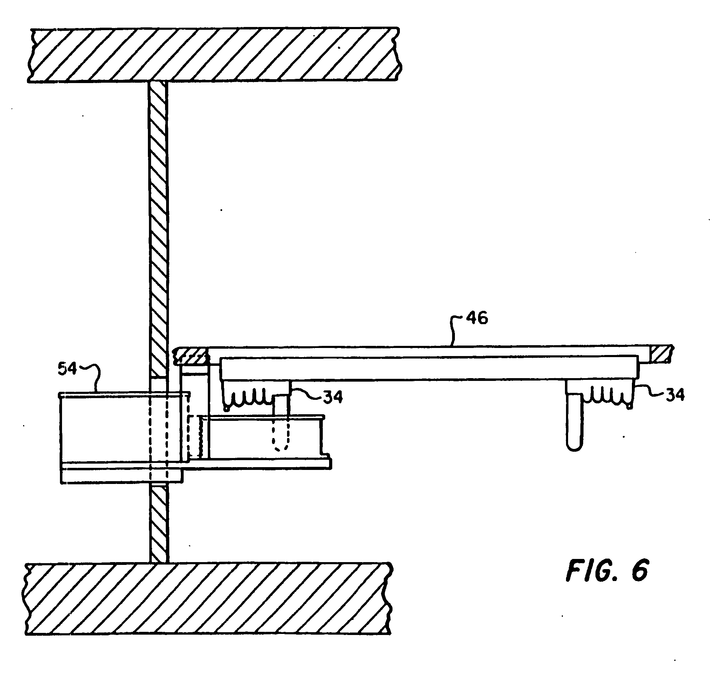

Referring to FIG. 6, the process carousel 46 is shown in an isolational cross-sectional side view. One reaction vessel 34 is at rest or nonoperative position and a second reaction vessel 34 is in position for FPIA read. The process carousel 46 is capable of bidirectional motion for timely movement of the various reaction vessels 34 to pipettor action, read, or transfer to and from the carousel. Up to about 36 or more reaction vessels 34 can be processed at one time on the process carousel 46 depending on diameter and sizing of the reaction vessels 34.

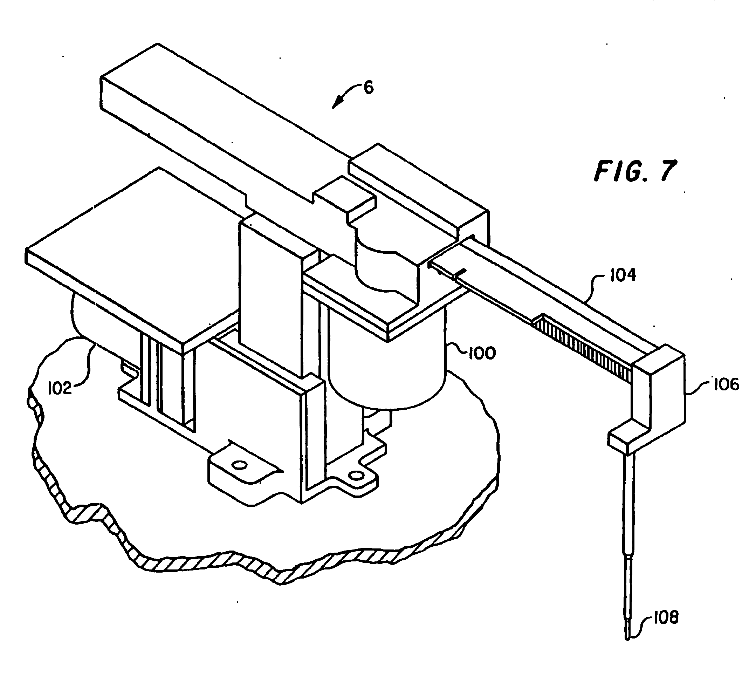

Referring now to FIG. 7, the first transfer pipette mechanism 6 shown in more detail includes a transfer pipette Z axis motor 102 which moves the probe arm 104, probe 106 and probe tip 108 in a vertical direction while transfer pipette R axis motor 100 drives the probe arm 104, probe adjustment means 106 and probe tip 108 in a horizontal motion. The first transfer pipette mechanism 6, sometimes labeled "Sample Probe Arm Mechanism", moves the probe between the sample cup 26, the reagent pack 30, the reaction vessel 34 and the wash cup 40. The wash cup 40 is used to wash the interior and exterior surfaces of the first transfer pipettor mechanism 6 probe. The drive of the first transfer pipette mechanism is a rack-and-pinion drive means along the Z and R axis by two-stepper motor drivers. A brake is provided to hold the Z axis position when power is lost, thus avoiding damage to the system apparatus. For example, the first transfer pipette mechanism can be designed to have a Z axis travel of about 3 inches (75 mm) and an R axis travel of about 11-1/2 inches (290 mm).

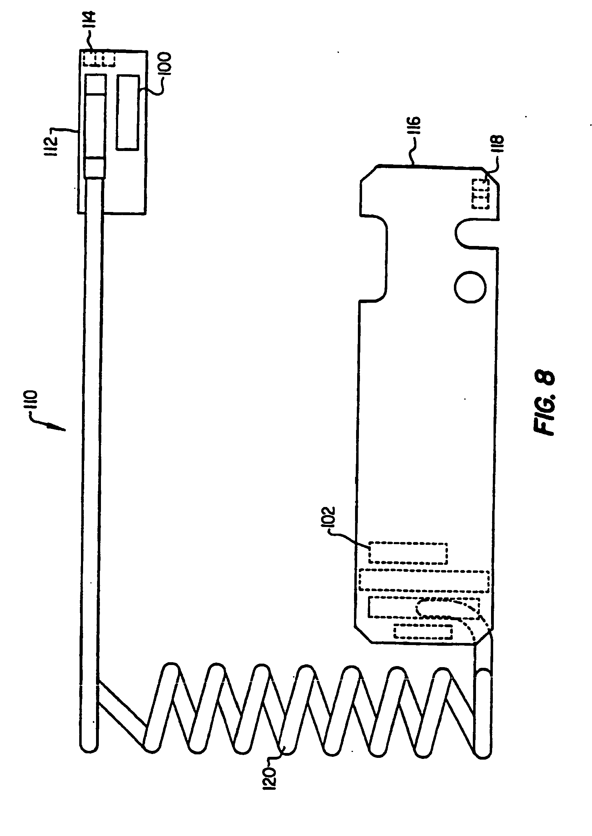

The first transfer pipette mechanism 6 and the second transfer pipette mechanism 50 are closely related in general system apparatus function and design, with variation on travel and size being the only substantial differences. Both units have a probe arm circuit 110 as illustrated by the schematic side view of FIG. 8. The schematic illustrates the R axis motor 100 and the Z axis motor 102 in relationship to an upper PCB 112 and a R axis home sensor 114. A lower PCB 116 is illustrated in relationship to the Z axis home sensor 118 with a coil cable 120 connecting the various elements.

The transfer station 42 plays a key role in apparatus and process function. Referring to FIGS. 9A and 9B, the transfer element at the transfer station 42 is shown engaging reaction vessel 34 by means of a reaction vessel transfer projection 172. The transfer arm 173 is projected out between reaction vessel elements of the reaction vessel carousel 36 and, by rotation of the transfer station 42, engages the reaction vessel transfer projection 172. By means of a transfer arm drive gear 174, the transfer arm 173 rack gear 176 moves the transfer arm 173 out and in relationship to the transfer station 42. The transfer station 42 has a rotation axis 178. A reaction vessel 34', shown in phantom, is shown as mounted on the front end carousel 4, reaction vessel carousel 36 being engaged by the transfer arm 173 by means of reaction vessel transfer projection 172. The reaction vessel 34' has a transfer handling means, i.e. transfer projection 172 which allows the transfer arm 173 of the transfer carousel to position an engagement means or pick 184 for engaging the reaction vessel 34' transfer projection 172. The reaction vessel 34 is illustrated onboard the transfer station by reaction transfer station 42 which moves the reaction vessel 34 between the front end carousel 4 and the process carousel 46. The transfer station 42 moves the discarded reaction vessel 34 from the process carousel 46 to the waste ejection station (not shown). The transfer station 42. is driven by a stepper motor drive and is supported by precision linear ball bearings and axis of rotation ball bearings.

### SYSTEM SCHEDULER

According to the present invention, the analytical system 2 is controlled by software executed by the system microprocessor (CPU) which also executes application software for generating and optimizing the tests being run on the analytical system 2 (hereinafter the "scheduler). The scheduler schedules the activities of assays that have been modelled by using a flexible protocol technology which enables the scheduler to minimize the time during which the mechanical components of the analytical system 2, i. e. , the resources, remain idle by properly sequencing the activities which comprise the assay. These activities can be, for example, pipetting (P), optical or other types of readings (R), cartridge washings (W), -and MUP dispensing (D), all of which are accomplished using the system's resources. The resources according to preferred embodiment of the analytical system 2 include the primary carousel 46, the auxiliary carousel 64, and the process pipettor 50. Generally, an activity uses only one resource, i.e., a reading (R), washing (W), or dispensing (D) at one station of a carousel. However, the pipetting (P) uses more than one resource, i.e., the pipettor 50 and one or both of the carousels 46, 64. The flexible protocol technology is of developmental software used by a chemist to model assays, such as the FPIA and MEIA assays, for execution by instrumentation software on the analytical system 2. When the chemist is modelling an assay, the flexible protocol inhibits any sequence of activities for the assay that will not run on the system 2. Thus, the system 2 never sees a corrupt assay because the flexible protocol rules are already imbedded in the assay protocol.

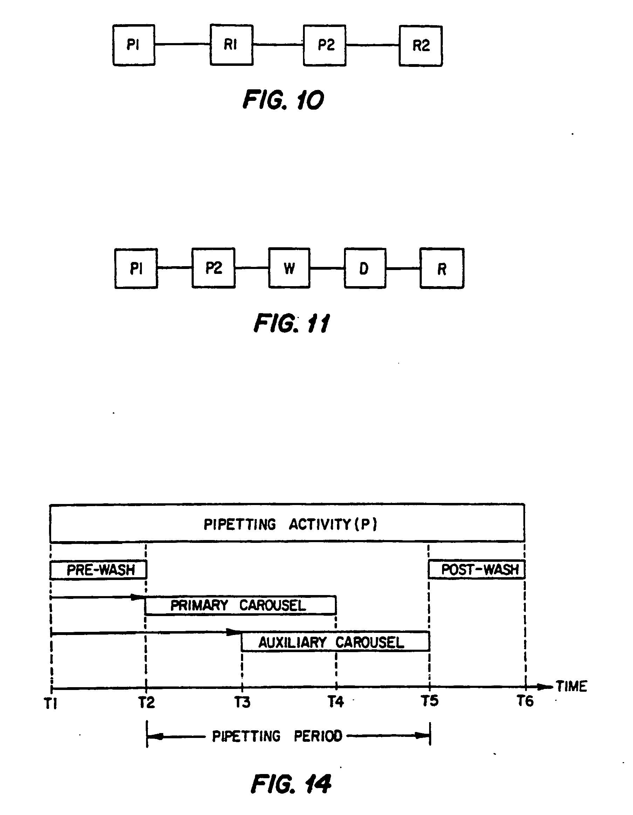

The flexible protocol technology used to model an assay specifies (1) what activities are to be performed for a particular assay and the order in which the activities are to be performed, (2) the incubation periods between the activities, (3) how the activities are to be performed and their time durations, and (4) the equilibration and evaporation constraints for each assay. With respect to the first specification of the flexible protocol, the activity protocol, FIGS. 10 and 11 show activities to be performed by an assay and the order in which the activities are to be performed. Referring more specifically to FIG. 10, a sequence of four activities is shown: a first pipetting activity (P1), a first reading activity (R1), a second pipetting activity (P2), and a second reading activity (R2). This sequence of activities can be, for example, the sequence for the FPIA assay as described in more detail below. Referring to FIG. 11, a second sequence of activities is shown including two pipetting activities (P1) and (P2), a washing activity (W), a dispensing activity (D), and a reading activity (R). This sequence represents, for example, the MEIA sequence of activities also described in more detail below.

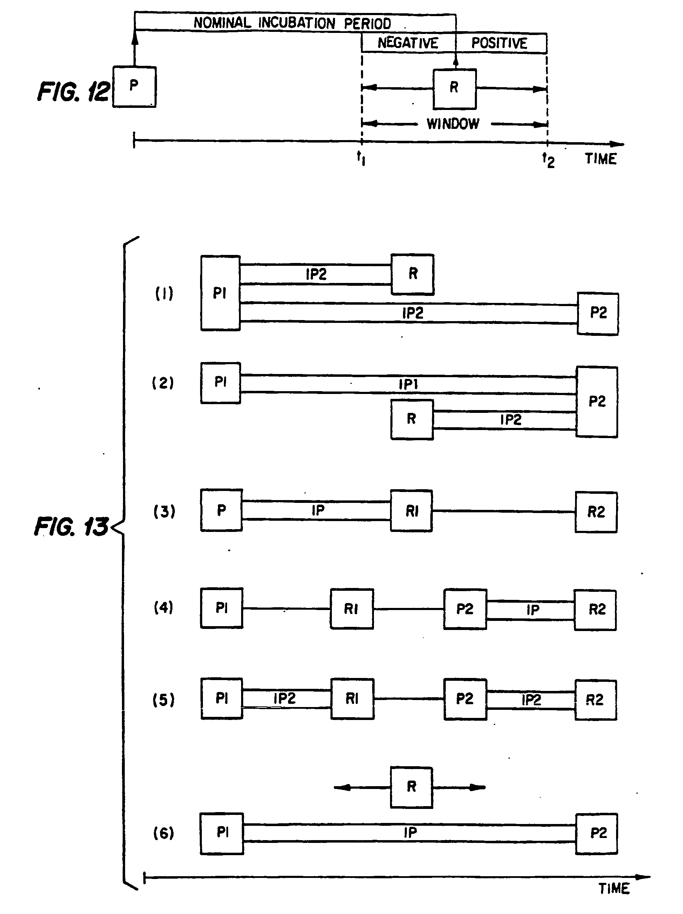

The second specification of the flexible protocol, i.e., the incubation schedule, relates to the incubation periods between the activities as shown in FIGS. 12 and 13. The incubation schedule defines the time periods between the activities, i.e., the time dependencies between the activities. More specifically, the incubation period includes a nominal time lapse between two activities, i.e., the nominal incubation period (NIP), and the amount of time that it can be varied, i.e., the incubation window. The incubation window includes the amounts of time which the scheduler may add to or subtract from the nominal incubation period (NIP) to optimize throughout of the system 2. Referring more specifically to FIG. 12, the nominal incubation period (NIP) defines the time between the pipetting activity (P) and the reading activity (R). The nominal incubation period can be reduced by the amount of time indicated by the negative portion of the window, in which case the reading activity (R) will occur sooner, or increased by the amount of time indicated the positive portion of the window, in which case the reading activity (R) will occur later. Thus, the scheduler has enough flexibility to vary the incubation period from time T1 to time T2 to optimize the task being performed on the system 2.

Referring to FIG. 13, six incubation schedule rules are shown with respect to time. These rules describe the proper and improper sequence of activities associated with incubation periods. Rule (1) specifies that one activity can initiate more than one incubation period. More specifically, the first pipetting activity (P1) initiates a first incubation period (IP1) constraining the reading activity (R), as well as a second incubation period (IP2) constraining the occurrence of a second pipetting activity (P2). However, the converse is not permitted. Referring to Rule (2), only one incubation period can terminate in an activity. In other words, one activity cannot be constrained by more than one incubation period. For example, the second pipetting activity (P2) cannot be constrained by the two incubation periods (IP1) and (IP2) initiated by the first pipetting activity (P1) and the reading activity (R), respectively. The flexible protocol technology would invalidate this sequence. Referring to Rule (3), the last activity of an assay must be a termination point for an incubation period. Thus, the flexible protocol technology would invalidate the second reading activity (R2) because it does not terminate an incubation period, unlike the first reading activity (R1) which terminates the incubation period (IP) initiated by the pipetting activity (P). Such "post-incubation" activities are not permissible. Referring to Rule (4), activities not constrained by an incubation period that occur prior to the first incubation period are permissible. These "pre-incubation" activities such as, for example, the first pipetting activity (P1) and the first reading activity (R1), are permissible activities in an assay even though they are not constrained by an intervening incubation period, as long as they occur prior to the first incubation period (IP) which constrains the second pipetting activity (P2) and the second reading activity (R2). Although pre-incubation activities are permissible, Rule (5) specifies that activities constrained by an incubation period cannot precede a pair of unrelated activities constrained by a second incubation period. More specifically, referring to the specific example for Rule (5), even though the pipetting activity (P2) and reading activity -(R2) are constrained with respect to each other by the second incubation period (IP2), they float in time because neither are constrained by either the first pipetting activity (P1) or the first reading activity (R1). Finally, Rule (6) states that an activity can float unconstrained between two other activities constrained by an incubation period. More specifically, the first and second pipetting activities (P1) and (P2) are constrained by the incubation period (IP) which does not constrain the reading activity (R). The reading activity (R) is a float activity which is not constrained by time, but only constrained by its order with respect to the other two activities, i.e. , it must occur after the first pipetting activity (P1) and before the second pipetting activity (P2).

The third specification of the flexible protocol technology, the activity description, specifies how activities are to be performed and their time duration, i.e., the timing protocol, as indicated above. Referring more specifically to FIG. 14, the timing protocol for a pipetting activity (P) is shown. This particular pipetting activity (P) is similar to the one used for the MEIA assay which requires three resources of the analyzing system 2, including the primary carousel 46, the auxiliary carousel 64, and the process pipettor 50. The pipetting activity (P) consists of 6 events, commencing with a pre-wash event at time T1 when the application software determines that the pipettor 50 must be cleaned of contaminants from a previous pipetting activity. If, however, the system software knows that the previous pipetting activity will not contaminate the current pipetting activity (P), the pre-wash event will not occur. The pre-wash event will be described in more detail below.

The duration of the pre-wash period is known to the system software which commences execution of the pipetting activity (P) relative to the second event related to the primary carousel 46. The second event occurs at time T2 corresponding to the amount of time that elapses before the reaction vessel 34 is available on the primary carousel 46 for the pipettor 50. The reaction vessel 34 will not be available until other activities have been completed and the primary carousel 46 has been repositioned if necessary. At time T2, the pipettor 50 begins aspirating fluid from the reaction vessel 34. When fully aspirated, the pipettor 50 moves into position with the auxiliary carousel 64. The pipetting period for the primary carousel 46, time T2 to time T4, includes the time necessary for the pipettor 50 to aspirate fluid from the reaction vessel 34 and the amount of time necessary for the pipettor 50 to move clear from the primary carousel 46. The third event occurs at time T3 representing the amount of time that elapses before the cartridge 68 is available on the auxiliary carousel 64 for the process pipettor 50. At time T3, the auxiliary carousel 64 is in position for the pipettor 50 to begin dispensing the fluid into the cartridge 68. Events 4 and 5 occur at times T4 and T5, respectively, and represent the time after which the carousels 46, 64 are no longer needed for the current pipetting activity (P), and are available for subsequent activities. When the auxiliary carousel 64 becomes available, the pipetting period from time T2 through time T5 is complete. After the pipetting period, the pipetting activity (P) concludes with the completion of a post-wash cycle at time T6. Whether or not the post-wash cycle is necessary is dependent on whether the current pipetting activity (P) would contaminate the next activity to be performed.

The foregoing description clearly shows that the flexible protocol technology enables the scheduler to properly sequence assay activities, compress incubation periods and perform other functions so that the analyzing system 2 is optimized to operate continuously at high throughput rates. The flexible protocol technology is to be distinguished from a "fixed" protocol, such as the one disclosed in European Patent Application 410,645 published January 30, 1991, which describes an analyzer restricted to a fixed cycle that cannot be optimized. When the scheduler begins the process of scheduling a test, the process is broken into two stages: (1) the scheduler reviews the assay activities just described and the fixed system activities, such as for example reaction vessel 34 loading and unloading activities and cartridge 68 loading and unloading activities, to ensure that execution of the test will not clash with the activities of other tests in process before the test is kitted, and (2) an attempt to perform each test activity prior to its original scheduled execution time within the parameters of the assay protocol to minimize the amount of time resources are idle and increase the throughput of tests in the system.

In the first stage, the operator chooses the order that tests are prepared to run on the system 2 by selecting the placement of samples 26 on the system 2. The sample 26 placed closest to the pipette station is the first sample prepared to run on the system 2. To guard against evaporation, a test will not be prepared until the scheduler ensures that all resources used by the test's activities will be available at the required times set forth in the test's assay protocol. Preparation of the next test in line is postponed when activities of other tests in progress are using resources at the time required by an activity of the next test. The sample preparation area of the system 2 remains idle until the next test is successfully scheduled without conflict. For example, if a pipetting activity (P) requiring twenty seconds must be performed sometimes during a two-minute window within 3-5 minutes after a kitting activity, preparation is postponed until the pipetting activity can be accomplished somewhere in that window. When proper scheduling of the next test can be achieved, the test will be prepared and transferred into the process area.

The second stage of the scheduling process is to optimize the workload for each system resource to minimize both the resource's idle time and the time required to perform the resource's workload. Once tests are transferred into the process area, the scheduler optimizes the existing schedule for each resource. At predetermined intervals, the scheduler examines the next interval of work for each resource. If there is any idle time in this interval, the scheduler attempts to minimize the idle time by rearranging the resource's workload to eliminate idle time, providing the activities remain within their allowed incubation windows. When optimization of this interval is complete, this section of the workload is performed by the resource at the designated times. The scheduler continues to prepare samples as long as there are samples 26 on the system 2 that have tests ordered to be run. Optimization of the resources' workloads will continue until all tests transferred into the system have finished processing.

Another feature of the invention provides a procedure for interrupting the scheduler's preparation of samples 26. According to this feature, the operator of the system 2 identifies a sample 26 for priority handling (hereinafter the "stat sample") in both the front-end sample area and the processing area of the analytical system 2. The operator chooses the order that tests are prepared to run on the system 2 by selecting the placement of samples 26 on the sample carousel 28. The sample 26 placed closest to the pipette station is the first sample prepared to run on the system 2. This pattern of sample 26 preparation is interrupted whenever the operator places a stat test on the system 2. Whenever a stat test is ordered, the system 2 will finish preparing the test on the current sample, and then move directly to the stat sample to prepare all its tests. To guard against evaporation, sample preparation will not begin for a test before proper scheduling of the test's activities in the processing area is ensured.

The system scheduling algorithm is also modified for stat processing. The scheduling algorithm used for normal tests attempts to maximize the number of tests processed in the instrument each hour. This occurs by allowing sufficient time between test activities to enable other tests' activities to be performed in these gaps. The scheduling approach used for stat tests attempts to process this one test in the shortest amount of time possible. Each activity of a stat test is scheduled at the earliest possible time of execution as defined in the test's assay definition. When all activities of a test are guaranteed proper scheduling in the system 2, sample preparation of the test will begin. After all tests on the stat sample are prepared, the system 2 will return to the sample 26 it was working on before it serviced the stat sample.

Stat tests receive special consideration in the processing area when there is idle time in a resource's workload. At predetermined intervals, the scheduler examines the next interval of work allocated to each resource in the processing area of the system. If there is any idle time during this interval, the scheduler attempts to minimize it by rearranging the resource's workload as described above in greater detail. Test activities scheduled for this resource that can be performed earlier than they are currently scheduled, as defined by their assay protocols, are moved forward to fill the idle time. Stat test activities are the first candidates to be pulled forward in the workload, thus further decreasing the amount of time needed to process the stat test in the instrument. Although stat tests receive special scheduling treatment, it does so without negatively affecting the system's throughout.

### SMART WASH

The system additionally provides a method and apparatus for identifying analytical interactions which are likely to occur between various steps in a random access analytical system, particularly steps involving pipetting sequences in which the likely interactions are carryover by or cross contamination of test samples or reagents. The method and apparatus determines when those interactions are likely and allows for random access processing even in those situations (that is, the method and apparatus allows the system to react in a different manner in instances in which those interactions are likely, than in instances in which the interactions are less likely). It can do this because the system software (in particular, the scheduler software) is able to randomly insert and remove pipetting events from the processing timeline in order to control carryover or cross-contamination. By so inserting and removing pipetting events, the system varies test sample and reagent wash volumes to correspond with wash volumes necessary for the particular test samples or reagents being processed in order to eliminate the possibility of interactions.

The system is capable of controlling carryover or contamination by utilizing a simple matrix, as described below in detail. The matrix is set up in order to relate the particular pipetting steps performed by the system to the potential of those steps for carryover and contamination. Based upon values determined by the system from that matrix, the system modifies wash volumes between pipetting steps to minimize wash volumes but to allow sufficient wash volumes to eliminate contamination or carryover. The apparatus and method of the invention is particularly useful when incorporated in the automated analytical system particularly described herein, which system is capable of simultaneously performing two or more assays on a plurality of test samples in a continuous and random access fashion.

In order to reduce carryover and contamination, the present system and method, in effect, looks at the sources that create the problem. This can be better understood in concept by considering the general scheme of the scheduler software and the pipetting and wash steps. Since each pipette step can result in carryover or contamination, as well as possibly be sensitive to carryover, the system provides simple categories for the contaminating potential of each pipette step and then identifies to which of those categories each assay step is sensitive. This is where the aforementioned matrix comes into play. The matrix is set up to identify when carryover or contamination is likely, based upon preceding and succeeding pipetting steps scheduled by the scheduler software. The apparatus and method, based upon values from the matrix corresponding to the preceding and succeeding pipetting steps, causes the analytical system to respond with appropriate wash characteristics to eliminate the possibility of undesirable carryover or contamination when they appear likely. In operation, the analytical system is automatically cleaned to a nominal level, suitable for eliminating carryover or contamination in the typical instance. In the prior systems, it was necessary that the system be cleaned at an extreme level which would eliminate carryover or contamination in the worst cases. The system, however, provides for extra washing in those cases in which the system software identifies, based on the scheduled sequence, the situation of a potentially contaminating step occurring before a sensitive step. In the instance of that combination, the software causes the system to activate a predetermined super wash that is adequate for controlling the carryover in those extreme instances.

This approach reduces the amount of washing performed by the system because sensitive steps do not necessarily always follow contaminating steps and so the super wash is not always employed. In short, the method of the system accounts for both the situation where normal wash is required and the situation where a greater wash is required, and determines which type wash is necessary in any instance, even though it is not possible, due to the random and continuous access nature of the system, to know a priori when carryover is or is not likely to occur. The system also allows for pipette steps to be removed or inserted into the processing timeline as required due to the random access nature of the system, and maintains the system to eliminate the possibility of a contaminating situation. Even further, the system allows the software to adjust the required washing without having to manipulate other pipetting steps in the processing timeline, even in a system which allows continuous operation.

The method and apparatus are designed to minimize wash fluid consumption by the instrument by having the system software track some basic information relating to the pipetting steps that immediately precede and follow any given step on the timeline. Since they involve the interaction of all assays with one another, it is preferred that all assays use the same approach to cleaning the pipette within their protocol. Unlike wash systems and methods previously described, the method (1) reduces wash volumes to help the management of onboard liquid and waste; and (2) reduces washing times to help improve throughput.

In particular, probe wash control in systems previously described was provided by recommendations for post washing after each pipetting block as follows:

| | | | | |
|---|---|---|---|---|
| Pipetting Sequence 1 | Post Wash 1 | ----- > | Pipetting Sequence 2 | Post Wash 2 |

The basic pipette cleaning is provided as before, i.e., with a post wash which should be sufficient to control carryover for most of assay steps that might follow it. However, if the recommended post wash is inadequate for controlling cross-contamination or carryover to the following step, then a prewash is incorporated for that second step as follows:

| | | | | | |
|---|---|---|---|---|---|
| Pipetting Sequence 1 | Post Wash 1 | ---- > | Pre Wash 2 | Pipetting Sequence 2 | Post Wash 2 |

The prewash is variable and has two levels, nominal and super. The nominal prewash is the volume that should be used all the time. When carryover is possible, the super wash would then be used. Typically, the nominal wash volume would be zero. Since the methodology software feature identifies when carryover is possible, the post wash volumes used across the system can be reduced in value from what they were prior to the method, whereby each assay is no longer required to be cleaned well enough to control the worst case carryover situation. Additional wash needed to control carryover will be added through the super wash when the software identifies a carryover potential.

### Parameters, Tables and Terminology for Smart Wash

The method preferably utilizes five parameters to describe each pipetting step, two index values and three wash parameters, wherein (i) the two index values are *sus* (susceptibility to contamination) and *con* (probability to contaminate); and (ii) the three wash parameters are *nom* (nominal prewash number), *sup* (super prewash number), and *pw* (post wash number). The wash parameters are not volumes. The wash parameters are numbers that identify washes in a wash library as described below.

| Current Wash Library | | | |
|---|---|---|---|
| Wash Number | Total | | |
| | Volume | Waste | Washcup |
| | | | |
| 0 | 0 ml | - | - |
| 1 | 2 | 1 ml | 1 ml |
| 2 | 2.5 | 1 | 1.5 |
| 3 | 3 | 1 | 2 |
| 4 | 3.5 | 1.5 | 2 |
| 5 | 4 | 2 | 2 |
| 6 | 4.5 | 2 | 2.5 |
| 7 | 5 | 2 | 3 |
| 8 | 1 | no | yes |
| 9 | 2 | no | yes |
| 10 | 3 | no | yes |
| 11 | 4 | no | yes |
| 12 | 5 | no | yes |

The *sus* and *con* parameters are used to flag the probability for carryover or cross-contamination to occur. They are related to each other through the matrix of the present method.

The matrix of the present method contains only 0's and 1's, corresponding to off and on, respectively; 0 = no probability for carryover; 1 = probability for carryover does exist.

### Method Matrix

| | | sus parameter | | | |
|---|---|---|---|---|---|
| | | 1 | 2 | 3 | |
| *con* parameter | none 1 | 0 | 0 | 0 | |
| | w/airgap | 2 | 0 | 1 | 1 |
| | w/o airgap | 3 | 0 | 0 | 1 |

*con* description
- 1: not contaminating (no sample)
- 2: aspiration of sample or sample mix with airgap
- 3: aspiration of sample or sample mix without an airgap
*sus* description
- 1: not susceptible to contamination
- 2: sensitive to aspiration of sample or sample mix with an airgap
- 3: sensitive to aspiration of sample or sample mix without and with an airgap

For example, a pipette block is susceptible to all sample pipetting (sus index = 3). For a preceding pipette step which has a *con* index of 1 (matrix value = 0), no super wash is performed. For a preceding pipette step which has a *con* index of 2 or 3 (matrix, value = 1), the super wash is performed.

The matrix of the present method provides information to the software that the probability for carryover or cross-contamination exists, but it does not provide information to the software as to what volumes to use for a wash step, which is instead provided from the *nom, sup* and *pw* parameters. The matrix of the present method may be expanded should other contaminating species in addition to sample be defined.

The *con* parameter and the *pw* numbers describe to the software what state the probe is in prior to the next pipetting step. The rules established for identifying these parameters for pipetting steps are requirements for all assays to follow.

The *con* and *pw* parameters are defined as follows:

| | Description | *con* value | *pw* number/vol |
|---|---|---|---|
| Not contaminating (no sample) | | 1 | (2 ml) |
| Asp of sample/sample mix with airgap | | | 2 |
| | *<= 50 µl aspirated | 1 | (2 ml) |
| | *<= 100 µl aspirated | 3 | (3 ml) |
| | *<= 150 µl aspirated | 5 | (4 ml) |

Aspirating > 150 µl of sample or sample mix with an airgap is discouraged because of the necessity to use excessive washing.

Asp of sample/sample mix without an airgap 3, use the same *pw* values as above.

"*" indicates the level of sample carryover present when the method is not utilized (post wash only) is 10 ppm or less with the above recommendations. In all cases, the minimum allowable *pw* value is 2 ml wash.

The *sus, nom* and *sup* parameters are under the control of the assay protocol. It is to be understood that any criteria established for identifying these parameters are recommendations, and that the assay protocol developer will best know which pipetting sequences are sensitive to carryover, which sequences create the problem and what wash volume is necessary to clean the probe.

Nominal and super washes are used for a susceptible pipette block for control of carryover. Use 0 for Wash Library numbers 8 through 12, where only wash to washcup is needed: *nom* = 0 - no nominal prewash is preformed; *nom* = 8 to 12 -- use Wash Library numbers 8 through 12 (1-5 ml wash-washcup); *sup* = 0; no super prewash is performed; *sup* = 8 to 12 -- use Wash Library numbers 8 through 12 (1-5 ml wash-washcup).

Because of scheduling constraints, the super wash volume may not be greater than the minimum post wash (2 ml), plus the nominal wash; if it is necessary to use more super wash volume, the nominal wash should be increased as well. For example, if the nominal wash is 0 ml, super wash may only be 0, 1 or 2 ml. If the required super wash is 4 ml, nominal wash must be at least 2 ml.

The minimum post wash requirement and super wash volume constraint not only ensures proper scheduling, but also protects the system from a highly contaminating step being "hidden" from a susceptible step because a simple step sits between them on the timeline that needs only a minimum wash. The minimum post wash requirement guarantees that the probe will be properly cleaned when that susceptible step is to be pipetted.

The kitting center is treated as one pipette block. Carryover experiments have shown that a post wash of at least about 2 ml is sufficient to clean the probe to a carryover level of 1 ppm or less when sample is kitted first followed by wash and pipetting of reagents. Total wash following sample should be about 4 ml total wash before next kitting activity. Contamination of the reagent bottle following sample will come from the outside of the probe. This is reduced to insignificant levels by wash to waste cup, e.g., 200 to 1,000 µl, followed by from between about 1 ml to about 2 ml wash to the wash cup.

### Chemiluminescent Test

According to another embodiment, methods and apparatus are provided for measuring a chemiluminescent signal produced by an immune complex formed with analyte from a test sample such as chemiluminescent homogeneous immunoassays and chemiluminescent heterogeneous immunoassays. According to one embodiment, a chemiluminescent detection signal is produced by an immobilized immune complex comprising antibody coated magnetic particles which are separated by a magnetic field. According to such method, an optical cuvette containing the immune complex bound to magnetic particles suspended in solution is utilized wherein a magnetic field is imposed along the wall of the cuvette to perform the separation. The particles which contain the immune complex are washed, a trigger reagent is added, and the resulting chemiluminescence from the labeled particles is detected and measured in the cuvette using a chemiluminescent detection system. According to another method, analyte is captured in a liquid phase employing, for example, microparticles, polyionic capture agents and the like, having a binding affinity for the analyte wherein the capture analyte is subsequentially immobilized by a porous element and a chemiluminescent signal is then chemically excited and detected. Accordingly, such method within a continuous and random access analytical system advantageously employs fast fusion rates in solution to provide highly sensitive assays for a wide range of analytes. Such methods are particularly useful with an automated, continuous and random access analytical system apparatus as described herein, and which can further include fluorescent and chemiluminescent assay processing on the same platform. Such automated analytical system of the present invention is capable of simultaneously performing two or more assays on a plurality of test samples in a continuous and random access fashion. In particular, the automated immunoassay analytical system apparatus of the invention can be viewed as a microprocessor based system of integrated subassemblies with different groups of assays being run through separate and changeable software modules. The microprocessor based system uses robotic arm pipetters with two degrees of freedom and bidirectional rotating carousels to process samples. Critical assay steps such as incubations, washes and specimen dilution are performed automatically by the instrument as scheduled.

In particular, the automated, continuous and random access analytical system apparatus is capable of performing chemiluminescent assays such as described in commonly owned U. S. Patent No. 5,089,424 and U.S. Patent Application No. 206,645 filed June 14, 1988. As described, at least two types of chemiluminescent assays are possible by the system apparatus, magnetic particle capture and microparticle membrane capture. Chemiluminescent assay processes operate by taking advantage of light scattering properties of certain conjugate molecules. Those processes may be either homogeneous or heterogeneous. In the homogeneous assay processes, light absorption of a liquid medium containing particular antibodies is measured. The antibodies are then reacted with antigens to form a precipitate. The antigen-antibody precipitate solution is then subjected to the light which is absorbable by the antibody. The difference in the light absorption measured by the two light absorption steps is determined and that difference is indicative of the presence or absence of agglutination of antibody and antigen. Because the agglutination reaction reduces the concentration of antibody in solution, the light absorption by the liquid medium is reduced in proportion to the degree of antigen-antibody precipitate formed. As is typical of methods and apparatus for performing homogeneous assays, these procedures do not require separation of a solid phase from the reaction mixture for further analysis. In heterogeneous assay processes, on the other hand, solid phase material must be separated. In these processes, small amounts of clinically significant compounds in a liquid test sample are quantitated by focusing a light source on the sample. For example, a fluorescent light source might be used. In that case, fluorescent particles in the sample cause fluorescent conditions, the intensity of which is related to the intensity of the light beam and the concentration of fluorescent particles in the sample. A detector employed in connection with the light beam senses photons forming the fluorescent emissions of the particles when excited by the light beam. Solid phase material in the.sample must be subsequently separated from the mixture for further analysis and before the fluorescent emissions can be detected and measured.

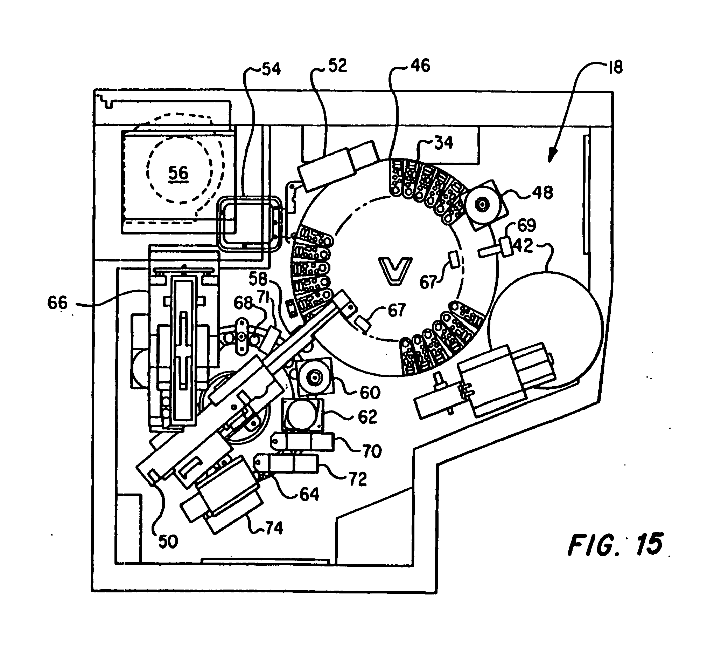

FIG. 15 is a top plan view of the automated analytical system in section with component covers removed to show the automated analytical apparatus in detail and relative position of the two types of detection systems utilizing chemiluminescent assay technology, a magnetic particle capture system and a microparticle membrane capture system, which may be employed in the present invention. In one of such detection systems, the process carousel has two magnetic separation stations 67 and a chemiluminescent reader detection module 69 for magnetic particle capture incorporated thereon for providing chemiluminescent magnetic particle capture assays. In the other one of the detection systems, the cartridge wheel carousel has mounted thereon a chemiluminescent reader 71 for providing microparticle membrane capture assays.

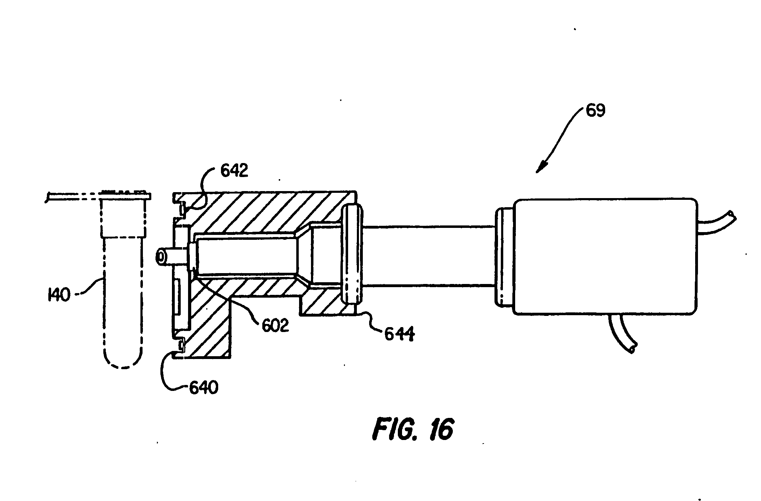

A depiction in cross-sectional view of a signal detection module 69 for use in the magnetic particle capture system 67, 69 is shown in FIG. 16. The detection module 69 comprises a light guide 602. The module 69 is mounted horizontally in a housing 638 and positioned near the disposable cuvettes 140 (shown in phantom) on the reaction carousel 46 (not shown in FIG. 16). In this position, the signal detection module 69 can take readings of the contents of each cuvette 140 as it passes the module 69.

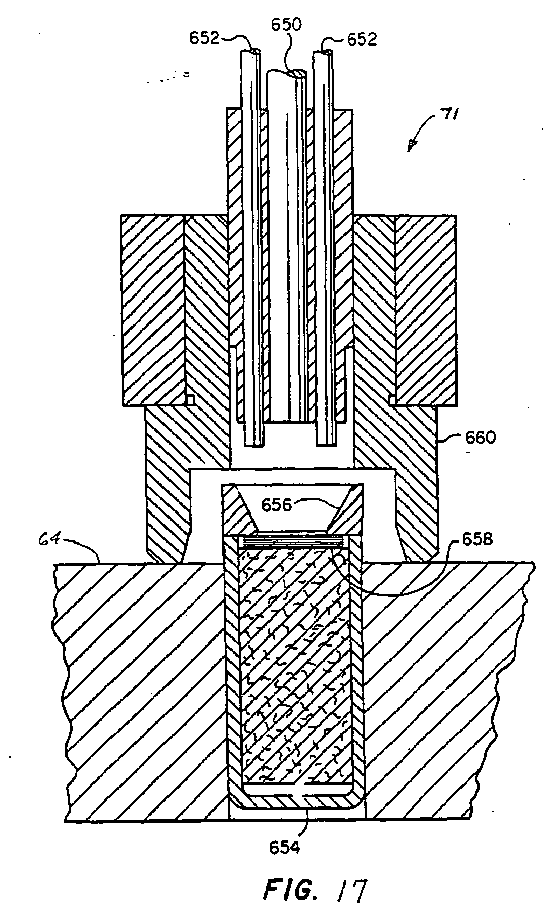

In FIG. 17, a depiction of a cross-sectional view of a signal detection module 71 for use in the microparticle membrane capture system is illustrated. The detection module 71 includes a light pipe 650. On either side of the light pipe 650 is positioned injection conduits 652. The module 71 is mounted vertically above the fiber cartridges 654 of the reaction carousel 46 (not shown in FIG. 17) to position the module 71 for detection of contents of each cartridge 654 as it passes the module. The module 71 also includes a light shield 660 which serves to shield environmental light from interfering with the reading. The cartridge 654 employed in this type system is a container which has a funnel-shaped aperture 656 to the cartridge 654 and a solid, porous element 658, preferably in the form of a fibrous matrix.

It is to be expressly understood that each of the magnetic particle capture system 69 and the microparticle membrane capture system 71 shown in FIG. 16 and 17, respectively, is intended as exemplary of those type systems. Other systems and arrangements and configurations are possible. The operation of any such system will, however, operate in about the same manner. Chemiluminescent released photons from the contents of the cuvette 140 or cartridge 654, as the case may be, are transmitted through a light pipe 602, 650 of the detection module 69, 71 to a photomultiplier tube (not shown). The module 69, 71 and photomultiplier tube assembly measures the chemiluminescent signal of the contents of the cuvette 140 or cartridge 654, as applicable.

### LIQUID LEVEL SENSING

The system includes a unique system and method for sensing fluid levels in the various sample containers of the automated analytical system. The fluid level sensing system detects whenever the automated pipette probe contacts a liquid. The system detects amplitude changes in a near-radio frequency. (RF) signal which is radiated by the probe and received by a series of antennas located below the various sample containers. The system can be thought of as detecting a change in capacitance between the probe and the applicable antenna when the probe contacts liquid. The system continually monitors the capacitance of the probe in air and detects a quick change in capacitance in response to the probe contacting liquid.

A significant feature of the fluid level sensing system is that liquid is reported only when both signal amplitude and rate of signal change indicate that the probe has contacted liquid. Previous systems which utilized signal amplitude to detect liquids reported liquid detections based only on signal amplitudes exceeding a preset threshold. Therefore, these systems were adversely affected by changes in signal amplitude induced by changes in temperature, humidity, aging of parts, parts variation, and most significantly, probe position in relation to other components in the automated analytical system. These conditions caused previous systems at times to falsely indicate the presence of fluid, or conversely, to fail to detect fluid presence. Basing liquid detections on both signal amplitude and rate of change of signal amplitude greatly reduces the number of such false or failed detections.

Some previous liquid level detection systems detected an electrical phase shift of a sinusoidal signal present on the pipette probe whenever the probe contacted a liquid. These phase-shift systems were limited, however, to analytical systems which utilized only de-ionized water in the fluid line to the probe. The use of signal amplitude for liquid detection enables the use of a conductive diluent, such as saline solution, in the fluid line to the pipette probe.

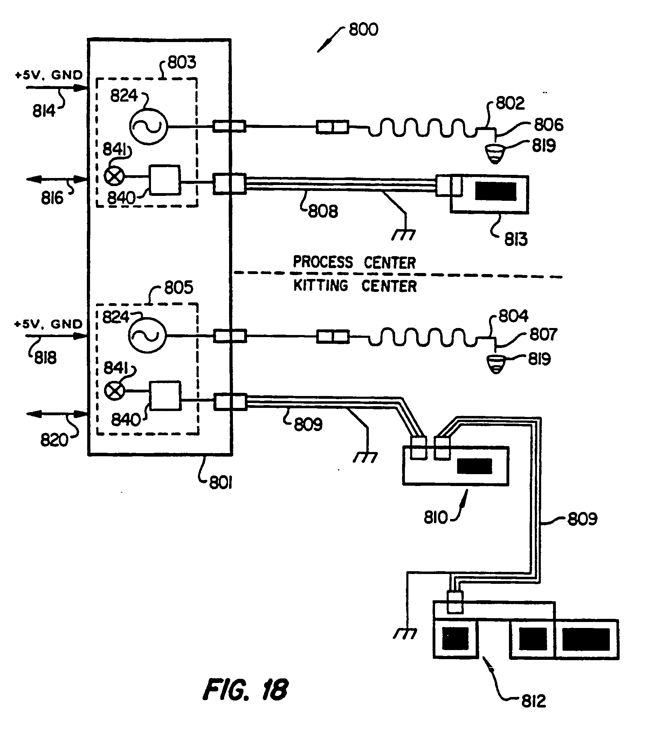

FIG. 18 is a simplified block diagram of the preferred embodiment of the liquid level sensing system 800 in connection with an automated analytical system. A liquid level sensing circuit board 801 is utilized to monitor pipette probes 806 and 807 when enabled by the automated analytical system computer (not shown), and to stop probe movement when the probes have contacted liquid. The liquid level sensing circuit board 801 mounts in a standard VME card cage, receiving only about +5V and ground from the VME bus at connections 814 and 818. DC/DC converters (not shown) on the board generate local operating voltages, isolating the board from the VME bus. Control signals to and from the board are routed to system I/O boards (not shown) through connections 816 and 820.

The board 801 contains a process liquid level sensing circuit 803 and a kitting liquid level sensing circuit 805, each completely independent of the other. The process liquid level sensing circuit 803 is dedicated to liquid detections by probe 806 in the process center, and the kitting liquid level sensing circuit 805 is dedicated to liquid detections by probe 807 in the kitting center.

The liquid detection systems in the process center and in the kitting center are essentially identical, and liquid detections occur in the same manner in each system. Therefore, the following description, although describing the liquid detection system in the process center, is equally applicable to the.kitting center.

Each of the two circuits 803 and 805 is controlled by a "CALIBRATE" signal from the analytical system computer, and each circuit provides two output signals, " READY" and "DETEST". In operation, CALIBRATE is set except when level sensing is desired. Calibration is performed by an auto-zero circuit 811 described in more detail below. The probe 806 is placed over a liquid sample container 819, preferably immediately over the fluid, and the analytical system computer sets desired gain bits which compensate for the various sizes of sample containers 819. When the circuit is calibrated so that its output signal level is zero, CALIBRATE is de-asserted and READY is asserted. The probe is then moved toward the sample container 819 until liquid is encountered, at which time DETECT is set. The analytical system computer receives the DETECT signal and, in turn, signals the motor controller (not shown) to stop vertical probe movement. DETECT remains set as long as the probe 806 is in liquid. When the probe is removed from liquid, DETECT is de-asserted, but will reset if liquid is contacted again. When the probe is withdrawn from the liquid, and fluid sensing is no longer required, CALIBRATE is again asserted. In CALIBRATE mode DETECTS do not occur, being disabled logically, regardless of the analog signal received.

A coax cable 802 carries an RF transmit signal from a low impedance driver signal source 824 on the sensing system circuit board 801 to the probe 806. A receiving antenna 813 is mounted in a stationary position below each rotating carousel, and beneath the area where liquid sensing is desired. In the kitting center, liquid detections occur in several locations; thus antenna 810 and antenna array 812 are mounted below the reaction vessel 34, the reagent pack 30, and the test sample segment container 26. The antennas are connected to the sensing system circuit board 801 by triax cables 808 and 809.

The RF signal is generated by the low impedance driver signal source 824, at a frequency of approximately 125 kHz, and is applied to the probe 806 through the coax cable 802. The RF signal then couples across the air space between the probe 806 and the receive antenna 813, located below the liquid sample container 819. In operation, when the probe 806 is lowered and contacts liquid, the signal from the probe to the antenna.increases slightly above that received when the probe is in air. The signal increases because the liquid surface, in effect, becomes part of the transmitting probe, increasing the amplitude of the transmitted signal and redirecting the probe's electromagnetic field toward the receiving antenna 813.

The signal is coupled from the probe 806 to the antenna 813 primarily by an electrical field, which can be mathematically modeled by capacitance. The transmission media may be considered as a small capacitance from the probe 806 to the receiving antenna 813. This type of level sensing may therefore be referred to as capacitance level sensing. Since the electrical field is actually part of an electromagnetic field radiating from the probe, the sensing device may also be referred to as an "RF" (radio frequency) sensing system, although the actual frequency employed is several octaves below standard radio frequencies.

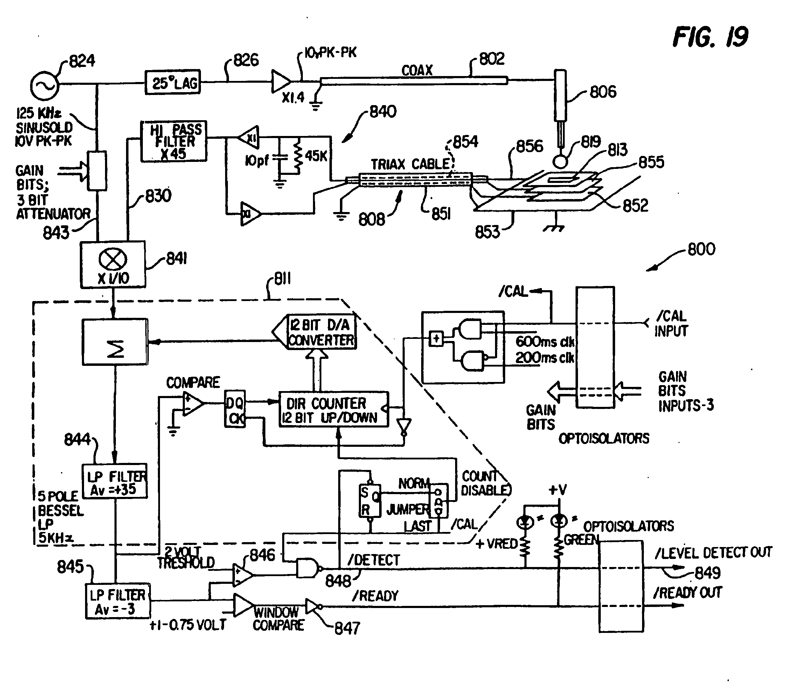

FIG. 19 is a more detailed block diagram of the liquid level sensing system 800 in connection with an automated analytical system. The fluid level sensing system 800 includes a synchronous (heterodyne) receiver which includes an amplitude detector 841 and a low pass filter 845. The receiver provides exceptionally narrow-band reception of the electrical signals detected by the antennas. In the synchronous receiver, the amplitude detector 841 multiplies the incoming signal 830 by a reference signal 843 in order to enable the extraction of amplitude information. The signal source 824 also uses the reference signal 843 to generate the transmitted signal 826, therefore both the transmitted and received signals are of substantially the same frequency. The incoming signal must also be substantially in phase with the reference signal.

After the incoming signal 830 is multiplied by the reference signal 843, the output of the amplitude detector 841 is passed through the low pass filter 845 to extract the amplitude information desired. The filter employed in the receiver of the preferred embodiment is a Bessel linear phase filter, which demonstrates minimal or no overshoot, and minimal or no ringing.

The liquid detection system 800 also includes an autozero circuit 811 which enhances signal detection. As the distance from the probe 806 to the antenna 813 changes, and as the probe approaches components within the automated analytical system with dielectric constants higher than the surrounding air, the level of the signal reaching the antenna 813 slowly changes. The autozero circuit 811 enables the fluid sensing system 800 to detect fluid with a very small increase in the received signal strength (approximately 0. 2 pf) because the increase when the probe contacts liquid occurs very rapidly compared to the change that occurs when moving the probe 806 toward the antenna 813 in air. The autozero circuit 811 nulls out signals which slowly change in amplitude, and reports only rapidly changing signals which exceed a predetermined threshold value.

The autozero circuit timing is such that the output of the circuit is maintained at about zero when the probe 806 is stationary or moving vertically. Changes in signal amplitude which occur slowly, -such as those caused by the probe approaching other components of the automated analytical system, are therefore reduced below the predetermined threshold value by the autozero circuit, and are not reported as liquid detections even if the amplitude variations exceed the threshold value. A rapid signal increase may occur in less than 200 microseconds due to fluid contact by the probe. When a rapid signal increase occurs, the autozero circuitry allows the output from low pass Bessel filter 844 to increase. The signal then passes through a second low pass filter 845 and is applied to a 2-volt simple fixed threshold 846. If the signal does not exceed the threshold value, the liquid detection system is maintained in the READY mode at 847. If the increase caused by fluid contact is sufficient to exceed the threshold 846, then a digital bit is output asserting DETECT at 848. At that time, the autozero circuit 811 is disabled. The DETECT signals are routed to the system motor control board at 849, so that when fluid is detected, the motor control board (not shown) can immediately stop the probe movement.

Still referring to FIG. 19, it may be seen that the fluid sensing circuit 803 is referenced to system ground in the immediate vicinity of its associated receiving antenna 813. As noted previously, the circuit 803 is connected to its antenna 813 by the triax cable 808 which, in the preferred embodiment, is about ten feet in total length. The outermost conductor of the triax cable 851, connects to a grounding plate for the antenna 852, and to a system baseplate 853, providing the ground reference for the circuit. The inner shield of the triax cable 854 is a "driven shield". The inner shield 854 is connected at one end to a driven shield plate for the antenna 855, and at the other end to the shield output side of a signal and driven shield circuit 840. The signal from the antenna 813 is carried by the inner conductor 856 to the input of the signal and driven shield circuit 840. The signal and driven shield circuit 840 acts as a buffer which, in turn, drives the inner shield 854. This reduces the effective capacitance of the cable 808 and antenna 813 by a factor of about sixty. The total capacitance of the antenna and ten-foot cable is normally about 600 pf. The signal and driven shield circuit 840 effectively reduces this capacitance to about 10 pf. This reduction greatly simplifies the detection of the 0.2 pf increase in signal strength that occurs when the probe 806 contacts liquid.

Bessel filters are used in the transmit circuits for repeatability and in the receive circuit for minimum ringing due to noise spikes, resulting in minimum noise levels within the system. Sharper filters have potentially high overshoots and actually result in a higher noise and ripple level.

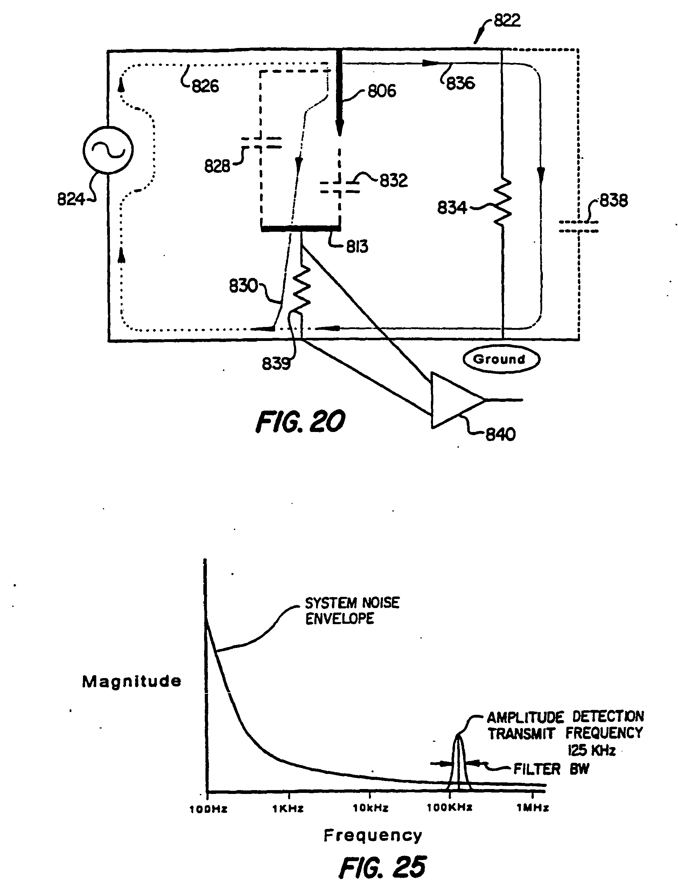

FIG. 20 is a simplified schematic diagram showing the current flow through the fluid level sensing system 800. Total current 826 flows from the signal source 824 to the probe 806 where it is divided into two paths. In one path, current 836 leaves the probe and flows through diluent to ground, returning to the signal source 824. The diluent is represented by diluent resistance 834 and diluent coupling capacitance 838. Separately, a much smaller current 830 enters the probe 806 and couples through space to the receive antenna 813. Capacitor 828 represents the capacitance of the probe 806 in air. When the probe contacts liquid, additional current flows through the additional fluid capacitance 832, added by the increased surface area of the liquid. The wavelength of the transmitted signal is small compared to the geometries within the automated analytical system, therefore almost all of the coupling from the probe 806 to the antenna 813 is by the electric field. By applying a low impedance transmit signal to the probe and receiving the signal with a separate antenna, shunting effects of conductive diluent in the probe plumbing are avoided. It should be noted that the signal and driven shield circuit 840 (FIG. 19) measures only current from the antenna 813, not the current through the diluent.

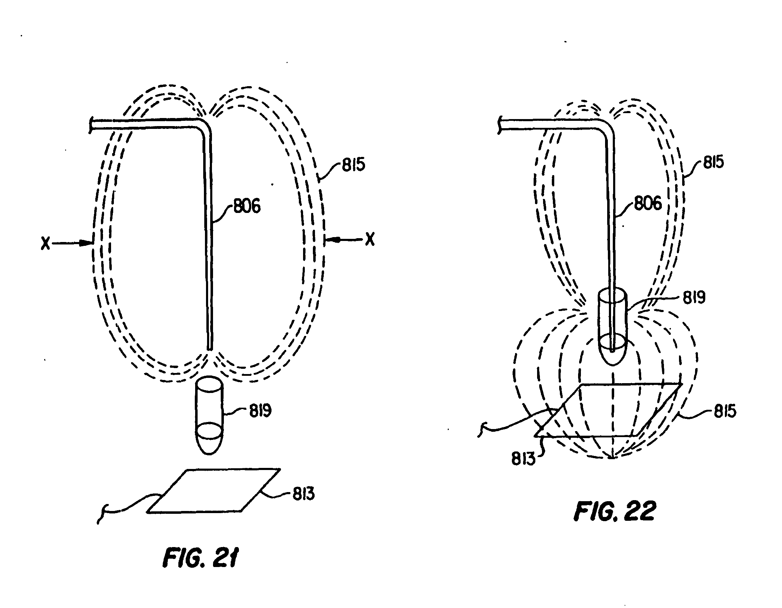

FIG. 21 is an illustration of the geometries between the probe 806, its electromagnetic field 815, a liquid sample container 819, and the antenna 813 when the probe is in air. The antenna 813 is positioned directly below the liquid sample container, along the extension of the longitudinal axis of the essentially linear probe 806. As shown in FIG. 21, the electrical signal 815 radiated by the probe in air is strongest on a plane X-X which is perpendicular to the longitudinal axis and at the center of the probe' s length. There is a null Y in the electrical signal along the extension of the longitudinal axis. Therefore, when the probe 806 is in air, very little signal reaches the antenna 813.

FIG. 22 is an illustration of the geometries between the probe 806, its electromagnetic field 815, a liquid sample container 819, and the antenna 813 when the probe contacts liquid. A greater signal level is radiated along the extension of the longitudinal axis than from the probe in air (see FIG. 21). Therefore, the signal level received by the antenna 813 rises significantly when the probe 806 contacts liquid.

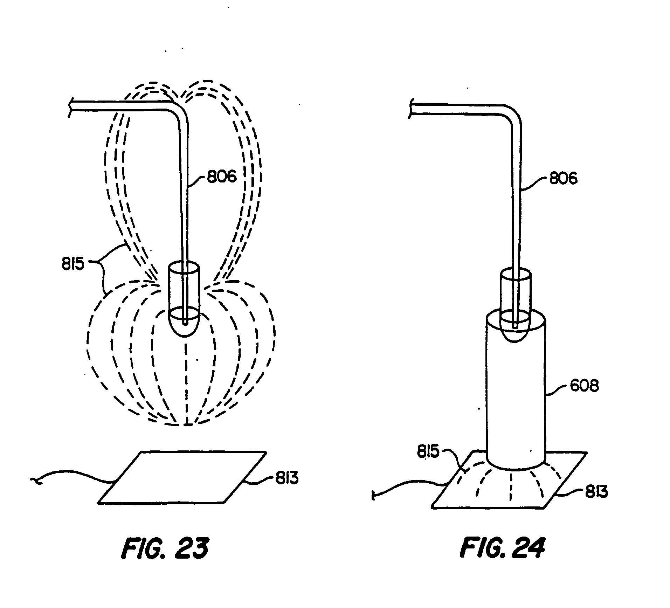

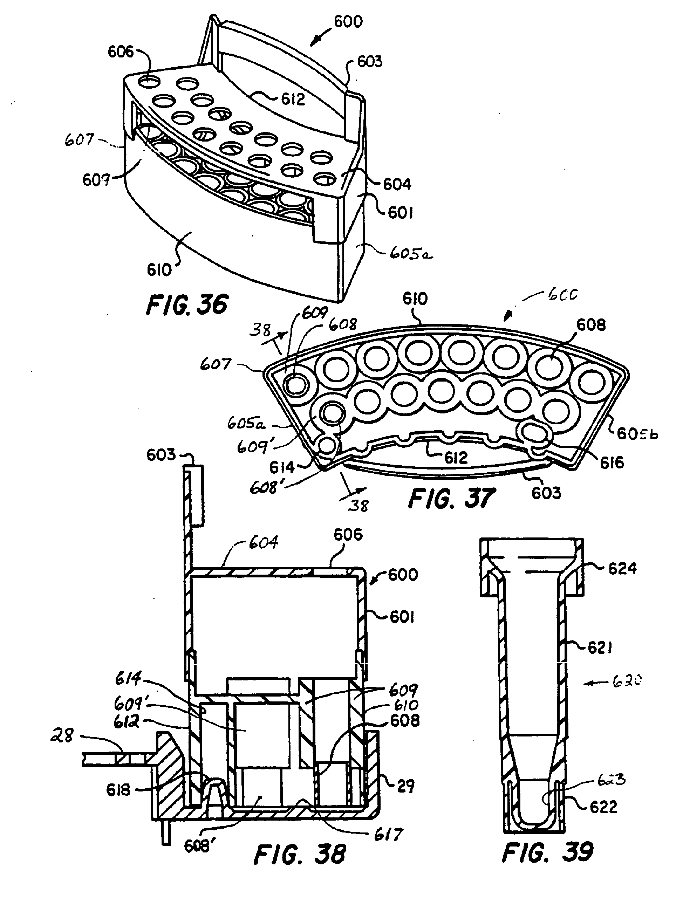

FIG. 23 illustrates that even the electromagnetic field 815 generated by the probe in liquid may be insufficient to generate enough signal at the antenna 813 to trigger a detection if the distance from the liquid sample container 819 to the antenna 813 is too great. This condition may arise when short sample cups are used in the sample segment container 600 (FIG. 36). Therefore, the sample segment container 600 is equipped with fluid level sensing sleeves 608 mounted directly below the positions where short sample cups are inserted. The sensing sleeves 608 may be constructed of aluminum or any other electrically conductive material.

As shown in FIG. 24, the sensing sleeve 608 functions to channel the electrical signal 815 from the probe/liquid combination to the vicinity of the receiving antenna 813. The sleeves 608 are mounted at a height at which the top of the sleeve approximately coincides with the top of the liquid in the sample cup. If the sleeve is mounted too high, it may cause false fluid detections due to channelling of the signal from the probe in air. If the sleeve is mounted too low, fluid detections will be missed because the sleeve will not adequately function to channel the electrical signal to the antenna 813.

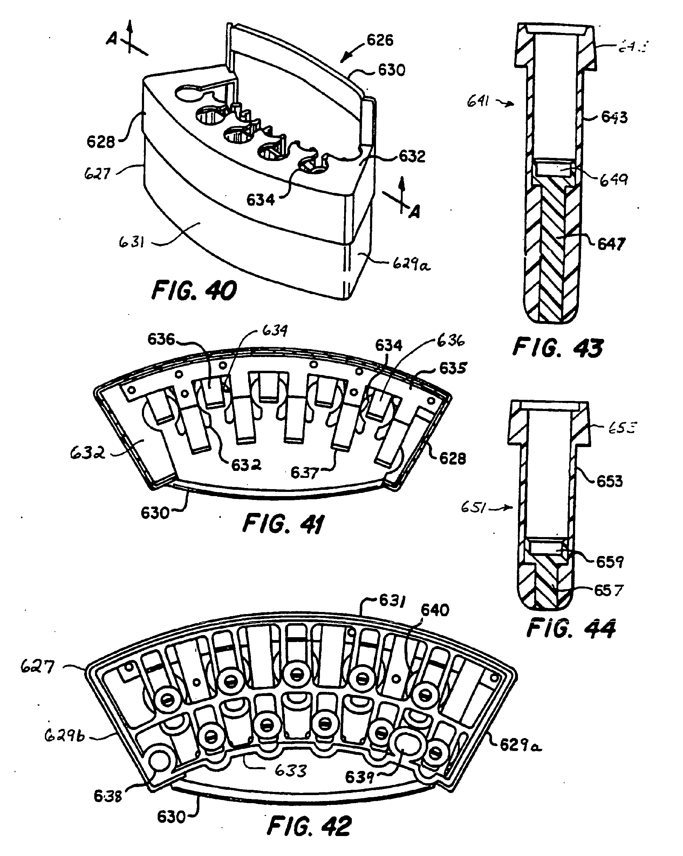

FIGS. 43 and 44 are cross sectional, elevational views of a long test sample cup adaptor sleeve 649 and a short test sample cup adaptor sleeve 655, respectively. These adaptor sleeves may be used with the short test sample Vacutainer^{®} tube segment assembly of FIG. 4G. Each adaptor sleeve 649 and 655 is constructed with an electrically conductive core material 651 which may be, for example, aluminum. A liquid sample cup 653 is placed in the top of either type of adaptor sleeve. When the probe contacts liquid in the sample cup, the core material 651 conducts the electrical signal from the probe/liquid combination to the vicinity of the receiving antenna 813 mounted below.

FIG. 25 is a graphical representation of system noise level versus signal frequency. The graph illustrates the importance of having a high center frequency (125 kHz) along with a narrow filter band width (250 Hz). The system noise level peaks at lower frequencies, and decreases as frequency increases. Thus, it is advantageous to operate at higher frequencies with narrow band width to reduce noise.

It is to be understood that the liquid level sensing system can be utilized in any automated instrument where liquid level sensing is desired.

### SYRINGE BUBBLE FLUSHER

The syringe can be used in any automated analytical system or other applications wherein fluidics are utilized in processes which are dependent on the precision and accuracy with which a syringe can aspirate and dispense fluids. A syringe which has the capacity for automatically flushing bubbles completely out of the fluidics system can achieve and maintain such precision and accuracy. The syringe, is configured such that a piston reciprocates through a seal into a close-fitting bore. The bore has a closed end, and the bore and piston define an annulus chamber in communication with fluid inlet and outlet means. Fluid is introduced near the seal, through the fluid inlet means, and into the annulus around the piston, creating a cross-flow flushing bubbles from the annulus. While the cross-flow is occurring, the piston is reciprocated within the bore. The reciprocation causes high fluid velocities in the annulus between the piston and the bore. The high fluid velocity dislodges any bubbles that may be adhering to the piston or bore wall. When the piston strokes to its full inward extension, it comes very close to the bore end, thus dislodging any bubbles stuck on the bore end or piston end. Bubbles dislodged by the piston movement within the annulus chamber are swept out of the syringe by the cross-flowing fluid near the seal.

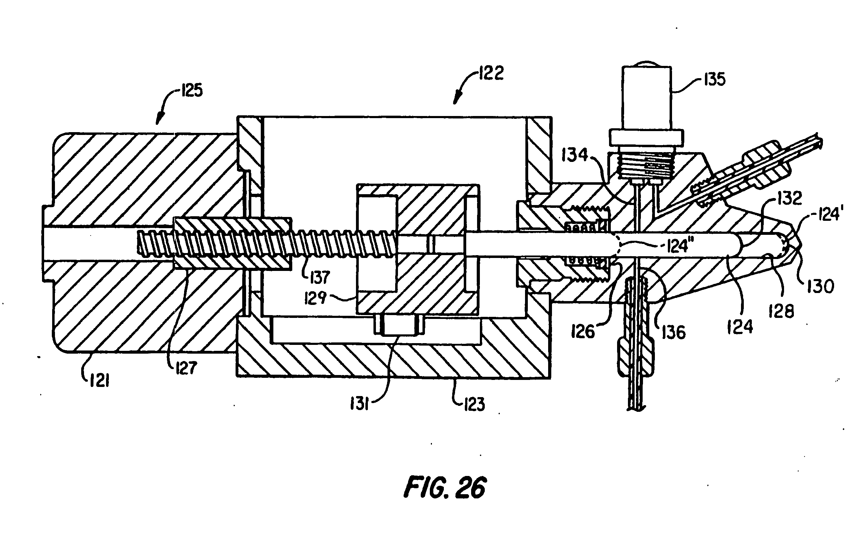

Referring now to FIGS. 26, 27, and 28 in' combination, there is shown a syringe 122 which aspirates and dispenses fluids to the various pipetting mechanisms and has the ability to automatically flush bubbles from the syringe 122. The ability of diagnostic instrumentation to accurately perform an assay is critically dependent on the precision and accuracy with which syringes, i.e. pipetting, can aspirate and dispense reagents and samples. The precision and accuracy of a syringe is severely degraded by the presence of small air bubbles inside a syringe. Bubbles, unfortunately, are all too common and are difficult to remove or avoid. Syringe 122 avoids these problems by automatically flushing bubbles completely out of the fluidics system. The syringe 122 is configured such that a piston 124 reciprocates through a seal 126 and into a close-fitting bore 128. The end of the bore 130 is closed. The piston 124 has a piston end 132 which approximates the geometry of the closed bore end 130. An annulus 138 exists between the piston 124 and bore 128. A fluid entry port 134 and a fluid exit port 136 are positioned 180 degrees apart and are located near the seal 126. Pressurized fluid is introduced to the fluid entry port 134. The fluid flows into the annulus 138, around both sides of the piston 124, and then exits through the fluid exit port 136. This crossflow flushes bubbles from the area near the seal 126.

Still referring to FIGS. 26, 27, and 28 in combination, while the cross flow through the annulus 138 near the seal 126 is occurring, the piston 124 is reciprocated inside the bore 128. This reciprocation causes high fluid flow velocities in the annulus 138 between the piston 124 and the bore 128. The high flow velocity dislodges any bubbles that may be adhering to the piston 124 or bore wall. The inward stroke of the piston 124 pushes these dislodged bubbles to the crossflow area where they are swept out of the syringe 122 by the crossflow. The piston end 132 and the bore end 130 have similar spherical shapes. When the piston 124 strokes to its full inward extension, it comes very close to the bore end 130. Any bubble that may be stuck on the bore end 130 is disrupted and dislodged. Likewise, the bubbles from the bore end 130 and the piston end 132 are dislodged and pushed to the crossflow area where they are swept out of the syringe 122 by the crossflow. The sequence of reciprocating the piston while cross flowing occurs can be automatically executed any time by the system apparatus.

Referring still to FIGS. 26, 27, and 28, once the fluid leaves the fluid exit port 136 of the syringe 122, it must travel through a tube fitting, through a length of tubing, through another tube fitting, into a probe 106 and out the probe tip 108. It is at the probe tip 108 that the aspirating and dispensing of reagents actually occurs. Any bubbles trapped between the syringe and the probe tip will also degrade performance, so there must be no place for the bubbles flushed out of the syringe to lodge. It is therefore necessary to use zero dead volume tubing fittings on the tubing between the syringe and the probe. In operation of the bubble flushing aspect of the syringe 122, the initial withdrawal velocity of the piston from the at rest or home position 124' is slower than the velocity of the piston as it approaches a total withdrawal position 124". This type of manipulation of the piston action in relationship to the end of the bore avoids high vacuum and bubble creation within the bore. On the other hand, the piston can be withdrawn from the home position 124' at full speed in order to expedite removal of preformed bubbles in the end of the bore. After such bubble flushing procedures, the valves are closed and aspiration can be perfected. If, however, the syringe is used for the dispensing aspect, the valve can be open for metered amounts of liquid to pass for dispensing purposes.

Referring now to FIG. 26, the operation of the aspirating aspect of the syringe 122 can now be explained. Following the bubble flushing operation explained above, the piston 124 is placed in a home position 124' and a zero dead volume two way solenoid valve 135 is closed. With the solenoid valve 135 closed, the fluid in the fluidics system is closed with the exception of the probe tip 108. The fluid in the fluidics system is a tryss fluid, or hydraulic fluid medium, which preferably will not react or mix with the sample or reagent to be aspirated. Examples of hydraulic fluid mediums would include but are not limited to deionized water, saline solution, and the like, depending on the properties of the fluid to be aspirated.

Still referring to FIG. 26, to aspirate a fluid the probe tip 108 is positioned within the fluid to be aspirated. The piston 124 is then moved from the home position 124' to a position representing the amount of aspirated fluid. The withdrawal of the piston causes the hydraulic fluid medium to withdraw from the probe tip 108, thereby drawing into the probe tip 108 the fluid to be aspirated. The probe tip 108 is then positioned to a location for expelling the fluid to be aspirated. The fluid to be aspirated is then expelled by moving the piston 124 back to the home position 124'. Residual aspirated fluid can occasionally be flushed from the fluidics system by positioning the probe tip 108 in a location for disposing fluids, opening the solenoid valve 135, and forcing the hydraulic fluid medium through the fluidics system. Once the fluidics system has been flushed, the solenoid valve 135 is closed and the syringe 122 can continue to be used for aspirating fluids.

Referring again generally to FIGS. 26, 27, and 28, in combination, the syringe 122 configuration can be, but is not intended to be limited to, about 8. 3" long, 3. 5" wide and about 2.7" deep. A linear actuator 125 is mounted to the frame 123. An actuator motor 121 spins a nut means 127 into which a mating lead screw 137 is screwed. The lead screw 137 is clamped to the coupler 129 which has a bearing 131 mounted on its bottom side. The bearing 131 runs in a groove in the frame 123. Since the coupler 129 is rotationally constrained by the bearing 131, the coupler 1.29 reciprocates when the linear actuator motor 121 spins the nut means 127 and the piston 124 which is clamped into the coupler 129, thus reciprocating the piston 124 through the seal 126. The piston 124 reciprocates through the seal 126 which is springloaded and carried by a polyethylene wear ring 133, the seal 126 being comprised of an O-ring over the polyethylene wear ring. The clearance between the piston and the bore is small and, according to a preferred embodiment, from between about 0.002" and about 0.008". When piston 124 reciprocates, very high flow rates are generated in the annulus 138 between the piston 124 and the bore 128. These high flow velocities flush bubbles in the bore 128 to the seal area where they are swept out of the syringe 122 by the cross flow. Zero (0) dead volume fittings are positioned between the syringe 122 and the tip release means to ensure that bubbles flushed out of the syringe 122 have no place to lodge as they are swept down the communicating tube and out the tip.

It is to be understood that the syringe can be used in any situation where the precise manipulation of fluids is desired, whether the syringe is operated manually or by an automated instrument, including, but not intended to be limited to, precision aspirating and dispensing of fluids such as found in many medical diagnostic instruments and devices, precision analytical pipetting, and like situations where precision manipulation of various volumes of fluid is needed, particularly small volumes of fluid. In addition, the inclusion of a second valve downstream of the syringe converts the syringe into a precision positive displacement pump.

The syringe is particularly useful with an automated analytical system which is capable of simultaneously performing two or more assays on a plurality of test samples in a continuous and random access fashion, such as the system described in greater detail herein. In particular, the automated immunoassay analytical system apparatus can be viewed as a microprocessor based system of integrated subassemblies with different groups of assays being run through separate and changeable software modules. The microprocessor based system uses robotic arm pipetters with two degrees of freedom and bidirectional rotating carousels to process samples. Critical assay steps such as incubations, washes and specimen dilution are performed automatically by the instrument as scheduled.

### REAGENT CAP AND PACK ACTUATOR

Diagnostic systems utilizing reagents to analyze fluids depend heavily on the correct concentration of these fluids or reagents. Since evaporation can affect reagent concentration, it is important to minimize evaporation of these fluids from their storage containers. For example, reagent containers have been described which utilize self-sealing septum designs to help control evaporation after, for example, a shipping seal is broken, removal of reagents, and the like. However, such designs contribute to cross-contamination or carryover of reagents from one container to another with a pipette probe. In addition, manual operation of presently known container closure systems or use of a septum cap can result in contamination and evaporation of costly reagents.

An apparatus and method for controlling evaporation of reagents are provided which facilitate the operation of multiple containers from an evaporatively sealed condition to an open position for access of reagents from the containers by transfer pipette mechanisms of the system. As described in greater detail below, the apparatus is capable of closing multiple containers to an evaporatively sealed condition employing opening and closing means. In particular, the reagent containers are opened and closed by the apparatus in a manner which minimizes the time the container remains in an open condition, thus minimizing or eliminating evaporation while, at the same time, maximizing accessibility to the liquid reagent contained therein by a pipette probe or transfer mechanism. The system methodology for opening and closing the closure and cap means accommodates variations in container evaporation differences and will open and close containers properly while maintaining an evaporative seal when not in use.

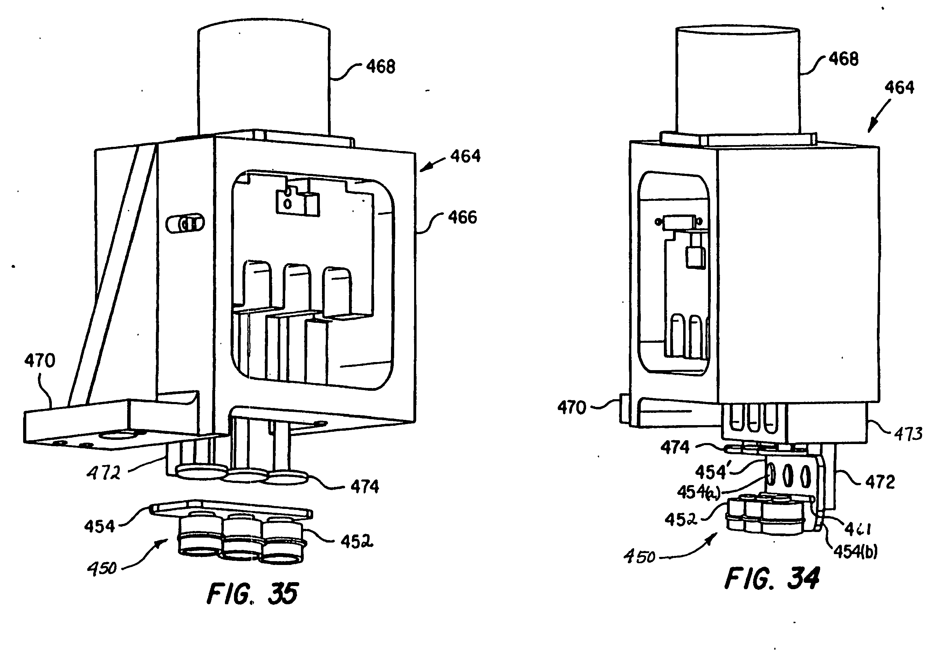

As shown in Figures 34 and 35, an opening and closing station 464 is provided for opening and closing closure and cap means 454 of a reagent vessel 450 to prevent contamination or carryover between different reagent containers by a common pipette probe while, at the same time, minimizing evaporation. Although closure systems having container closures which remain open with no automatic closing means have been described, such closure systems are not capable of opening and closing closure and cap means as described herein. For example, the opening and closing station 464 is capable of opening the closure and cap means 454 to various positions, such as, for example, an evaporatively sealed soft closure for routine day-to-day usage of opening and closing, or an evaporatively sealed hard closure for periods of non-use of reagents or for handling of the reagent pack.

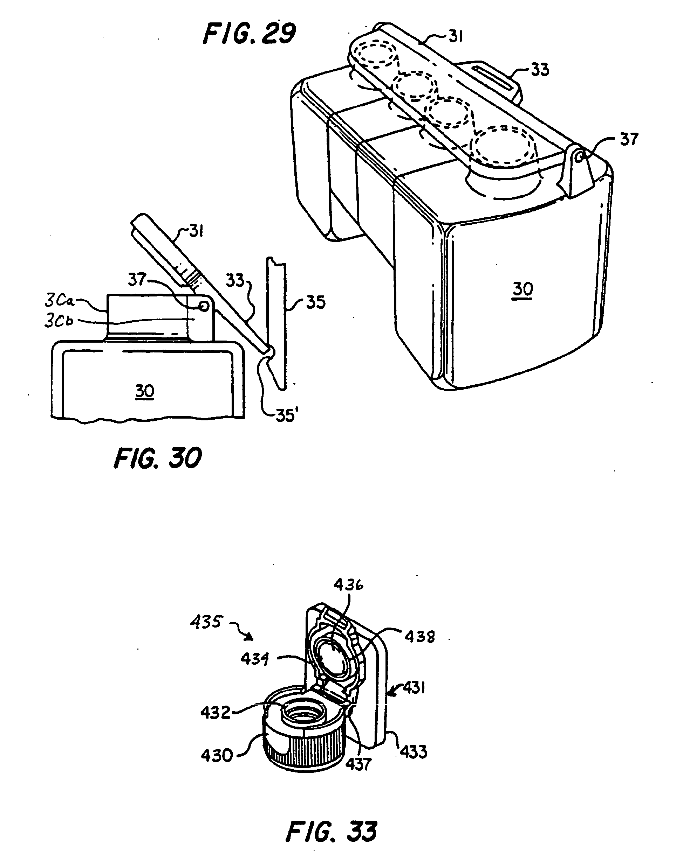

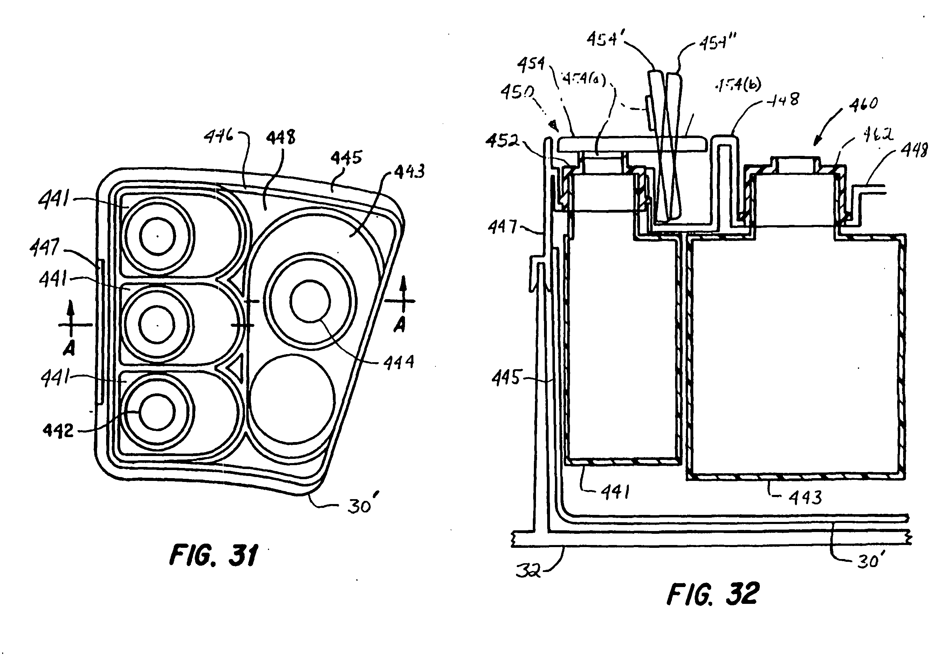

A reagent container or pack 30 (FIGS. 31 and 32) is moved below the opening and closing station 464 where the station opens a closure and cap means 454. The fluid in the container is withdrawn by a pipette probe, and the opening and closing station closes the container to an evaporatively sealed condition. As shown in FIGS. 29 and 30, a reagent pack 30 has a cover 31 which pivots between an open and closed position on a pin 37. The pin 37 can include biased spring means to facilitate movement of the cover 31 between opened and closed positions. For example, such spring means can be a hinge formed from a stretched material, such as stretched plastic, to provide the desired bias.

The apparatus and method is also capable of controlling the opening acceleration of the reagent container closure systems to reduce or prevent contamination between reagent containers, and to prevent the loss of reagents by, for example, randomly sprayed or airborne liquid reagents, typically in the form of droplets, which may otherwise result from abrupt opening of the containers. The cover 31 extends radially from the other side of the pin 37 forming a tab 33 as a lever arm for the cover 31. According to this, the system apparatus comprises a linear actuator (not shown) positioned at an opening and closing station (not shown) on the front end carousel 4. The linear actuator reciprocates a plunger 35 having a notched lower end 35' for engaging the tab 33. When the linear actuator extends the plunger 35 downwardly, its notched lower end 35' engages the tab 33 to open the cover 31. When the actuator withdraws the plunger 35, its notched lower end 35' pulls the tab 33 up to close the cap 31.

In (FIGS. 31-35), there is shown, in FIG. 31 a top view of a reagent pack 30 containing reagent containers 450 having reagent container openings 452 closed by closure and cap means 454. The reagent containers 450 are maintained within reagent pack walls 456 as well as an open bulk liquid container 460. The reagent pack walls. 456 give the reagent pack 30 a configuration which is suitable for insertion into the reagent pack carousel 32 (FIG. 3) of the front end carousel 4 (FIG. 3). The reagent containers 450 and the open bulk liquid container 460 are maintained within the reagent pack 30 by reagent pack container mounting and stabilization surfaces 458. The side sectional view of FIG. 32 is a section taken along section A-A of FIG. 31 and shows three positions of the closure and cap means 454. For example, the closure and cap means 454 is shown in an open position 454', preferably biased by spring means to a locked position to prevent closure of the closure and cap means 454 such as by, for example, movement of the front end carousel 4, and in an opened but not locked position 454", and closed position 454. It is to be understood that open positions 454' and 454" provide adequate access of a pipette probe to withdraw liquid reagents from a reagent container 450 through the container opening 452. In this regard, movement and positions of the closure and cap means 454 are not intended to be limited as shown, and other open positions are contemplated provided that the reagent in a reagent container 450 is accessible by a pipette probe. The isometric view of FIG. 33 illustrates a reagent container 450, closure and cap means 431, contact surface 433 and reagent container opening 452. The closure and cap means 454 are shown in an open position exposing a cap member 462 which fits inside the reagent container opening 452 and, together with a spaced-apart ring member 463, provides an evaporatively sealed reagent container 450 when the closure and cap means 454 are in a closed position as described above. It is to be understood that such evaporatively sealed closed position can either be a hard seal as described above, whereby the cap member 462 is securely fixed to the opening 452 for extended periods of non-use or handling of the reagent pack 30, or it can be a soft seal, whereby the cap member 462 is ajar, but nevertheless in an evaporatively sealed position, against the opening 452 with no or minimal assistance from the ring member 463.

An opening and closing station 464 is shown in a perspective side elevational view in FIG. 34, and a different perspective side elevational view of the opening and closing station is shown in FIG. 35. The opening and closing station 464 has a housing 466 and a drive motor 468 mounted thereon. The housing 466 has a mounting means 470 for mounting and fixing the opening and closing station 464 above the reagent carousel 32 (FIG. 3). The opening and closing station 464 comprises opening pins 472 which contact a tab portion 453 of the closure and cap means 454 in a downward motion to thereby bring the closure and cap means 454 to a desired open position as shown in FIG. 34. Rotational movement of the reagent carousel 32 positions the desired reagent containers 450 below the opening and closing station 464 for either opening of the closure and cap means 454, or for closing the closure and cap means 454 by the opening and closing station 464, as previously described.

In operation, the reagent carousel 32 rotates to position the containers 450 in FIG. 34 away from the opening pins 472 and under reagent pack closure activators 474 which are brought into frictional contact with the open closure and cap means 454 to thereby push said closure and cap means 454 from the substantially vertical open position 454' to the open locked position 454" shown in FIG. 32, wherein the closure and cap means 454 are locked by internal spring means (not shown) as described above. For example, FIG. 34 illustrates the closure and cap means 454 in an open position subsequent to contact of the tab 453 of the closure and cap means 454 by opening pins 472, whereby said closure and cap 454 pivot about pivot means 476 to open the closure and cap means 454 to a substantially vertical position as shown. The opening and closing station 464 further comprises reagent pack closure activator members 474 which are in the form of three valve-shaped heads and which are in an inactive position while the opening pins 472 are pushed down and against the tab 453 of the closure and cap means 454 for opening the reagent containers 450. Alternatively, the reagent pack closure activator members 474 can be in the form of a single valve-shaped head, having dimensions similar to the dimensions of the closure and cap means 31 (FIG. 30) or the closure and cap means 454 (FIG. 34).

In order to close the reagent containers 450, when in the open position 454' or 454" as shown in FIG. 34, the reagent container 450 is positioned under the reagent pack closure actuator members 474 by rotational movement of the carousel 32, whereby the closure and cap means 454 are brought into frictional contact with either partially lowered opening pins 472, or partially lowered reagent pack closure actuator member 474, which drag against the open locked closure and cap means 454 to overcome the spring means and return the closure and cap means 454 to a partially closed or soft seal position. Alternatively, the closure and cap means 454 can form a soft seal on the reagent containers 450 by contact of the reagent pack closure actuator member 474 with the closure and cap means 454, or the actuator members 474 can be brought into a more firm contact with the soft closed closure and cap means 454 to force the closure and cap means into a hard closed position, or both. It is to be understood that such soft seal and hard seal can be similarly accomplished utilizing the opening pins 472 without assistance from the reagent pack deserve actuator members 474 whereby in all instances the reagent container 450 will then be returned to a closed evaporatively sealed condition.

It is to be understood that the closure and cap means 454 can be individual for each reagent container 450 as shown in FIG. 33 or can be of the gang-type as shown in FIG. 34 and 35. In addition, the opening and closing station 464 can, for example, have three opening pins 472 and three reagent pack closure actuator members 474 which can function independently and operate for opening either individual reagent containers or, preferably, simultaneously open all of the reagent containers 450, whether the closure and cap means 454 are individual for each reagent container 450 or joined together in presenting an expanded closure and cap means covering multiple reagent containers 450 as shown in FIGS. 30 and 34.

The acceleration of the opening of the closure and cap means 454 is controlled by the reagent pack closure members 474 whereby the reagent pack closure members 474 contact the upper surface of, and reciprocate in an upward direction with, the closure and cap means 454 during the process of opening the closure and cap means 454 as described above. When reciprocally contacting the upper surface of the closure and cap means 454, the reagent pack closure members 474 provide downward resistance onto the closure and cap means 454 to control the upward or opening acceleration thereof while, at the same time, allowing the closure and cap means 454 to open to the desired open position for access of liquid reagent in the reagent container 450 by a pipette probe.

It is to be understood that other variations of the closure and cap means 454 and operation of the opening the closing station 464 are contemplated For example, spring means can be associated with the pivot means 476 of the closure and cap means 454 or with the hinge means 437 of the closure and cap means 431 (FIG. 33), whereby closing of the closure and cap means 454 or 431, respectively, can be accomplished without the assistance of the downward force of the opening pins 472 or reagent pack actuator members 474. For example, the closure and cap means 454 or 431 can be spring biased to the closed position, whereby the opening pins 472 remain in contact with the tab 453 of the closure and cap means 454 or the tab 433 of the closure and cap means 431 subsequent to the opening operation as described above to maintain the closure and cap means 454 or 431 in an open position to allow removal of reagents from a reagent container 450 with a pipette probe. One pipette has been withdrawn from the reagent container 450, the opening pins 472 move in an upward direction away from tabs 453 or 433 to thereby allow the closure and cap means 454 or 431 to return to their evaporatively sealed closed positions. The spring means can be in the form of a stretched material, such as stretched plastic, tension springs, and the like, whereby the desired bias can be ascertained by one skilled in the art apprised of the foregoing considerations. As would be understood by one skilled in the art, such embodiment can be employed, for example, in an Abbott IMx^{®} analyzer or TDx^{®} analyzer wherein means for movement of a reagent pack mounted therein are not provided.

In addition, a pipette probe transfer mechanism can either be located at a remote location or station from the open and closing station 464, thereby requiring movement of a reagent pack to such pipette probe transfer mechanism for access of reagents in a reagent container by the pipette probe or can be associated with the open and closing station 464, whereby such movement or positioning of a reagent pack is not necessary. Moreover, the opening and closing station 464 is not intended to be limited for use with the rotational movement of a carousel as described herein. For example, a reagent pack can be mounted or otherwise positioned on a non-concentrical or linear conveyor system, whereby such non=concentrical.system reciprocates with a reagent pack to facilitate the opening and closing of a closure and cap means as described herein. Similarly, the opening and closing station can be utilized in conjunction with carousels and non-concentrical conveyor systems which are not necessarily in the horizontal plane.

The Kitting Center is treated as one pipette block. Carryover experiments have shown that a post wash of at least about 2 ml is sufficient to clean the probe to a carryover level of 1 ppm or less when sample is kitted first followed by wash and pipetting of reagents. Total wash following sample should be about 4 ml total wash before next kitting activity. Contamination of the reagent bottle following sample will come from the outside of the probe. This is reduced to insignificant levels by wash to waste cup, e.g., 200 to 1,000 µl, followed by from between about 1 ml to about 2 ml wash to the wash cup.

In order to insure consistent, rapid resuspension and continued mixing of reagents with minimal operator involvement, the reagents are mixed automatically each time a new reagent pack is added to the reagent carousel, and periodically during instrument operation. This automated mixing can be accomplished by a back and forth motion of the reagent carousel with asymmetric pauses and is complete within approximately 1-2 minutes. The carousel acceleration, velocity, distance moved, and pause-asymmetry are optimized to yield the most rapid reagent resuspension without foaming or bubble formation for the range of fill volumes used on the instrument.

Automated reagent mixing provides the following benefits. The operator need not manually mix (e.g. by inversion or shaking) reagents which have been stored prior to their placement on the instrument. This allows the reagents to be loaded onto the instrument in less time and with less involvement of the operator. There is less tendency for reagents to foam or form bubbles with automatic mixing than with manual mixing such as inversion. Foam and bubble formations are detrimental to instrument function and can negatively impact assay performance. Automated mixing insures that reagents are always mixed sufficiently and that they are mixed consistently. Occasional automatic mixing during instrument operation keeps reagents in a consistent suspension, and makes it unnecessary for the operator to periodically remove reagent packs in order to mix the reagents. In some circumstances, automated mixing can dissipate bubbles present at the start of mixing. A detailed description of kitting and process activities are presented later herein for FPIA procedures for a phenobarbital assay; and for MEIA procedures for a CEA assay.

### TEST SAMPLE CONTAINER SEGMENT

A test sample container segment assembly adapted to receive a plurality of test sample containers of varying dimensions for use with an automated analytical instrument is provided. The segment assembly cooperates with a test sample carousel of the automated analytical instrument to provide variability in test sample containers which can be processed by such automated analytical instrument. Accordingly, the test sample container segment assembly obviates the need to transfer test samples from an otherwise incompatible test sample container to a test sample container which is required for use with an analytical instrument.

In particular, the test sample carousel comprises a plurality of positions for receiving the test sample segments. For example, the carousel is preferably adapted to receive six test sample segments, with each segment containing receiving positions for either ten or fifteen test sample containers. The segment can accommodate, for example, one or more sample cups and primary tubes, wherein the number of primary tube sizes and dimensions will vary. The primary tubes and sample cups may be mixed within the same sample segment. The different sample segments will support the primary tubes and sample cups so that the aspiration of samples will be accomplished at substantially the same height to provide the automated analytical system with a common level for a probe, which is utilized in combination with pipetting means, to provide sample transfer and kitting of reagents into a reaction vessel on a reaction vessel carousel. Accordingly, since probe pipetting means are in demand in relationship to time and use, the test sample segment assembly enables, for example, less travel and level sense time when such primary tubes and sample cups are utilized to thereby increase throughput of the system. Preferably, each sample segment includes an identifying number and code which is read by an instrument and associated with sample segments for kitting and processing within the automated, random access analytical system instrument. Accordingly, the test sample carousel, in combination with the test sample segment assemblies, provide a maximum amount of accessibility for loading and unloading of sample containers, such as the primary tubes, sample cups, and the like containers as described herein.

The test sample container segment assembly 600 is shown in FIG. 36 in a perspective view. It is to be understood that test sample containers contemplated include, but are not intended to be limited to, Vacutainer^{®} tubes, test tubes, cuvettes, vials, sample cups and the like, all of which can be of varying sizes and dimensions. The assembly has a frame 601 and a handling means 603. The assembly 600 has a test sample container mounting shelf 604 into which test sample containers can be inserted through container insertion openings 606. Once inserted into the container insertion opening 606, the containers are received and surrounded by liquid level sense sleeves 608. However, insertion openings 606 of the assembly can adequately support and fix said test sample containers without use of sleeves 608. The test sample container segment assembly 600 has two curved dimensions which are in a parallel relationship and are equal to the radius of curvature of the test sample container carousel. The outer curved portion 610 of the test sample container segment assembly 600 and the inner curved portion 612, both being vertical and in relationship to the container mounting shelf 604, present an assembly which is mateable with the test sample container carousel. The test sample container carousel 28 has positioning and mounting pins which are receivable within pin receivers 614 and 616 of the test sample container segment assembly 600. These pin receiving elements allow an operator to properly position and mount the test sample container segment assembly 600 into the test sample container carousel 28. The test sample container carousel 28 has mounting pins 618 as shown in FIG. 38 which are received by the test sample container of assembly 600 receiving segments 614 and 616. These receiving segments 614 and 616 are shown in FIG. 37, which is a bottom view of the test sample container segment assembly of FIG. 36.

A cross sectional view in isolation of the test sample container carousel with a mounted test sample container segment assembly 600 mounted therein is shown in FIG. 38. The view in FIG. 38 clearly illustrates the adaptation of the test sample container segment assembly 600 to the test sample container carousel 28 providing an operator with the handling means 603 and alignment pins 618 for fitting into test sample container segment assembly 600 receiving curved portions 610 and 612.

A cross sectional view of a modified test sample cup 620 is shown with upper and lower skirt portions 624 and 622, respectively, in FIG. 39. Such modified test sample cup 620 can be utilized within the test sample container segment assembly for presenting to the assembly uniform outer dimension which fits routinely into the test sample container segment assembly 600, even though the interior of the test sample cup 620 is buried for various purposes.

A short test sample Vacutainer^{®} tube sample assembly 626 is shown in perspective view in FIG. 40. The short test sample Vacutainer^{®} tube segment assembly 626 has a frame 628 and a handling means 630. The assembly has Vacutainer^{®} tube mounting shelf 632 in which Vacutainer^{®} tube insertion opening 634 is provided for guiding and mounting the short test sample Vacutainer^{®} tubes into the short test sample Vacutainer^{®} tube segment assembly 626.

A top cross sectional view of the short test sample Vacutainer^{®} tube segment assembly is shown in FIG. 41; the view is taken along line A-A of FIG. 40. Vacutainer^{®} tube mounting spring means 636 provide a holding means for the insertable Vacutainer^{®} tube elements which are of a tubular or test tube configuration. In addition to the Vacutainer^{®} tube mounting spring means 636, Vacutainer^{®} tube holding arms 637 are presented which further stabilize and maintain the Vacutainer^{®} tubes in a specified position in relationship to the short test sample Vacutainer^{®} tube segment assembly 626 so that when the assembly is inserted into the test sample carousel 28, the test sample Vacutainer^{®} tubes will not only be positioned as to a uniform height, but will also be positioned at a specific location within the carousel and mounted short test sample Vacutainer^{®} tube segment assembly 626.

A bottom view of the short test sample Vacutainer^{®} tube segment assembly of FIG. 40 is shown in FIG. 42. Test sample carousel 28 mounting pin receiving elements 642 and 644 provide assembly mounting positioning guides within the test sample carousel 28. In FIG. 43 and 44 test sample cup adaptor sleeves of various lengths are presented. In FIG. 43, a cross sectional view of a long test sample cup adaptor sleeve 649 is shown. In FIG. 44, a cross sectional view of a short test sample cup is shown. FIGS. 43 and 44 allow the sample cups of FIG. 39 to be used in Vacutainer^{®} tube segments.

The sample acquisition carousel utilized in the present instrument preferably has, for example, six positions or sections for mounting test sample container segment assemblies as illustrated in FIGS. 36 and 40. The test sample segment assemblies are interchangeable, as the operator desires, with positioning pins either on the segments or on the carousel with appropriate receiving means ensuring proper positioning of the segments to the carousel. Accordingly, such interchangeable segments allow the acquisition of a variety of test sample containers which can reside on the carousel at any given point in time. It is to be understood, of course, that the number of sample acquisition carousel positions or sections may vary, and such number will depend upon the size of each portion or section and dimensions of the carousel.

Different types of segment assemblies can be placed in the test sample carousel such as, for example, (1) test sample cup segments which can be used in conjunction with the instrument sample cup 620, wherein the segment can hold up to fifteen of such test sample cups; (2) large Vacutainer^{®} tube segments, which can be used with Vacutainer^{®} tubes from about 0.400 to about 0.650 inches (10-16.5 mm) in diameter and about 3.000 to 4.000 inches (75-100 mm) in length. Such large Vacutainer^{®} tubes can be positioned in the segments to accommodate up to ten Vacutainer^{®} tubes; and (3) small Vacutainer^{®} tubes, which can be utilized with the short test Vacutainer^{®} sample tube segment assembly of FIG. 40, can accommodate Vacutainer^{®} tubes of about 0.400 to about 0.650 inches (10-16.5 mm) diameter and about 2.000 to 3.000 inches (50-75 mm) in length, with about ten positions to accommodate up to ten Vacutainer^{®} tubes.

Sample container adapters are also available which allow sample cup containers to be placed in a Vacutainer^{®} tube segment, particularly for sample cups 620. Such adapters allow sample containers to be used when a minimum number of sample containers are needed and space for a sample container is not available.

Level sensing of fluid in any of the test sample container in the sample segments can be accomplished as described in detail above. In addition, bar code identification is provided on the segment assemblies and adaptor sleeves. Such bar code identifications are used to identify the segment type and the substitution of, for example, an adaptor sleeve, as well as, of course, identifying the test sample, itself.

An operator can load empty test sample cups 620 into a test sample segment and pipette test samples into the sample cups 620. The operator may choose to configure the test samples according to a predetermined load list generated via a data entry process. Vacutainer^{®} tube adaptor sleeves and other tubes can, of course, be used in place of a sample cup 620 in the appropriate segment. The operator will then place the test sample segment on the test sample carousel and indicate to the self-contained scheduling and computer system that further test samples are to be processed. The test sample carousel will scan all segments at the appropriate time in order to track all onboard test samples. The instrument will continue, in sequence, to process test samples unless a "stat" test is ordered. When a "stat" test is ordered, the instrument will scan all segments until it finds the one which contains the "stat" test sample. When the stat kitting cycle has completed, the front end carousel will return to a previous sequence. The operator may choose to brace the instrument into a hold phase during loading and unloading of test sample segment assemblies. The kitting process will be suspended after the next kitting cycle. Once loading and unloading is complete, a second instruction will cause the kitting process to resume. The status of onboard test samples will be subject to audit through a data entry screen of the instrument. With such audit, the operator will know which segment assemblies are completed and may be removed. Single test samples may be placed into a segment assembly which is residing on the test sample carousel.

### REACTION VESSEL AND LOADER

Unit dose disposable reaction vessels play an important role in automated, continuous and random access analytical systems which are capable of simultaneously performing at least two different forms of assays on a plurality of test samples in a continuous and random access fashion, with generally one reaction vessel required for each assay, wherein multiple reaction vessels are utilized by such systems. Means for handling and loading such reaction vessels in multiple units into the reaction vessel carousel is particularly useful for the uniform and continuous operation of the system by the operator.

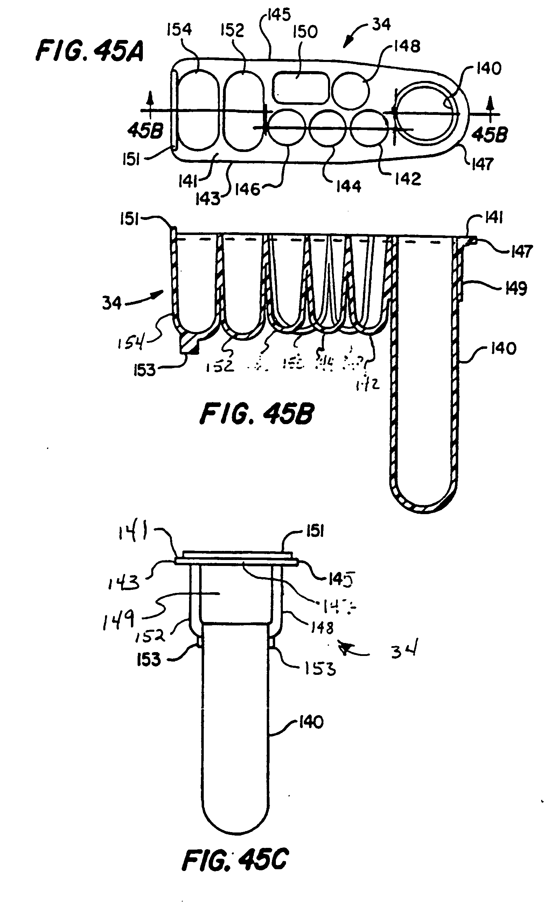

Referring now to FIGS. 45A-C in combination, there is illustrated a reaction vessel 34. The reaction vessel 34 has a top surface 141 with side edges 143 and 145. The side edges 143 and 145 of the reaction vessel 34 taper at one end to a rounded profile 147. At the opposite end of the top 141, a vertical tab 151 extends above the top surface 141. At the end of the top 141 near the rounded profile 147 is a holding means 149 extending below the top 141 for securing the cuvette 140 to the reaction vessel 34. The holding means 149 is typically a press fit aperture securing the cuvette 140 below the top 141. Extending through, and below, the top 141 are wells 142, 144, 146, 148, 150, 152, and 154. The wells 142, 144, 146, 148, 150, 152 and 154 can be selected for a specific size, location, and shape necessary for containing the reagents, samples, buffers and/or dilution liquids necessary for the apparatus operation. At the bottom of well 154 is a reaction vessel tab 153 for use in movement of the reaction vessel 34 by the transfer station as explained above.

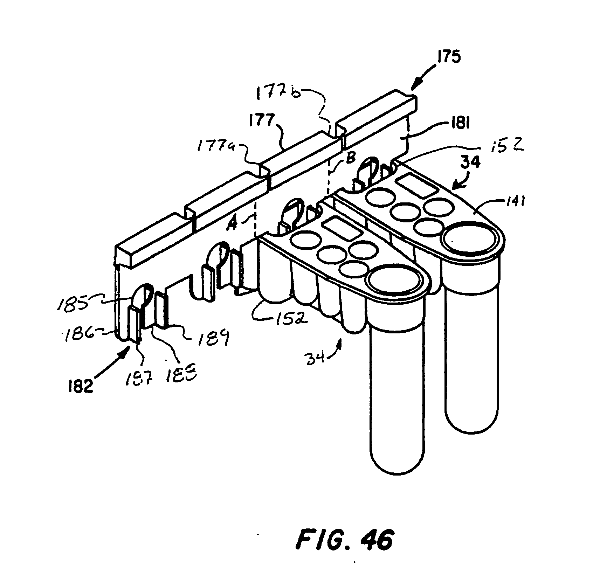

Referring now to FIG. 46, there is shown an isometric view of the reaction vessel loading strip 175, in section, having two reaction vessels 34 mounted thereon, illustrating the continuous strip upper portion handling ledge segments 177 which are separated by ledge cut-outs 179. Each ledge segment 177 coincides with a reaction vessel mounting means 182, which means 182 are on a lower portion of the continuous strip wall 181. The reaction vessel mounting means 182 includes flexible leg portions 183 having double fin sets 187 on each leg portion 183 for mounting of each reaction vessel 34. The double fin sets 187 project perpendicularly from the plane of the strip continuous wall 181 and leg portions 183. The leg portions 183 are flexible and in combination with the double fin sets 187 allow for firm holding of the reaction vessels 34 when mounted on the reaction vessel loading device strip 175 and yet release the reaction vessels 34 when inserted into the reaction vessel carousel.

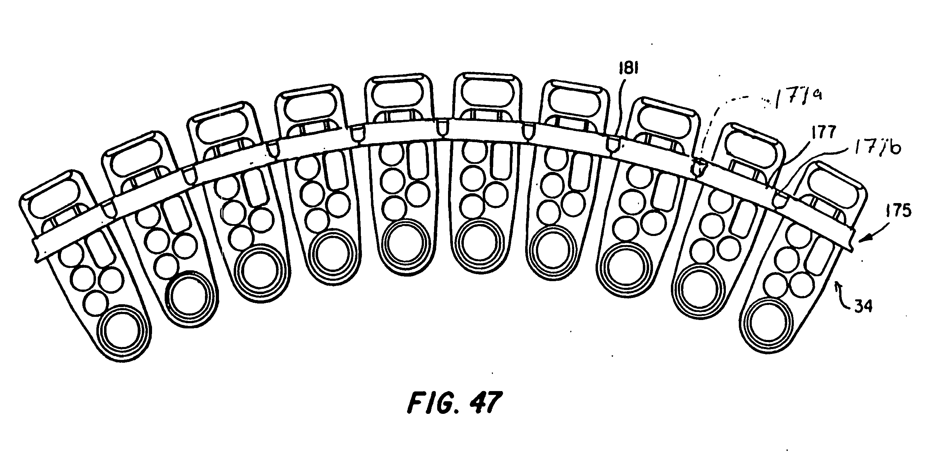

Referring now to FIG. 47, there is shown a top view of the reaction vessel loading device strip 175 having ten reaction vessels 34 mounted thereon. The reaction vessels 34 are mounted on the reaction vessel holding device strip 175 through assertion of the reaction vessel mounting means 182 into well 152. The reaction vessel holding device strip is utilized for loading multiple reaction vessels at one time into the reaction vessel carousel by arcing the strip continuous wall 181 to correspond to the radius of curvature of the reaction vessel carousel. In the embodiment illustrated, ten reaction vessels 34 are shown attached to one continuous strip wall 181; however, the reaction vessel loading device strip 175 can be expanded in length to accommodate more than ten reaction vessels or less than ten reaction vessels can be mounted on the same length strip or reduced strip lengths.

Referring now to FIGS. 46 and 47 in combination the reaction vessel loading device 175 is comprised of a semi-rigid plastic strip which holds a plurality of reaction vessels 34 at one time for loading the reaction vessels 34 onto the reaction vessel carousel. An operator would bend the loading device 175 into an arc which matches the radius of the carousel. Then the plurality of reaction vessels 34 mounted on the loading device 175 are inserted into their respective slots on the carousel. The multiple reaction vessels 34 are snapped into place on the carousel and then the reagent vessel loading device 175 is removed for reuse or for discarding.

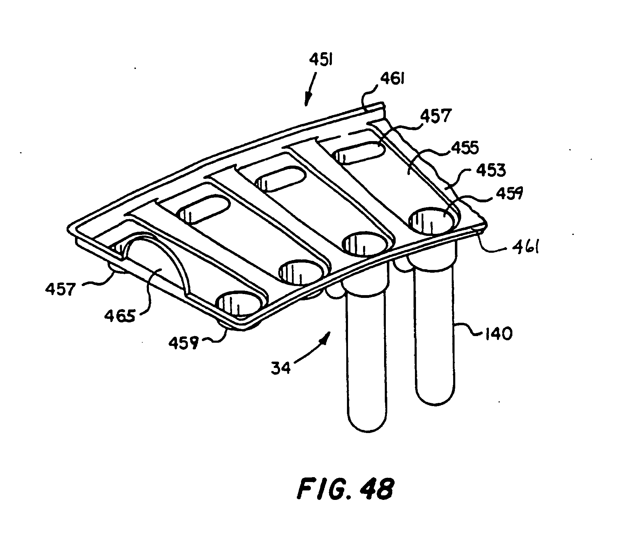

Referring now to FIG. 48, there is shown an isometric view of an alternate reaction vessel loading device 451, in section, having two reaction vessels 34 mounted thereon. The loading device 451 has a planar surface 453. Extending below the recessed planar surface 453 are a plurality of recessed planar surfaces 455 which have a shape generally conforming to the shape top surface 141 of the reaction vessels 34. Extending below the recessed planar surfaces 455 are cuvette plugs 459 which are sized and located for insertion into the cuvette 140 of the reaction vessel 140. Also extending below the recessed planar surfaces 455 are well plugs 457 which are sized and located for insertion into one of the wells 142, 144, 146, 148, 150, 152, or 154 of the reaction vessels 34. The well plug 457 and cuvette plug 459 have a diminishing size, or taper, as the plugs progress downward from the recessed planar surfaces 455, thereby providing ease of insertion or removal of the well and cuvette 140 of the reaction vessel 34. Extending upward around the outer parameter of the planar surface 453 is a continuous elevated rim 461. On the upper edge of the elevated rim 461 is a top surface 462 which is substantially flat and parallel to the planar surface 453. At either end of the loading device 451 are handling fins 465 which are parallel to, and extend upward from, the elevated rim 461.

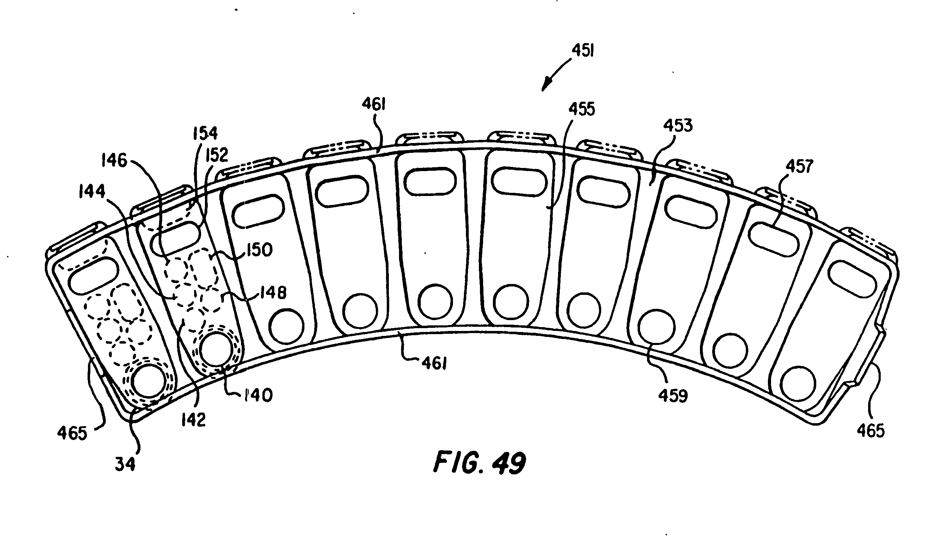

Referring now to FIG. 49, there is shown a top view of the alternate reaction vessel loading device 451 of FIG. 48 having ten (10) recessed planar surfaces 455 for holding ten (10) reaction vessels 34. Although the embodiment illustrated holds ten (10) of the reaction vessels 34, the loading device 451 can be configured to have any number of recessed planar surfaces 455 for holding any number of reaction vessels 34. The well plug 457 and the cuvette plug 459 of the loading device 451 insert into, and engage, the well 152 and the cuvette 140, respectively, thereby securing the reaction vessel 34 to the loading device 451. Although the loading device 451 secures the reaction vessels 34 by engaging the well 152 and the cuvette 140, the loading device could secure the reaction vessel 34 by engaging singly or in combination any number of the wells 142, 144, 146, 148, 150, 152, and 154 and the cuvette 140.

Referring now to FIGS. 48 and 49 in combination, the loading device 451 is preferably manufactured from a semi-ridged plastic and is generally formed in a shape corresponding to the radius of curvature of the reaction vessel carousel. The recessed planar surfaces 455 are spaced to correspond to the locations for mounting the reaction vessels 34 on the reaction vessel carousel. The reaction vessels 34 are loaded onto the loading device 451 by insertion of the well plug 457 and the cuvette plug 459 into the corresponding well 152 and cuvette 140 of the reaction vessel 34. In this manner, the recessed planar surfaces 455 provide a cover for the reaction vessels. Also, the well plug 457 and cuvette plug 459 provide a positive seal for the well 152 and the cuvette 140.

Still referring to FIGS. 48 and 49 in combination, the loading device 451 with reaction vessels 34 thereon is positioned on the reaction vessel carousel with the location of the reaction vessels 34 on the loading device 34 corresponding to the locations on the reaction vessel carousel for the reaction vessels 34. An operator is not required to use extraordinary care in shaping the loading device 451 since the loading device 451 is preshaped to fit the dimensions of the reaction vessel carousel. In this regard, the reaction vessel loading device is a "drop in" type of loading device for reaction vessels 34. Once the reaction vessels 34 on the loading device 451 are aligned, the reaction vessels 34 are snapped into place on the reaction vessel carousel using the handling fins 465 and elevated rim 461 of the loading device 451. In this manner, a plurality of reaction vessels 34 can be loaded on the reaction vessel carousel at one time saving the operator time over a method of individually loading the reaction vessels 34 into the reaction vessel carousel.

Referring still to FIGS. 48 and 49 in combination, once the reaction vessels 34 are loaded into the reaction vessel carousel, the loading device 451 can be left in place to provide a cover and seal for the reaction vessels. 34 until used as described herein. Removal of the loading device 451 from the reaction vessels 34 is then accomplished by pulling upward on the loading device, utilizing, for example the handling fins 465 or the elevated rim 461. Removal of the loading device 451 does not dislodge the reaction vessels 34 from the reaction vessel carousel because the holding force of the well plug 457 and cuvette plug 459 to the reaction vessels 34 is less than the reaction vessel carousel holding force on the reaction vessels 34. This reduced force is due in part to the tapered profile of the well plug 457 and the cuvette plug 458, which also eases the insertion of the reaction vessels 34 onto the loading device 451.

### ENVIRONMENT AND TEMPERATURE CONTROL

A controlled environment zone is necessary for incubation and chemical reactions within automated continuous and random access analytical systems. Temperature control is maintained in the controlled environment zone for purposes of controlling the temperature of disposables, chemicals, tubing, mechanisms and the like within the incubation and reaction zone which is optimum to the appropriate chemical reactions. Temperature control is achieved utilizing air flow and air temperature as the thermal dynamic working fluid. Although air or gases do not transfer heat as rapidly as a liquid bath, air does not have the associated problems of leakage, evaporation or contamination.

The controlled environment zone contains carousels carrying chemistries of different reagents and volumes thus requiring an unusual temperature control approach of forcing the heated air to negotiate a pathway with a substantial pressure drop immediately upstream of the process carousel. The pressure drop of the pathway is higher than the pressure drop the air experiences as it passes under the carousel, whether the carousel is fully loaded or not. Thus, the heated air distributes itself evenly about the carousel rather than preferentially funneling itself into a gap which may exit at the empty position of the carousel. Air flow control within the controlled environment provides for minimal air flow above the top surfaces of the carousels. Slow-moving air above liquid surfaces exposed by the open containers on the top portions cause less evaporation than air moving rapidly. However, total air flow is relatively high within the controlled environment zone and air flow along the carousel under sides can be a combination of turbulent and laminar flow. A reasonably high turnover rate and air flow is necessary to minimize temperature variation.

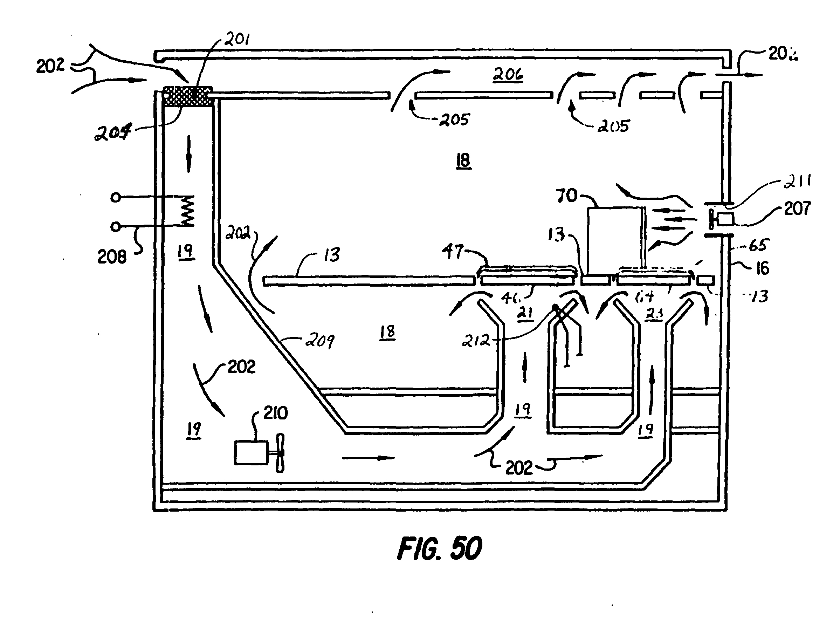

Referring now to FIG. 50, there is shown a schematic view illustrating the environmental airflow and temperature control system. Temperature control, for reaction and incubation zones of a continuous analytical system, is achieved utilizing heated air flow to control a carousel 19 of the controlled environment zone 18, which includes, of principle importance, a process carousel 46. Air flow. 202 is motivated by a fan element 210 and enters through an air inlet 205 including an appropriate air filter system. The air flow 202 is forced past an air heater element 208 and through conduit means embodied in the base plate of the instrument. As the air emerges from the ducting, the air is directed toward the underside of the carousel 46 which is the carousel environment zone 19 and contains the samples to be analyzed, the necessary reagents, and the disposables used in the processes. As the heated air passes through the carousel environment zone 19, its temperature is sampled by a sensor 212. The sensor 212 output is monitored by a controller and when the controller determines that the system requires additional amounts of heat, the controller energizes a solid state relay which applies electrical power to the heating element 208.

Still referring to FIG. 50, it can be seen that after leaving the carousel environment zone 19 air is allowed to circulate within the controlled environment zone 18. An air exhaust conduit 206 allows air to exit the controlled environment zone 18 in a controlled manner through multiple openings. While fan element 210 forces the heated air throughout the system, ambient air is introduced through air inlet 207 downstream from the most critical areas of temperature control within the controlled environment zone 18 in order to provide cooling for fluidics of the system through provision of cooling fluid to the heater assembly 501 which are utilized in the fluidics system. This introduction of ambient air through air inlet 207 is near the air exhaust 206 conduit and the various outlets which are in communication with the controlled environmental zone and the air exhaust conduit 206.

Referring still to FIG. 50, the controlled environment zone 18 is kept at a desired temperature by heating air to the correct temperature and applying air in large amounts to the most critical areas of the zone, such as the carousel environment zone 19. Heat is transferred by convection to the critical areas by using air which is generally experiencing turbulent flow, thus in this manner, critical areas may be brought to temperature as rapidly as possible. The less critical areas are downstream and are heated under less forceful conditions, i. e. slow moving air flow. In addition to having turbulent flow in the critical areas, the total air flow is relatively high within the controlled environment zone 18 with the air being completely expelled. Potential problems which could occur in dealing with fully loaded carousels versus partially loaded carousels is solved by forcing the heated air to negotiate a pathway with a large pressure drop immediately upstream of the carousel. The pressure drop of the pathway is higher than the pressure drop the air experiences as it passes under the carousel, whether the carousel is fully loaded or not. Thus, the air distributes itself evenly about the carousel rather than preferentially funneling into a gap which may exist at the empty positions on the carousel. Both FPIA and MEIA procedures utilize the system apparatus commonly through and including the process carousel 46.

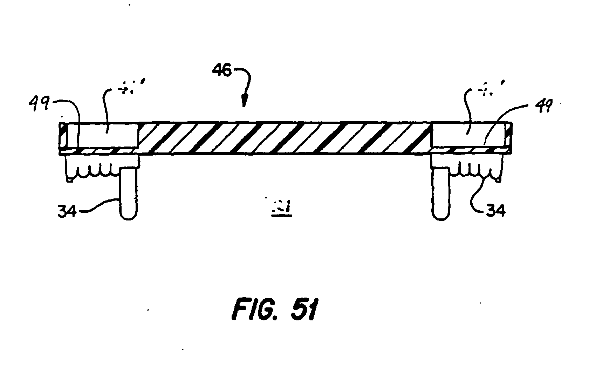

Referring now to FIG. 51, there is shown a cross-sectional view of the process carousel 46. The carousel 46 holds a plurality of reaction vessels 34. The lower portion of the reaction vessels 34 are disposed within the carousel environment zone 19 of the controlled environment zone 18. The carousel 46 includes reaction vessel top zones 47 which are open to the controlled environment zone 18. At the bottom of the reaction vessel top zones 47, the carousel 46 and the reaction vessels 34 substantially seal off the top zones 47 from communicating with carousel environment zone 19.

Still referring to FIG. 51, it can be seen that the walls of the reaction vessel top zones 47 will isolate the air above the reaction vessels 34 from the movement of the air flow 202 in the controlled environment zone 18. Because the air in the reaction vessel top zones 47 is not directly subjected to the air flow 202, there will be less movement of the air directly over the open containers of the reaction vessels 34, thereby reducing the amount of evaporation from the reaction vessels 34. Sealing the top zones 47 from the carousel environment zone 19 allows air flow 202 to pass over the underside of the reaction vessels 34 without disturbing the air directly over the reaction vessels 34, thereby transferring heat to, and from, the reaction vessels without increasing evaporation of the fluids in the reaction vessels 34.

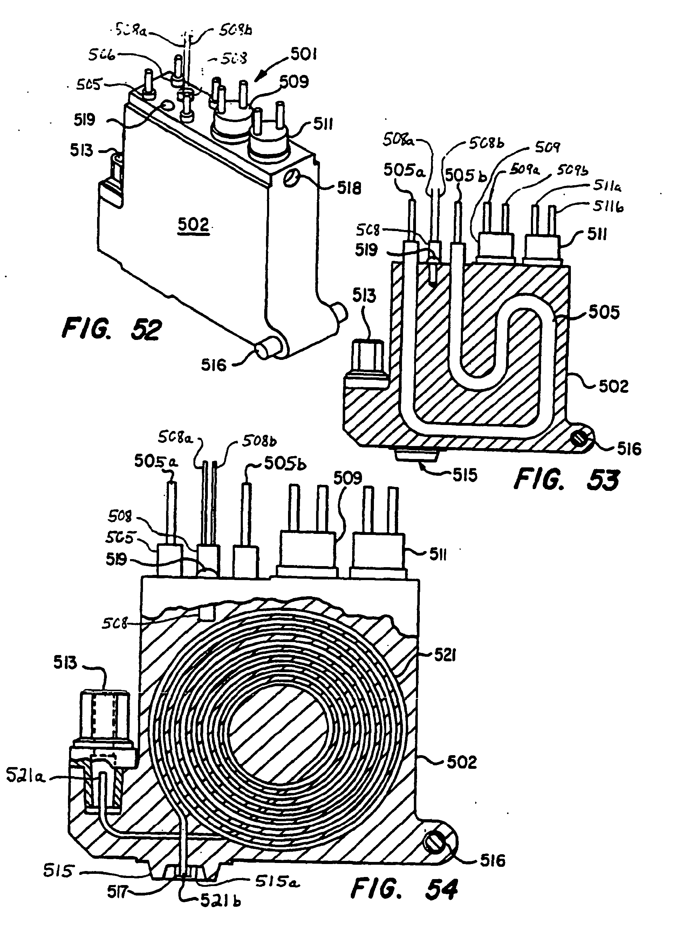

Referring now to FIGS. 52-54 in combination, there is shown the heater assembly 501 from FIG. 50. The heater assembly generally comprises a heater block or body 502 having therein a pair of heater elements 505a-b, and a coiled liquid tubing 521 between the heater elements 505a-b. The heater body 502 is constructed of, for example, a metal such as aluminum, silver, copper, or the like, or any other suitable thermoconductive material known in the art, with the various electrical terminals for an electrical resistive heater system, as well as sensing and control elements for controlling precision temperature of the heater assembly for purposes of precision temperature control heat exchange with liquids which flow through the heater assembly and maintained at specific temperatures. Mounting means 516 and 518 are shown as well as a ground pin 519 imbedded in the metal block 502.

Still referring to FIGS. 52-54 in combination, liquid enters the coiled liquid tubing 521 through a liquid inlet 513. After passing through the coiled liquid tubing 521, the liquid leaves the heater assembly through a liquid outlet 515 for deposit into the MEIA cartridge 68 (not shown). A teflon sleeve 517 is secured to the outside of the liquid outlet 515 to prevent the accumulation of liquid that might cling to the liquid outlet 515. The heater elements 505a-b are positioned within the heater body 502 on opposite sides of the coiled liquid tubing 521. Heater electrical posts 504 and 506 provide controlled amounts of energy to the resistance heater elements 505a-b. A thermistor 508 is positioned in the heater body 502 so that it is centrally located with respect to the heater elements 505a-b and the coiled liquid tubing 521. The thermistor 508 provides an electrical resistor whose resistance varies sharply or in a precise manner with temperature. The thermistor 508 cooperates with a thermostatic connection 509 and a backup thermostatic connection 511 to power the heater elements 505a-b and precisely control the temperature of the heater assembly 501 as well as any liquids contained therein or passing through.

Referring still to FIGS. 52-54 in combination, the heater assembly 501 can be sized to accommodate increased or decreased liquid volume capacity; as well as heating means for such increased or decreased liquid capacities. The positioning of the heater assembly 501 immediately above the use point avoids significant temperature change during the transfer from the heater assembly 501 to the receiving materials. Temperature controls of the liquids within the heater assembly are controlled from between about ± 1.0°C and about ±°C of the required liquid temperature. Positioning of the heater assembly 501 in relationship to the receiving means, for example, an MEIA cartridge, allows for about 3/8 inch (9.5 mm) or less of an air gap transfer from the tip of the liquid outlet 515 to the point of deposition of a liquid onto the cartridge, whereby the liquid is deposited with little or no temperature change thereof.

### MEIA CARTRIDGE, FEEDER, AND CARTON

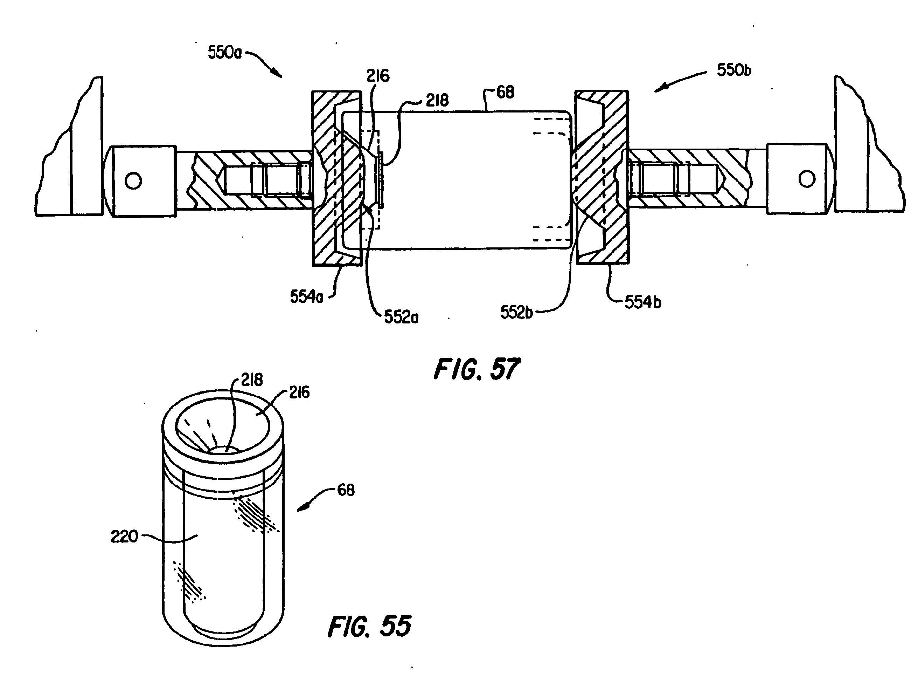

Referring now to FIG. 55, there is shown an MEIA cartridge 68. The MEIA cartridge is generally a cylindrical shape containing a support matrix material 222 therein. In the top of the MEIA cartridge 68 is a funnel throat 216 which tapers down into a cartridge opening 218. The cartridge opening 218 allows access to the support matrix material 222 contained therein. The bottom of the MEIA cartridge 68 is flat and has no funnel throat or opening.

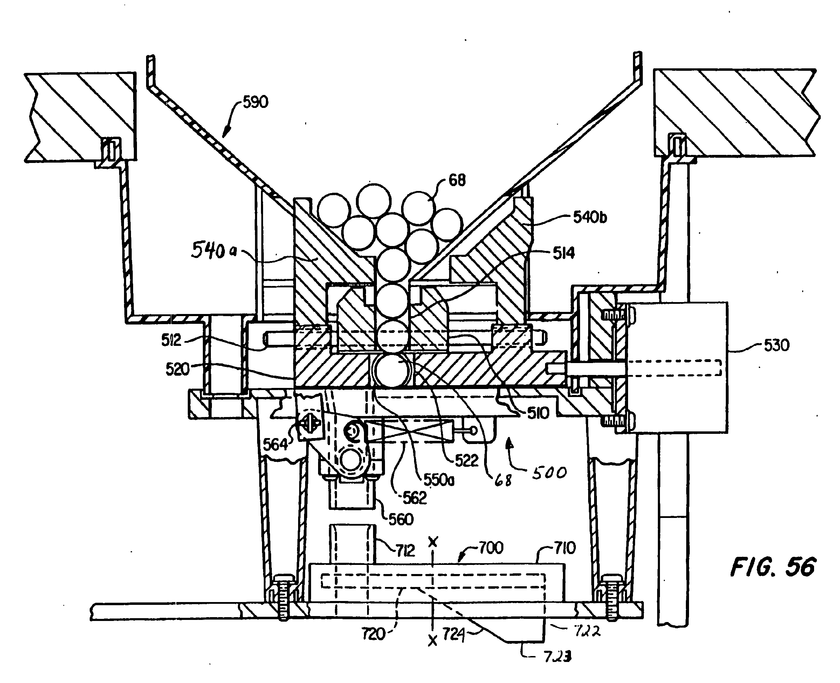

Referring now to FIG. 56, there is shown a cartridge feeder apparatus 500 which will feed MEIA cartridges 68 singly, upright, and on-demand from a hopper 590 into a trap door 700. The cartridge hopper 590 contains a plurality of MEIA cartridges 68 which are positioned horizontally and laterally in the cartridge hopper 590 with the cartridge opening 218 facing either direction. The cartridge hopper 590 is removably attached to a bridge 510 that is a stationary portion of the cartridge feeder apparatus 500. The bridge 510 has a bridge throat 514 which accepts the MEIA cartridges 68 from the hopper 590 and allows those cartridges to pass through the cartridge feeder apparatus 500. The bridge 510 also has guide rods 512a-b, upon which a shuttle 520 is slidably mounted.

Referring still to FIG. 56, a linear motor 530 moves the shuttle 520 forward and backward on the guide rods 512a-b of the bridge 510. The shuttle 520 has a shuttle throat 522 for receiving MEIA cartridges 68 from the bridge throat 514 when the shuttle 520 is in a home position. The linear motor 530 then slides the shuttle 520 to a drop position. As the linear motor 530 slides the shuttle 520 from the home position, the cup pins 550a-b grasp the MEIA cartridge 68. When the shuttle 520 reaches the drop position, the cup pins 550a-b release the MEIA cartridge 68 upright into a chute 560.

Still referring to FIG. 56, the chute 560 has a tapered inner profile which assists in orienting the MEIA cartridge 68 in an upright position as the MEIA cartridge 68 drops into the trap door 700. The chute 560 is rotatably mounted to the cartridge feeder apparatus 500. A spring 562 helps hold the chute 560 in position for operation of the cartridge feeder apparatus 500. A dump lever 564 connects to the chute 560, and when pressed rotates the chute 560 against the force of the spring 562. In this manner, any MEIA cartridge 68 which lodges in the chute 560 can be cleared by pressing the dump lever 564, which rotates the chute 560 and dumps the MEIA cartridge 68. After the MEIA cartridge 68 has been dumped, the dump lever 564 is released and the spring 562 returns the chute 560 to its normal operational position.

Referring still to FIG. 56, it can be seen that mounted to the shuttle 520 are pushers 540a-b. The pushers 540a-b pass through an opening in the cartridge hopper 590 and contact the MEIA cartridges 68 in order to prevent the MEIA cartridges 68 from blocking the passage through the bridge throat 514 of the cartridge feeder apparatus 500. In the home position of the shuttle 520, the pusher 540a passes through an opening in the hopper 590 and aligns the MEIA cartridges 68 above the bridge throat 514. In the drop position of the shuttle 520, the pusher 540b passes through an opposing opening in the cartridge hopper 590 and also aligns the MEIA cartridges 68 for passage through the bridge throat 514.

Referring now to FIG. 57, there is shown the cup pins 550a-b from FIG. 56. The cup pins 550a-b have a center profile 552a-b which extends outward and has a contour matching the funnel throat 216 of the MEIA cartridges 68. The center profile 552a-b does not have sufficient height to extend past the funnel throat 216 and come in contact with the cartridge opening 218 or the support matrix material 222 of the cartridge 68. The cup pins 550a-b also have an outer lip 554a-b which extends circumferentially around the center profile 552a-b. The outer lip 554a-b has a inner diameter sufficient for allowing the MEIA cartridge 68 to fit within the outer lip 554a-b. Also, the outer lip 554a-b does not extend beyond the center section 552a-b.

Referring now to FIGS. 56 and 57 in combination, it can be seen how the cartridge feeder apparatus 500 can feed cartridges singly in an upright position to a trap door 700. As previously mentioned, the MEIA cartridges 68 pass from the bridge throat 514 to the shuttle throat 522 when the shuttle 520 is in the home position. As the shuttle progresses from the home position, the cup pins 550a-b close in on the MEIA cartridge 68. When the cup pins 550a-b close in on the cartridge 68, the center profile 552a-b of the cup pin 550a-b facing the cartridge opening 218 will engage and fit within the funnel throat 216 of the cartridge 68. In this position, the center profile 552a-b of the cup pin 550a-b facing the cartridge opening 218 will engage the funnel throat 216 and will also surround the outside of the MEIA cartridge 68 with the outer lip 554a-b. The bottom of the MEIA cartridge 68 has no recess for the center profile 552a-b of the cup pin 550a-b to fit into. As a result, the outer lip 554a-b of the cup pin 550 engaging the bottom of the cartridge 68 will not surround the bottom of the cartridge 68 with the outer lip 554a-b.

Referring still to FIGS. 56 and 57 in combination, as the shuttle 520 approaches the drop position, the cup pins 550a-b begin to separate for dropping the cartridge 68 into the chute 560. When the cup pins 550a-b begin to separate, gravity will pull the cartridge 68 downward. Because the cup pins 550a-b engaging the bottom of cartridge 68 neither extends into a funnel throat, nor surrounds the cartridge 68 with an outer lip, the bottom of the cartridge 68 will be the first portion of the cartridge 68 to begin to drop toward the chute 560. As the cup pins 550a-b continue to separate, the center profile 552a-b and outer lip 554a-b of the cup pins 550a-b engaging the top of the cartridge 68 will continue to engage the top portion of the cartridge 68 while the bottom portion of the cartridge 68 is dropping due to gravitational forces. Once the cup pins 550a-b have separated by sufficient distance, the funnel throat 216 of the cartridge 68 will disengage from the center profile 552a-b, and the outer lip 554a-b of the cup pins 550a-b engaging the top portion of the cartridge 68 will disengage, allowing the cartridge 68 to fall in an upright manner through the chute 560. It can be seen from FIG. 18, that the design of the cup pins 550a-b will drop the cartridge 68 in an upright position regardless of whether the cartridge 68 is inverted or not in the cup pins 550a-b. In this manner, MEIA cartridges 68 are dispensed on demand, singly, and in an upright position, from the hopper 590 through the cartridge feeder apparatus 500 into a trap door 700.

Referring back to FIG. 56, the trap door 700 has a trap door body 710 with a cartridge passage 712, and a semicircular door 720 with a cartridge height adjustor 722. The MEIA cartridge 68 dropped by the cartridge feeder apparatus 500, falls into the cartridge passage. Initially, the semicircular door 720 blocks the cartridge passage 712. When the carousel under the trap door 700 is positioned to receive the cartridge 68, the semicircular door 720 rotates to a position that does not block the cartridge passage 712 and allows the cartridge to pass through to the carousel. After the cartridge 68 has passed through the cartridge passage 712, the semicircular door 720 continues to rotate until the cartridge height adjuster 722 presses the cartridge into the carousel to a height sufficient for accurate testing at a later time.

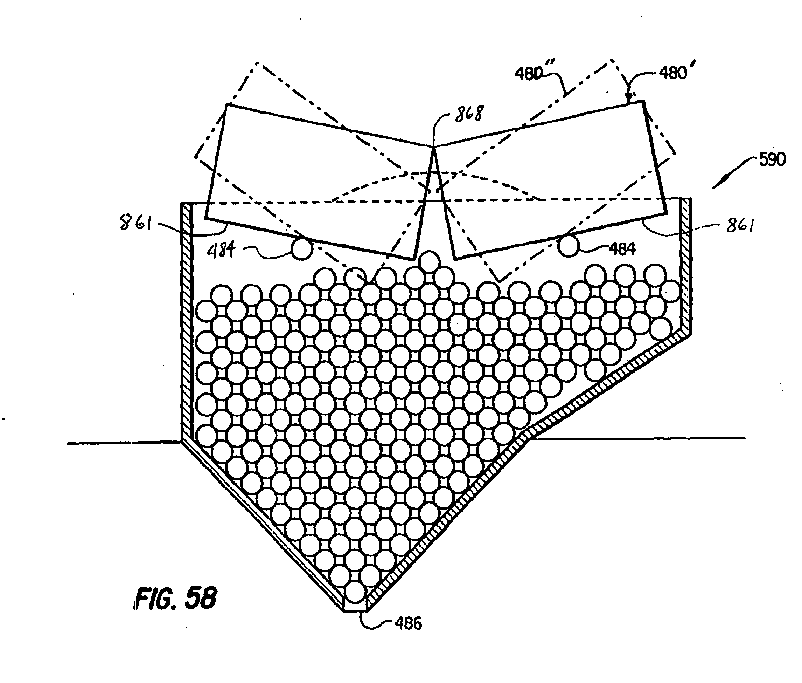

Referring now to FIG. 58, there is shown a side cross sectional view of a cartridge hopper 590 with a cartridge carton 480 positioned for unloading cartridges 68 into the cartridge hopper 590. The lower portion of the cartridge hopper 590 is tapered to a hopper release opening 486. The upper portion of the cartridge hopper 590 is large enough to act as a reservoir for the cartridges 68 and has two unloading roller pins 484. The cartridge carton 480 is shown resting on unloading roller pins 484. The cartridge carton 480 is also shown in phantom at a maximum open unloading position 481, again resting and being guided by the roller pins 484.

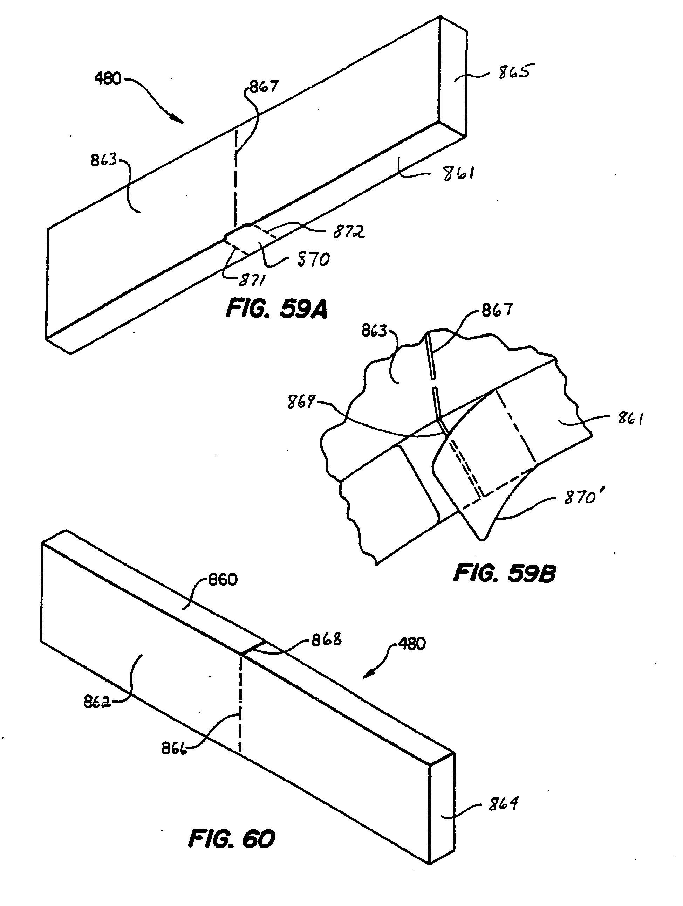

Referring now to FIGS. 59A, 59B, and 60 in combination, there is shown the cartridge carton 480 from FIG. 58. The cartridge carton 480 has break open or tab opening 482 for ease of opening and unloading in combination with the roller pins 484. Perforations 821 in the cartridge cartons 480 extend from the tab opening 482, up sides 839a-b of the cartridge cartons 480, and to a top 860 of the cartridge carton 480. The top 860 of the cartridge carton 480 has a hinge means 880 which connects the two perforations 821. The cartridge carton 480 can be designed to contain any number of cartridges; however, a carton capacity of about 100 is suitable for operation within the environments of the cartridge hopper 590 and the roller pins 484 locations. The cartridges 68 are loaded in the cartridge carton 480 in a lateral orientation with the cartridge opening 218 facing either direction.

Referring now to FIGS. 58, 59A, 59B, and 60 in combination, it can be seen how the cartridges 68 in the cartridge carton 480 are loaded into the cartridge hopper 590. To load the cartridge hopper 590, the tab opening 482 is removed from the cartridge carton 480. The cartridge carton 480 is then positioned on the roller pins 484 with the hinge means 880 facing upward. A slight downward force on the hinge means 880 of the cartridge carton 480 will separate the perforations 821 and open the cartridge carton 480 to the maximum open position 481 as shown in phantom. The cartridges 68 will then be deposited in the cartridge hopper 680 in the correct laterally horizontal position. Some of the cartridges 658 will be deposited in an inverted orientation, however, the cartridge feeder assembly 500 will still be able to drop those inverted cartridges in an upright position into the feeder means because of the design of the cup pins 550a-b.

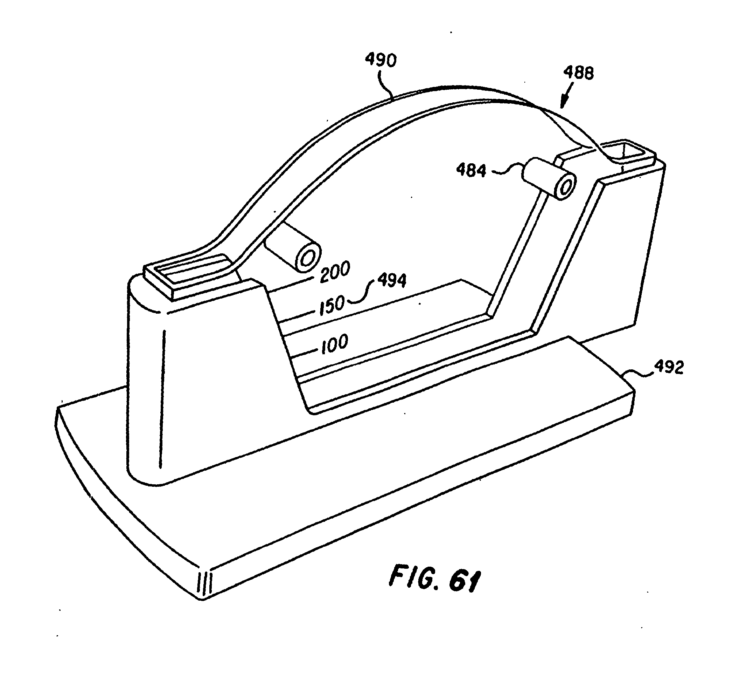

Referring now to FIG. 61, there is shown an isometric view of an alternate embodiment of a stand alone hopper 488 which is detachable from the remainder of the feed means, the stand alone hopper 488 being easily detached for loading purposes. The hopper presents cartridge availability indication 494 through a transparent wall portion for operator inspection. The stand alone hopper has an attached stand alone base or platform 492 for supporting the hopper during loading of multiple cartridges from a carton 480 as shown in FIGS. 59A, 59B, and 60, utilizing the roller pins 484.

### OPTICS CONTROL SYSTEM

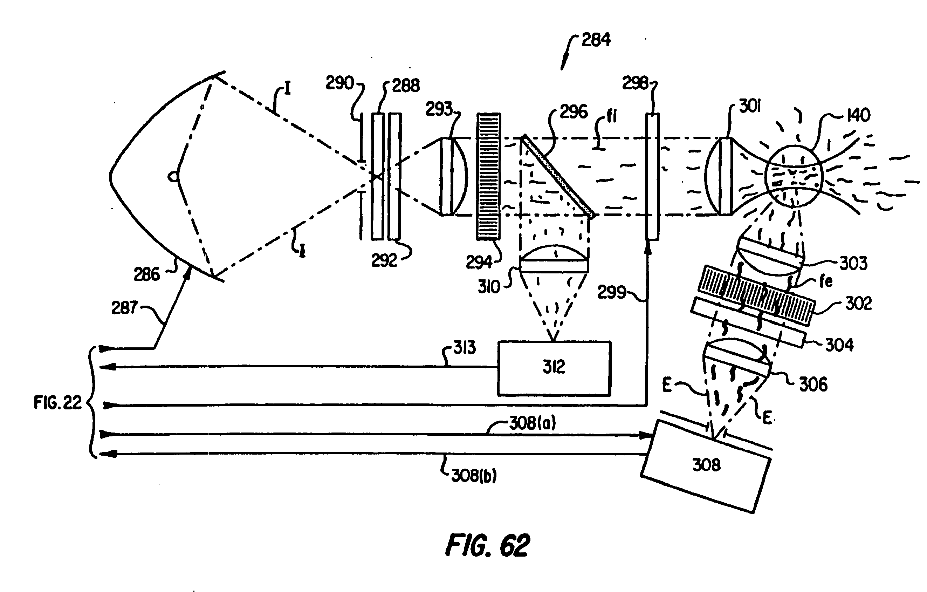

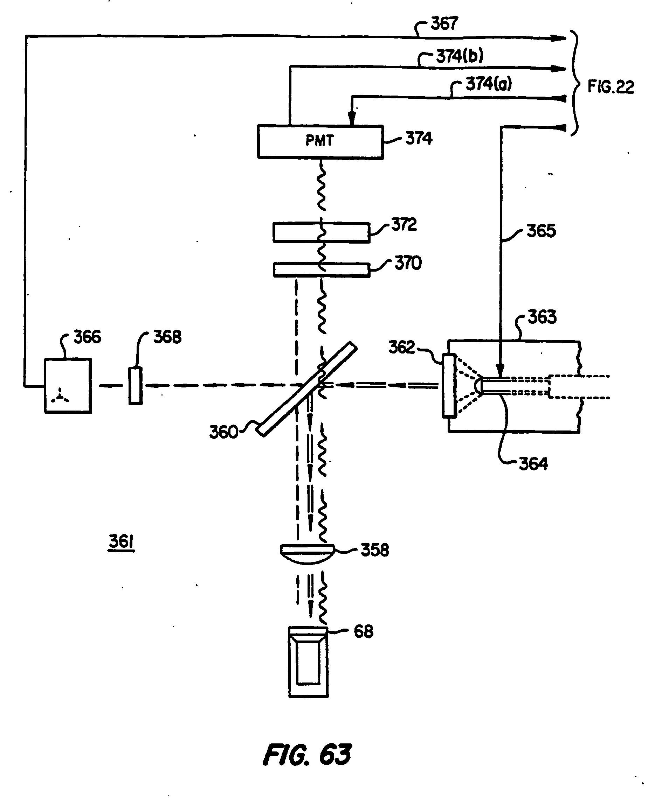

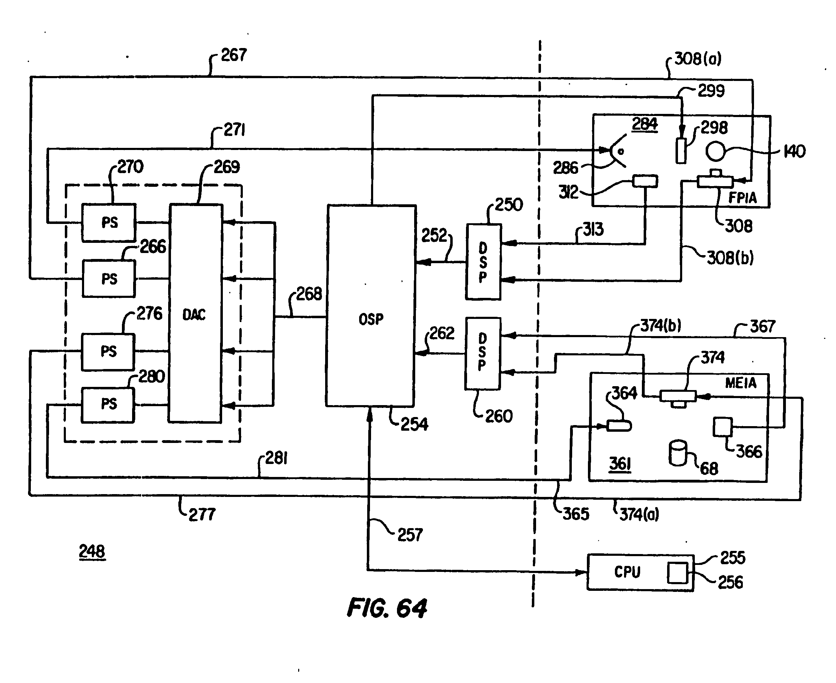

The system includes an optics control system shown generally at 248 in FIG. 64 which simultaneously and continuously manages in real time an optical system for the FPIA shown generally at 284 in FIG. 62 (the "FPIA optics system") and an optical system for the MEIA shown generally at 361 in FIG. 63 (the "MEIA optics system"), both of which contain optics used in Abbott's IMx^{®} and TDx^{®} analyzers which are well known in the art. The heart of the optics control system 248 is an optical signal processor 254 ("OSP") which is dedicated to the optics systems 284, 361 and communicates with the central processor 255 over a bidirectional bus 257. The scheduler 256 running on the central processor 255 sends macro-commands to the OSP 254 which interprets them and generates micro-commands for controlling the FPIA optics system 284 and the MEIA optics system 361. Although the scheduler 256 has a priori knowledge of what both optics systems 284, 361 will read because of its knowledge of the reagents, the OSP 254 collects data from both and transmits it back to the central processor 255 which continues operating the random access analytical system in real time. The OSP 254 has no such prior knowledge, but is essential for controlling, collecting and transmitting the large volume of data in real time.

To better understand how the optics control system 248 manages the FPIA and MEIA optics systems 284 and 361, both are defined more specifically as follows. Referring to FIG. 62, the light source for the FPIA optics system 284 is a tungsten halogen lamp 286 which provides a source of light energy for illuminating the FPIA reaction mixture in the cuvette .140 along an incident path I. The lamp 286 focuses the light through an aperture 290, a heat reflector 288, and heat absorber 292 to a plano convex lens 293 which collimates the light through an excitation filter 294 at a wavelength of 485 nm represented by the fine short lines fᵢ, i.e., the incident frequency. The collimated beam of light is split by a beamsplitter 296, the reflected portion being focused by a plano convex lens 310 to a reference detector 312, a photodiode, and the transmitted portion propagating through a transmissive liquid crystal 298 and focused by another plano concave lens 301 through the FPIA reaction mixture in the cuvette 140 which fluoresces at a higher wavelength of 535 nm represented by the darker short lines fₑ, i.e., the emitted frequency. A plano convex lens 306 collimates the light emitted from the fluorescing mixture along an emitted path E through an emission filter 302 and a polarizer 304 to another plano convex lens 306 which focuses the emitted light on a photo multiplier tube ("PMT") 308. Power is supplied to the lamp 286 and the PMT 308 via inputs 287 and 308(a), respectively, and control signals are sent to the liquid crystal 298 via an output 299 which controls the state of the liquid crystal 298 to be either vertically or horizontally polarized. The reference detector 312 provides an output 313 to the optical control system 248 which controls the input 287 to the lamp 286. The PMT 308 also provides an output 308(b) to the optical control system 248 which transmits data from the PMT 308 to the central processor 255.

Referring to FIG. 63, the light source for the MEIA optics system 361 is a mercury vapor lamp 364 which provides a source of light energy for illuminating the contents of the MEIA cartridge 68 along an incident path shown by the double-lined arrows. The light from the lamp 364 illuminates an excitation filter 362 which transmits the light at a wavelength of 365nm. Most of that light is reflected by a chromatic beamsplitter 360 and transmitted through a plano convex lens 358 that focuses the light into the open end of the MEIA cartridge 68. The remainder of the excitation light is transmitted through the chromatic beamsplitter 360 and illuminates an optical bandpass filter 368 which transmits 365nm to a reference detector 366, a photodiode, which provides an output 367 to the optical control system 248.

As a result of being exposed to excitation light energy, the contents of the MEIA cartridge 68 fluoresce at emission wavelengths which include 450nm, represented by the S-shaped arrows. The emission light is collected by a lens 358 and, because of the longer wavelength than the excitation, transmits through the chromatic beamsplitter 360. The emission proceeds through emission filters 370 and 372, which transmit light at 450nm, and finally illuminates a PMT 374. Power is supplied to the lamp 364 and the PMT 374 via inputs 365 and 374(a), respectively, and the PMT 374 correspondingly provides an output 374(b) to the optics control system 248 which transmits data from the PMT 374 to the central processor 255.

Another feature is the heater block 363 which maintains the temperature of the lamp 364 at a minimum temperature of about 70°C during periods of nonuse. This temperature must be high enough to ensure that the mercury in the lamp 364 remains in a vapor state to facilitate full brightness within about one second without adversely affecting the life of the lamp 364. The normal time period for changing from cold to full brightness is twenty (20) seconds. This one-second cycle time for the lamp 364 is necessary for high-speed operation in a continuous and random-access analytical system, which will be described in more detail below.

The FPIA and MEIA optics system 284, 361 and the optics control system 248 are shown in FIG. 64 separated by a dashed line. The output 308(b) from the PMT 308 and the output 313 from the reference detector 312 are analog inputs to a digital signal processor A/D chip 250 ("DSP") which can be, for example, the type supplied by Crystal Semiconductor. The DSP 250 converts the analog signals to digital signals and sends them to the OSP 254 via an input bus 252. The OSP 254 is an 8-bit microcontroller which can be, for example, an HC11 sold by Motorola. A digital output from the OSP 254 is provided to a digital to analog converter ("DAC") 269 via a serial output bus 268. Separate converter modules on the DAC 269 are connected to separate power supplies 266 and 270 which drive the PMT 308 and the lamp 286, respectively, via outputs 267 and 271, respectively. The OSP 254 cycles the lamp 286 according to macro-commands received from the scheduler 256 and, when turning the lamp 286 on, increases its intensity to provide sufficient illumination for the contents of the cuvette 140 based on data stored in the scheduler 256 and feedback from the reference detector 312. Typically, the illumination is set at about 200 microwatts at a wavelength of 485 nm as shown in FIG. 20. The data in the scheduler 256 is part of a table which prescribes the required sample illumination based on the reagents known to be used in that particular FPIA reaction mixture. The OSP 254 simultaneously adjusts the output gain of the PMT 308 in response to commands from the scheduler 256 based on the assay being conducted. The OSP 284 also controls the liquid crystal 298 via the output 299 by creating and removing an E-field to switch between vertical and horizontal polarization based on commands from the scheduler 256. As indicated above and throughout this paragraph, all of the knowledge regarding the assays and the reagents are resident in the scheduler 256 which relies on the OSP 254 for real-time execution in response to the macro-commands.

The same is true when applied to the MEIA optics system 361. The output 374(b) from the PMT 374 and output 367 from the reference detector 366 are analog inputs to another DSP 260 which converts the analog signals to digital signals for transmission to the OSP 254 via another input bus 262. The OSP 254 provides a digital output to separate converter modules on the DAC 269 via the serial output bus 268. These converter modules on the DAC 269 are connected to separate power supplies 276 and 280 which drive the PMT 374 and the lamp 364, respectively, via outputs 374(a) and 365, respectively. The OSP 254 cycles the lamp 364 according to micro-commands received the scheduler 256 and, when turning the lamp 364 on, increases its intensity to provide sufficient illumination for the contents for the MEIA cartridge 68 based on data stored in the scheduler 256 and feedback from the photo diode 366. Again, the data in the scheduler 256 is part of a table which prescribes the required sample illumination based on the reagents known to be used in that particular MEIA reaction mixture. The OSP 254 simultaneously adjusts the output gain of the PMT 374 in response to commands from the scheduler 256 based on the assay being conducted.

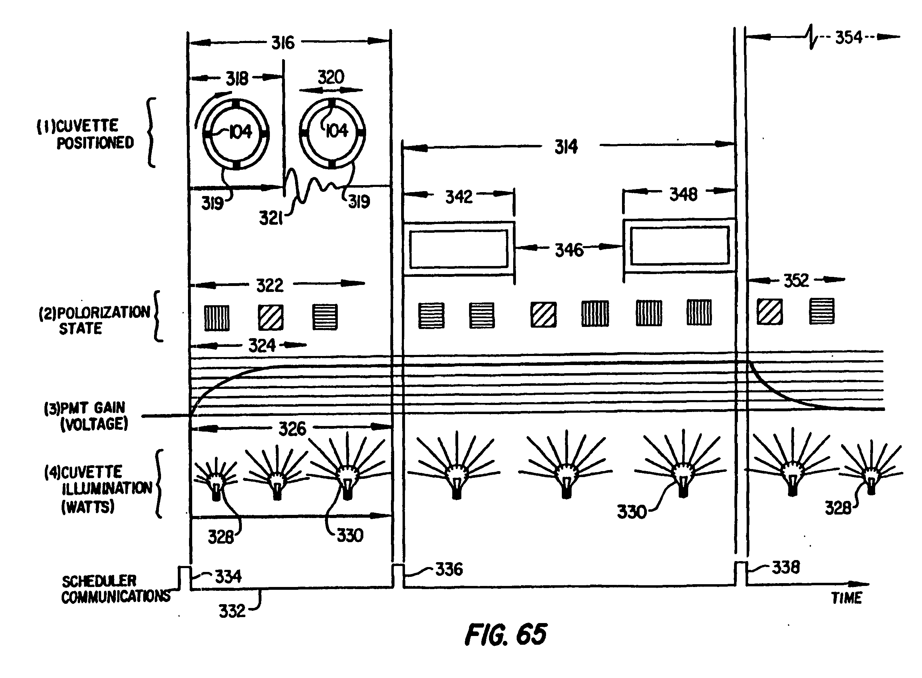

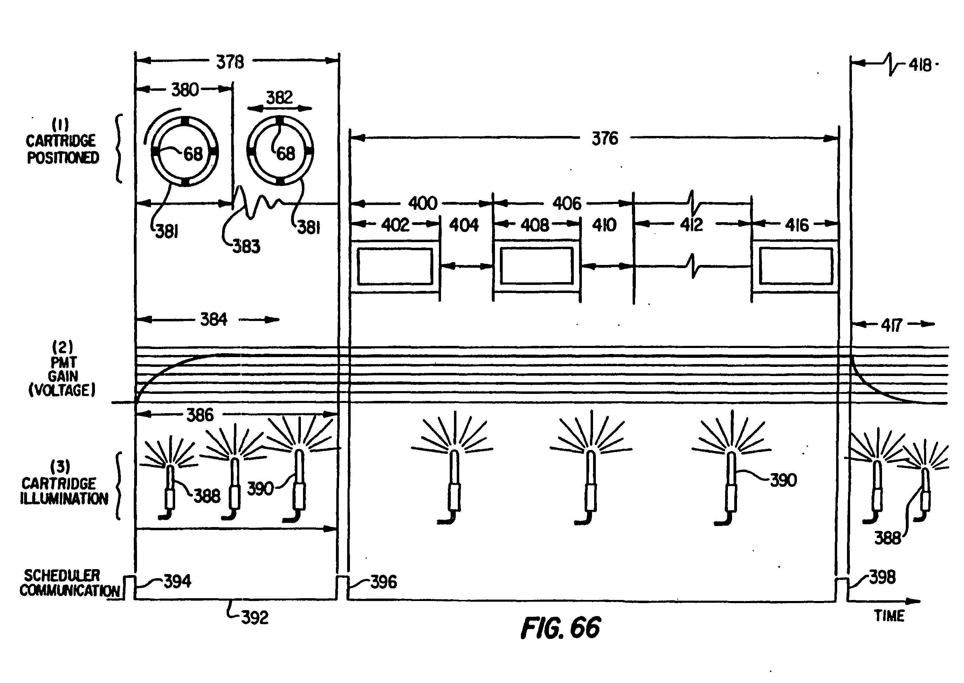

The operation of the optics control system 248 in conjunction with the FPIA and MEIA optics systems 284, 361 can best be shown by the pictorial time graphs in FIGS. 65 and 66, respectively, which illustrate a simultaneous sequence of events. Referring to FIG. 65, time is divided into the following operational periods: the preread activity period 316, the read sequence period 314, and the normalization period 352. Each operational period is initiated by communications between the scheduler 256 and the OSP 254 as represented by communication signals 334, 336, 338 on the time line 332. During the period of each communication signal 334, 336, 338, the scheduler 256 determines the amount of time necessary to simultaneously accomplish all the events required for the corresponding operational period which is initiated by the trailing edge of the communication signal. More specifically, when the scheduler 256 determines the duration of the preread activity period 316, the trailing edge of the communication signal 334 initiates the preread activity period 316 during which the following events occur: (1) the cuvette 140 is positioned by the carousel represented symbolically at 319 to be read by the PMT 308, (2) the polarization state of the liquid crystal 298 is properly set, (3) the gain of the PMT 308 is set, and (4) the intensity of the lamp 286 is increased to a level sufficient to illuminate the FPIA mixture in the cuvette 140.

During the first event, the scheduler 256 allots enough time 318 for the carousel 319 to rotate the cuvette 140 to the proper position to be read. When the carousel 319 stops, the scheduler 256 then allots' a predetermined amount of time 320 for the carousel 319 to stop moving or oscillating as indicated by the decaying sinusoidal curve 321. During the second event, the scheduler 256 allots enough time 322 for the OSP 254 to transition the liquid crystal 298 from a vertical state of polarization represented by the vertically-lined icon to a horizontal polarization represented by the horizontally-lined icon, the slanted-line icon therebetween representing the transition period. During the third event, the scheduler 256 allots enough time 324 for the OSP 254 to adjust the gain of the PMT 308. And finally, during the fourth event, the scheduler 256 allots enough time 326 for the OSP 254 to increase the intensity of the tungsten lamp 286 from a standby intensity 328, simmer state, to a higher full intensity 330, burn state, sufficient for illuminating the FPIA mixture in the cuvette 140. Cycling the lamp 286 from off to the full intensity 330 consumes too much time for a rapid and continuously operating analytical system and shortens the life of the lamp 286. The standby intensity 328 is sufficiently low to extend the life of the lamp 286, but sufficiently close to its thermal operating point to facilitate a rapid increase to the full intensity 330 required for illuminating the FPIA mixture within the allotted period of time 326. This feature is critical in a continuously operating analytical system not only because it extends the life of the lamp 286, but also because it stabilizes the full intensity 330 by maintaining an elevated temperature Although other events occur during the preread activity period 316, those just described are most relevant to the instant invention.

The scheduler 256 also determines the proper duration of the read sequence period 314 during the communication period 336, the trailing edge of which initiates the read sequence period 314 while holding the gain of the PMT 308 and the illumination of the tungsten lamp 286 constant after the preread-activity period 316. During the read sequence period 314, the scheduler 256 allots enough time 342 for the PMT 308 to sense the energy level of the light emitted from the fluorescing mixture in the cuvette 140 during horizontal polarization as represented by the two horizontally-lined icons and send the corresponding analog signals to the DSP 250. The scheduler 256 then allows enough time 346 for the OSP 254 to transition the liquid crystal 298 from horizontal to vertical polarization as represented by the slanted-line icon. At the end of the read sequence period 314, the scheduler 256 allots enough time 348 for the PMT 308 to sense the energy level of the light emitted from the fluorescing mixture in the cuvette 140 during vertical polarization as shown by the vertically-lined icons and send the corresponding analog signals to the DPS 250. After the read sequence period 314 and during the normalization period 352, the OSP 254 automatically returns the liquid crystal 298 back to its normal state as indicated by the icons, reduces the gain of the PMT 308, and reduces the intensity of the tungsten lamp 286 back to the standby intensity 328. The scheduler 256 is free to initiate another period sequence at any time during the period of unspecified length 354. The OSP 254 transmits all the data collected during the read sequence period 314 to the CPU 255 during the scheduler communication period 338.

The operation of the optics control system 248 in conjunction with the MEIA optic system 361 is shown in FIG. 66 wherein time is divided into the following similar operational periods: the preread activity period 378, the read sequence period 376, and the normalization period 417. Each operational period is initiated by communication between the scheduler 256 and the OSP 254 as represented by communication signals 394, 396, 398 on the time line 392. During the period of each communication signal 394, 396, 398, the scheduler 256 determines the amount of time necessary to simultaneously accomplish all the events required for the corresponding operational period which is initiated by the trailing edge of the communication signal. More specifically, when the scheduler 256 determines the duration of the preread activity period 378, the trailing edge of the communication signal 394 initiates the preread activity period 378 during which the following events occur: (1) the MEIA cartridge 68 is positioned by the carousel represented symbolically at 381 to be read by the PMT 374, (2) the gain of the PMT 374 is set, and (3) the intensity of the mercury vapor lamp 364 is increased to a level sufficient to illuminate the MEIA mixture in the MEIA cartridge 68.

During the first event, the scheduler 256 allots enough time 380 for the carousel 381 to rotate the cartridge 68 to the proper position to be read. When the carousel 381 stops, the scheduler 256 then allots a predetermined time 382 for the carousel 381 to stop moving or oscillating as indicated by the decaying sinusoidal curve 383. During the second event, the scheduler 256 allots enough time 384 for the OSP 254 to adjust the gain of the PMT 374. During the third event, the scheduler 256 allots enough time 386 for the OSP 254 to increase the intensity of the mercury lamp 364 from a standby intensity 388, simmer state, to a full intensity 390, burn state, sufficient for illuminating the MEIA mixture in the cartridge 68. Cycling the lamp 364 from off to the full intensity 390 consumes too much time for a rapid and continuously operating analytical system and shortens the life of the lamp 364. In order to extend the life of the lamp 364, a means for maintaining the thermal operating point of the lamp 364 must be employed for periods of time when the lamp 364 is not needed for illumination. Either of two methods are used. One method is to operate the lamp 364 at a current which is sufficiently low to extend the life of the lamp 364, but sufficiently close to its thermal operating point to facilitate a rapid increase to the full intensity 390 required for illuminating the MEIA mixture within the allotted period of time 386. The other method of maintaining the lamp 364 close to its thermal operating point is to encase the lamp 364 in a heater housing 363, which is controlled so as to maintain the lamp 364 at an elevated temperature of approximately 70 degrees C at all times. This feature is critical to a continuously operating analytical system not only because it extends the life of the lamp 364, but also because its stabilizes the full intensity 390 by maintaining an elevating temperature. Although other events occur during the preread activity period 378, those just described are most relevant to the instant invention.

The scheduler 256 also determines the proper direction of the read sequence period 376 during the communication period 396, the trailing edge of which initiates the read sequence period 376 while holding the gain of the PMT 364 and the illumination of the mercury vapor lamp 364 constant after the preread activity period 378. During the read sequence period 376, the scheduler 256 allocates enough time 400 for the PMT 374 to sense the energy level of light emitted from the fluorescing mixture in the cartridge 68 during a sub-read period 402 and send the corresponding analog signals to the DSP 260 during a dwell period 404. The read sequence period 376 continues with similar cycles like cycle 406, including sub-read period 408 and dwell period 410, as represented by the broken time line 412. After about eight (8) of such sub-readings depending upon the assay being performed, the read sequence period 376 concludes with a final sub-reading 416. After the read sequence period 376 and during the normalization period 417, the OSP 254 automatically reduces the gain of the PMT 374 and the intensity of the mercury vapor lamp 364 back to the standby intensity 388. The scheduler 256 is free to initiate another preread sequence at any time during the period of unspecified length 418. The OSP 254 also transmits all of the data collected during the read sequence period 376 to the CPU 255 during the scheduler communication period 398.

### KITTING AND PROCESS ACTIVITIES

It is to be appreciated that the following description comprises an outline of the various functions and steps involved in preferred methods of the automated analytical system of the invention, in exemplary kitting and process activities, which functions and methods as will be appreciated by those skilled in the art, are conducted under computer control using various types of mathematical algorithms and associated computer software, depending on the particular menu of assays being performed on the instrument.

### DESCRIPTION OF ACTIVITIES FOR FPIA ASSAY

### KITTING AREA

### A. ASSUMPTIONS

1. Analyzer is in Standby/Ready mode when sample is loaded. System has been previously initialized (All motors are homed, syringe and pumps are purged, all electronics and sensors are checked.)
2. Waste has been emptied, Diluent, MEIA buffer, MUP, and Quat bulk liquid consumables have been checked for sufficient volume.
3. All consumable inventory files have been updated.

### B. PREPARATION STEPS

1. User loads empty Reaction Vessel (RV) into RV carousel.
2. To load a reagent pack(s), the user must first pause the front end carousels. The system will complete kitting of the current test and transfer the test to the process area.
3. User opens the reagent carousel cover, loads reagent pack(s) into reagent carousel, closes the reagent carousel cover, then resumes the front-end.
4. Instrument automatically scans all reagent packs onboard to verify reagent status.
   (a) Each reagent pack is positioned in front of the reagent pack barcode reader by rotation of the reagent carousel.
   (b) Reagent pack barcode reader reads barcode to identify assay type and carousel location.
   (c) If the barcode is unreadable, the system will request a barcode override.
   (d) If the barcode is good or override complete, the system will check the system inventory. The user will be notified if the pack is found to be empty, invalid or outdated. Once the reagent pack is found to be good, it is ready to use.

### C. REQUESTING A TEST

1. User has two options for requesting a test or group of tests on one or more patient samples.
   (a) User may download the test request loadlist from a host computer to create an order list.
   (b) User enters test request or creates an order list on the System directly.
2. If sample cups (no barcode) are used, the following scenario occurs:
   (a) User refers to order list for segment ID and position number to place sample.
   (b) User loads a sample cup into referenced position in segment.
   (c) User transfers patient sample from blood collection tube into sample cup.
   (d) Segment is placed into sample carousel.
   (e) Indication is made to instrument that samples have been loaded.
   (f) Instrument checks consumable inventories, waste status, cal status, etc.
   (g) Sample carousel rotates segment to segment identification reader.
   (h) Instrument reads segment identification.
3. If primary tubes (with barcode) are used, the following scenario occurs (two types of carriers are used for primary tubes: one for tubes with heights of 75 mm and a second for tubes with heights of 100 mm. ):
   (a) User loads primary tube into next available segment location on sample carousel.
   (b) Indication is made to instrument that samples are available to be run.
   (c) Instrument checks consumable inventories, waste status, cal status, etc.

### D. SCHEDULING A TEST

1. When the sample is presented to the pipettor, the System attempts to schedule the tests ordered on that sample for processing. Each test ordered for the sample will be scheduled separately.
   (b) The System checks for adequate inventory (reagent packs, cartridges, buffer, MUP), system resources, sample time to complete the test.
   (c) The System checks for valid calibration or orders for them on the order list.
   (d) If all test requirements are met, the test is scheduled for processing.
   (a) If all test requirements are not met, the test request is moved to the exception list. Once the test requirements have been met, the test request is moved back to the order list by the user.
2. When a test has been scheduled, the System moves it to the processing list and attempts to schedule other tests ordered for that sample.
3. When all tests for the current sample have been kitted, the System advances to the next sample on the sample carousel.

### E. KITTING A TEST

1. Once a test is scheduled, it is immediately kitted. (No tests are kitted until the scheduler ensures that the test can be transferred onto the process carousel immediately and processed within the timing requirements of the assay. )
2. RV carousel is rotated clockwise until an RV is detected in pipette axis position.
3. Reagent pack carousel is rotated until reagent pack for test ordered is at the actuator position. The actuator opens the reagent cartridge caps and the reagent pack carousel is then rotated until a reagent pack for test ordered is in the pipette axis position. After all pipetting steps have been completed, the reagent pack carousel is rotated back to the actuator position where the reagent cartridge caps are closed.
4. Sample carousel is rotated until sample cup (or primary tube) is in pipette axis position.
5. Pipette is always at "HOME" position (Pipette R-axis is parked over wash station and Pipette Z-axis is at the Z-clear position) when not in use.
6. Sample kitting.

### (a) Sample aspirate.

(i) Syringe aspirates "X" µl of air at a rate of "X" µl/sec.
(ii) Pipette R-axis is moved over sample cup.
(iii) Pipette Z-axis is moved down to the Z-above position.
(iv) LLS is enabled to ensure that no liquid is currently detected.
(v) Pipette Z-axis is moved down at constant speed until fluid is detected or until Z-Asp limit has been reached (It will be assumed that fluid is detected)
(vi) Based on the Z-height position at which fluid is detected and the Z-height/volume table, the System calculates the volume of fluid in the well and compares it to the volume specified in the pipetting description. If sufficient volume is present in the well, the aspiration sequence is initiated (If insufficient volume is present, the test is aborted and the test request moved to the exception list. The exception list provides notice to an operator of tests which cannot be completed).
(vii) The following occur simultaneously until the total volume of sample required is aspirated:
   (1) Pipette Z-axis motor is moved down at a rate of "X" steps/sec.
   (2) Syringe motor aspirates "X" µl at a rate of "X" µl/sec.
   (3) Liquid Level Sense (LLS) is checked to ensure probe still in liquid. LLS is disabled. Pipette Z-axis is moved up to Z-clear position.
   (4) Pipette R-axis is moved over the RV sample well.
   (5) Pipette Z-axis is moved down to the dispense position within the RV sample well.
   (6) Syringe dispenses "X" µl of sample at a rate of "X" µl/sec.
   (7) Pipette Z-axis is moved up to Z-clear position.

### (b) Probe Post-Wash

The probe is washed to ensure that it is free from contamination. It is to be understood that all pipette activities (in both kitting and process areas) are followed with a probe post-wash to minimize carryover from one fluid aspirate to another. In some cases, pipette activities may be preceded with a probe prewash if necessary to guarantee the validity of the next fluid aspirate. For this assay description, it will be assumed that only a post-wash is used.
(i) The inside of the probe is cleaned first.
   (1) Pipette R-axis is moved over waste area.
   (2) Pipette Z-axis is moved down to appropriate position within the waste area.
   (3) The wash valve is opened for the amount of time specified in the assay protocol.
   (4) Wash valve is closed.
   (5) Pipette Z-axis is moved up to the Z-clear position.
(ii) The outside of the probe is cleaned next.
   (1) Pipette R-axis is moved over wash cup.
   (2) Pipette Z-axis is moved down to wash position within the wash cup.
   (3) The wash valve is opened for the amount of time specified in the assay protocol.
   (4) Wash valve is closed.
(iii) Pipette is returned to "HOME" position.

7. Popper kitting ("Popper" is defined as a substance which eliminates interfering substances in assays such as, for example, those discussed and claimed in U. S. Patent 4, 492, 762 issued January 8, 1985.)

### (a) Popper aspirate.

(i) Syringe aspirates "X" µl of air at a rate of "X" µl/sec.
(ii) Pipette R-Axis is moved over the popper reagent bottle in the Reagent Pack.
(iii) Pipette Z-axis is moved down to the Z-above position.
(iv) LLS is enabled to ensure no liquid currently detected.
(v) Pipette Z-axis is moved down at constant speed until fluid is detected or until the Z-aspiration-lower (Z-Asp) limit is reached (it will be assumed that fluid is detected).
(vi) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
(vii) The following occur simultaneously until the total volume of popper required is aspirated:
   (1) Pipette Z-axis motor is moved down at a rate of "X" steps/sec.
   (2) Syringe aspirates "X" µl at a rate of "X" µl/sec.
   (3) LLS is checked to ensure probe still in liquid.
   (4) LLS is disabled.
   (5) Pipette Z-axis is moved up to Z-clear position.
   (6) Pipette R-axis is moved over the RV reagent 1 well.
   (7) Pipette Z-axis is moved down to the dispense position within the RV reagent 1 well.
   (8) Syringe dispenses "X" µl of popper at a rate of "X" µl/sec.
   (9) Pipette Z-axis is moved up to Z-clear position.

### (b) Probe post-wash.

The probe is again washed to ensure that it is free from contamination as described in section 6 (Sample Kitting).

### 8. Antiserum kitting

### (a) Antiserum aspirate

(i) Syringe aspirates "X" µl of air at a rate of "X" µl/sec.
(ii) Pipette R-Axis is moved over the antiserum reagent bottle in the Reagent Pack.
(iii) Pipette Z-axis is moved down to the Z-above position.
(iv) LLS is enabled to ensure no liquid currently detected.
(v) Pipette Z-axis is moved down at constant speed until fluid is detected or until the Z-Asp limit is reached (it will be assumed that fluid is detected).
(vi) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
(vii) The following occur simultaneously until the total volume of antiserum required is aspirated:
   (1) Pipette Z-axis motor is moved down at a rate of "X" steps/sec.
   (2) Syringe aspirates "X" micro liter (µl) at a rate of "X" µl/sec. LLS is checked to ensure probe still in liquid.
   (3) LLS is disabled.
   (4) Pipette Z-axis is moved up to Z-clear position.
   (5) Pipette R-axis is moved over the RV reagent 2 well.
   (6) Pipette Z-axis is moved down to the dispense position within the RV reagent 2 well.
   (7) Syringe dispenses "X" µl of antiserum at a rate of "X" µl/sec.
   (8) Pipette Z-axis is moved up to Z-clear position.

### (b) Probe post-wash.

The probe is again washed to ensure that it is free from contamination as described in section 6 (Sample Kitting).

### 9. Tracer kitting.

### (a) Tracer aspirate.

(i) Syringe aspirates "X" µl of air at a rate of "X" µl(sec.
(ii) Pipette R-Axis is moved over the tracer reagent bottle in the Reagent Pack.
(iii) Pipette Z-axis is moved down to the Z-above position.
(iv) LLS is enabled to ensure no liquid currently detected.
(v) Pipette Z-axis is moved down at constant speed until fluid is detected or until the Z-Asp limit is reached (it will be assumed that fluid is detected).
(vi) Based on the Z-height position at which fluid is detected and the Z-height/volume table, the System calculates the volume of fluid in the well and compares it to the volume specified in the pipetting description. If sufficient volume is present in the well, the aspiration sequence is initiated. (if sufficient volume is not present, the test is aborted and the test request moved to the exception list).
(vii) The following occur simultaneously until the total volume of tracer required is aspirated:
   (1) Pipette Z-axis motor is moved down at a rate of "X" steps/sec.
   (2) Syringe aspirates "X" µl at a rate of "X" µl/sec.
   (3) LLS is checked to ensure probe still in liquid.
   (4) LLS is disabled.
   (5) Pipette Z-axis is moved up to Z-clear position.
   (6) Pipette R-axis is moved over the RV reagent 3 well.
   (7) Pipette Z-axis is moved down to the dispense position within the RV reagent 3 well.
   (8) Syringe dispenses "X" µl of tracer at a rate of "X" µl/sec.
   (9) Pipette Z-axis is moved up to Z-clear position.

### (b) Probe post-wash.

The probe is again washed to ensure that it is free from contamination as described in section 6 (Sample Kitting).

### F. TRANSFER OF REACTION VESSEL (RV) INTO PROCESS AREA

1. RV carousel is rotated to transfer station.
2. Process carousel is rotated so that the empty position is aligned with the transfer station.
3. Transfer mechanism 0-axis is rotated to sample entry area.
4. Transfer mechanism R-axis grabs the RV and pulls it into the transfer mechanism.
5. Transfer mechanism 0-axis is rotated so that RV is aligned with the empty position on the process carousel.
6. RV is loaded onto process carousel.

### PROCESS AREA

A. Wait for temperature equilibration time and evaporation window to expire.
B. FIRST PIPETTE ACTIVITY (preparation of sample blank comprising diluted sample and popper).
   1. Incubation timer is set according to assay file specifications.
   2. Precision diluent aspirate. The following activities are performed simultaneously:
      (a) Syringe aspirates "X" µl at a rate of "X" µl/sec.
      (b) Wash valve is opened.
      (c) Wait "n" seconds.
      (d) Wash valve is closed.
   3. Sample aspirate.
      (a) Pipette R-axis is moved over the RV sample well.
      (b) LLS is enabled to ensure no liquid' currently detected.
      (c) Pipette Z-axis is moved down at constant speed until fluid is detected OR until the Z-Asp limit is reached (it will be assumed that fluid is detected).
      (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (e) The following occur simultaneously until the total volume of sample required is aspirated:
         (i) Pipettor Z-axis motor is moved down at a rate of "X" steps/sec.
         (ii) Syringe aspirates "x" µl of sample at a rate of "X" µl/sec.
         (iii) LLS is checked to ensure probe still in liquid.
         (iv) LLS is disabled.
         (v) Pipette Z-axis is moved up to Z-above position.
   4. Diluent/sample dispensed to the RV predilute well.
      (a) Pipette R-axis is moved over the RV predilute well.
      (b) Pipette Z-axis is moved down to the dispense position within the RV predilute well.
      (c) Syringe dispenses "X" µl of diluent/sample at a rate of "X" µl/sec.
      (d) Pipette Z-axis is moved up to Z-clear position.
   5. Probe post-wash.
      The probe is again washed to ensure that it is free from contamination as described in section 6 (Sample kitting).
   6. Precision diluent aspirate. The following activities are performed simultaneously:
      (a) Syringe aspirates "X" µl at a rate of "X" µl/sec.
      (b) Wash valve is opened.
      (c) Wait "n" seconds.
      (d) Wash valve is closed.
   7. Popper aspirate.
      (a) Pipette R-axis is moved over the RV Reagent (popper) well.
      (b) LLS is enabled to ensure no liquid currently detected.
      (c) Pipette Z-axis is moved down at constant speed until fluid is detected or until the Z-Asp limit is reached (it will be assumed that fluid is detected).
      (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (e) The following occur simultaneously until the total volume of popper required is aspirated:
         (i) Pipette Z-axis motor is moved down at a rate of "X" steps/sec.
         (ii) Syringe aspirates "X" µl at a rate of "x" µl/sec.
         (iii) LLS is checked to ensure probe still in liquid.
         (iv) LLS is disabled.
         (v) Pipette Z-axis is moved up to the Z-above position.
   8. Diluted sample aspirate.
      (a) Pipette R-axis is moved over the RV predilute well.
      (b) LLS is enabled to ensure no liquid currently detected.
      (c) Pipette Z-axis is moved down at constant speed until fluid is detected or until the Z-Asp limit is reached (it will be assumed that fluid is detected).
      (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (e) The following occur simultaneously until the total volume of diluted sample required is aspirated:
         (i) Pipette Z-axis motor is moved down at a rate of "X" steps/sec.
         (ii) Syringe aspirates "X" µl at a rate of "x" µl/sec.
         (iii) LLS is checked to ensure probe still in liquid.
         (iv) LLS is disabled.
         (v) Pipette Z-axis is moved up to the Z-above position.
   11. Diluted sample/popper diluent dispensed to RV cuvette.
      (a) Pipette R-axis is moved over to the RV cuvette position.
      (b) Pipette Z-axis is moved down to the dispense position in the RV cuvette.
      (c) Syringe dispenses "X" µl of diluted sample/popper/diluent at a rate of "X" µl/sec.
      (d) Pipette Z-axis is moved up to the Z-above position.
   12. Probe post-wash.
      The probe is again washed to ensure that it is free from contamination as described in section 6 (sample kitting) to complete first pipette activity.
C. BLANK PREREAD ACTIVITY PERIOD (allotted time 378). When the incubation timer expires, the FPIA optics system 284 simultaneously performs the following activities in response to the scheduler 256 and the optics control system 248 as described above in more detail:
   1. The carousel 46 positions the cuvette 140 for a reading.
   2. The polarization state of the liquid crystal 298 is set.
   3. The gain of the PMT 308 is set.
   4. The intensity of the lamp 286 is increased from the simmer state to the burn state.
D. BLANK READ SEQUENCE PERIOD (allotted time 314). The FPIA optics system 284 then performs the following in response to the scheduler 256 and the optics control system 248 as described above:
   1. The intensity with horizontal polarization is read during the allotted time period 342.
   2. The state of the liquid crystal 298 is transitioned to vertical polarization, allowing enough time for settling, during the allotted time 246.
   3. The intensity with vertical polarization is read during the allotted time period 348.
   4. The OSP 254 converts the digitized analog signals from the PMT 308 to normalized readings by comparing the intensity at the detector 312 to the intensity of the lamp 286 ("background readings").
E. BLANK NORMALIZATION PERIOD.
   1. The OSP 254 transmits the background readings to the CPU 255 to be stored.
   2. The intensity of the lamp 286 is decreased back to the simmer state.
F. SECOND PIPETTE ACTIVITY (for reaction between diluted sample, popper, tracer and antiserum).
   1. Incubation timer is set according to assay file specifications.
   2. Precision diluent aspirate.
      (a) The following activities are performed simultaneously:
         (i) Syringe aspirates "X" µl at a rate of "X" µl/sec.
         (ii) Wash valve is opened.
         (iii) Wait "n" seconds.
         (iv) Wash valve is closed.
   3. Antiserum aspirate.
      (i) Pipette R-axis is moved over the RV Reagent 2 (antiserum) well.
      (ii) LLS is enabled to ensure no liquid currently detected.
      (iii) Pipette Z-axis is moved down at constant speed until fluid is detected OR until the Z-Asp limit is reached (it will be assumed that fluid is detected).
      (iv) Based on the Z-height position at which fluid is detected and the Z-height/volume table, the System calculates the volume of fluid in the well and compares it to the volume specified in the pipetting description. If sufficient volume is present, the aspiration sequence is initiated. (If sufficient volume is not present, the test is aborted and the test request moved to the exception list.)
      (v) The following occur simultaneously until the total volume of antiserum required is aspirated:
         (1) Pipette Z-axis motor is moved down at a rate of "X" steps/sec.
         (2) Syringe aspirates "X" µl at a rate of "X" µl/sec.
         (3) LLS is checked to ensure probe still in liquid.
         (4) LLS is disabled.
         (5) Pipette Z-axis is moved up to the Z-above position.
   4. Tracer aspirate.
      (a) Syringe aspirates "X" µl of air at a rate of "X" µl/sec.
      (b) Pipette R-axis is moved over the RV Reagent 3 (tracer) well.
      (c) LLS is enabled to ensure no liquid currently detected.
      (d) Pipette Z-axis is moved down at constant speed until fluid is detected OR until the Z-Asp limit is reached (it will be assumed that fluid is detected).
      (e) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (f) The following occur simultaneously until the total volume of tracer required is aspirated:
         (i) Pipette Z-axis motor is moved down at a rate of "X" steps/sec.
         (ii) Syringe aspirates "X" µl at a rate of "X" µl/sec.
         (iii) LLS is checked to ensure probe still in liquid.
         (v) LLS is disabled.
         (vi) Pipette Z-axis is moved up to the Z-above position.
   5. Diluted sample aspirate.
      (a) Pipette R-axis is moved over the RV predilute well.
      (b) LLS is enabled to ensure no liquid currently detected.
      (c) Pipette Z-axis is moved down at constant speed until fluid is detected OR until the Z-Asp limit is reached (it will be assumed that fluid is detected).
      (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list.)
      (e) The following occur simultaneously until the total volume of diluted sample required is aspirated:
         (1) Pipette Z-axis motor is moved down at a rate of "X" steps/sec.
         (2) Syringe aspirates "X" µl at a rate of "X" µl/sec.
         (3) LLS is checked to ensure probe still in liquid.
         (4) LLS is disabled.
         (5) Pipette Z-axis is moved up to the Z-above position.
   6. Diluted sample/tracer/aspirate/antiserum/diluent dispensed to RV cuvette.
      (a) Pipette R-axis is moved over to the RV cuvette position.
      (b) Pipette Z-axis is moved down to the dispense position in the RV cuvette.
      (c) Syringe dispenses "X" µl of diluted sample/tracer/air/antiserum/diluent at a rate of "X" µl/sec.
      (d) Pipette Z-axis is moved up to the Z-above position.
   7. Probe post-wash.
      The probe is again washed to ensure that it is free from contamination as described in section 6 (Sample kitting) to complete the second pipette activity.
   8. Next activity started when incubation timer expires.
G. FINAL PREREAD ACTIVITY PERIOD (allotted time 378). When the incubation timer expires, the FPIA optics system 284 simultaneously performs the following activities in response to the scheduler 256 and the optics control system 248 as described above in more detail:
   1. The carousel 46 positions the cuvette 140 for a reading.
   2. The polarization state of the liquid crystal 298 is set.
   3. The gain of the PMT 308 is set.
   4. The intensity of the lamp 286 is increased from the simmer state to the burn state.
H. FINAL READ SEQUENCE PERIOD (allotted time 314). The FPIA optics system 284 then performs the following in response to the scheduler 256 and the optics control system 248 as described above:
   1. The intensity with horizontal polarization is read during the allotted time period 342.
   2. The state of the liquid crystal 298 is transitioned to vertical polarization, allowing enough time for settling, during the allotted time 246.
   3. The intensity with vertical polarization is read during the allotted time period 348.
   4. The OSP 254 converts the digitized analog signals from the PMT 308 to normalized readings by comparing the intensity at the detector 312 to the intensity of the lamp 286 (" final readings").
I. FINAL NORMALIZATION PERIOD:
   1. The OSP 254 transmits the final readings to the CPU 255 to be stored.
   2. The CPU 255 is used to calculate the NET intensity (I) and the millipolarization (mP) which is calibrated against a standard curve to determine a concentration result.
   3. The intensity of the lamp 286 is decreased back to the simmer state.
J. RV UNLOAD (this activity occurs when resources are not in use). The following are performed simultaneously:
   1. Process carousel is rotated so that the empty position is at the transfer station. Transfer mechanism 0-axis is moved to process carousel.
   2. RV is grabbed with the transfer mechanism R-axis and pulled into the transfer mechanism.
   3. Transfer mechanism O-axis is rotated so that RV is aligned with the waste container.
   4. RV is pushed into the waste container.

### DESCRIPTION OF ACTIVITIES FOR MERA ASSAY

### KITTING AREA

### A. ASSUMPTIONS

1. Analyzer is in Standby/Ready mode when sample is loaded. System has been previously initialized (All motors are homed, syringe and pumps are purged, all electronics and sensors are checked).
2. Waste has been emptied, dilution, MEIA buffer, MUP, and Quat bulk liquid consumables have been checked for sufficient volume.
3. Cartridges have been placed into hopper and are available for loading onto auxiliary carousel when needed (for MEIA assays only).
4. All consumable inventory files have been updated.

### B. PREPARATION STEPS

1. User loads empty RVs into RV carousel.
2. To load a reagent pack(s), the user must first pause the front end carousels. The system will complete kitting of the current test and transfer the test to the process area.
3. User opens the reagent carousel, loads reagent pack(s) into reagent carousel, closes the reagent carousel cover, then resumes the front-end.
4. Instrument automatically scans all reagent packs onboard to verify reagent status.
5. Each reagent pack is positioned in front of the reagent pack barcode reader by rotation of the reagent carousel.
6. Reagent pack barcode reader reads barcode to identify assay type and carousel location. If the barcode is unreadable, the system will request a barcode override.
7. If the barcode is good or override complete, the system will check the system inventory. The user will be notified if the pack is found to be empty, invalid or outdated. Once the reagent pack is found to be good, it is ready to use.

### C. REQUESTING A TEST

1. User has two options for requesting a test or group of tests on one or more patient samples.
   (a) User may download the test request loadlist from a host computer to create an order list.
   (b) User enters test request or creates an order list on the System directly.
2. If sample cups (no barcode) are used, the following scenario occurs:
   (a) User refers to order list for segment ID and position number to place sample.
   (b) User loads a sample cup into referenced position in segment.
   (c) User transfers patient sample from blood collection tube into sample cup.
   (d) Segment is placed into sample carousel.
   (e) Indication is made to instrument that samples have been loaded.
   (f) Instrument checks consumable inventories, waste status, assay calibration, etc.
   (g) Sample carousel rotates segment to segment identification reader.
   (h) Instrument reads segment identification.
3. If primary tubes (with barcode) are used, the following scenario occurs:
   (a) User loads primary tube into next available segment location on sample carousel (two types of carriers are used for primary tubes: one for tubes with heights of 75 mm and a second for tubes with heights of 100 mm.).
   (b) Indication is made to instrument that samples are available to be run.
   (c) Sample carousel rotates segment to segment identification reader.

### D. SCHEDULING A TEST

1. When the sample is presented to the pipettor, the System attempts to schedule the tests ordered on that sample for processing. Each test ordered for the sample will be scheduled separately.
   (a) The System checks for adequate inventory (reagent packs, cartridges, buffer, MUP), system resources, sample time to complete the test.
   (b) The System checks for valid calibration or orders for them on the order list.
   (c) If all test requirements are met, the test is scheduled for processing.
   (d) If all test requirements are not met, the test request is moved to the exception list. Once the test requirements have been met, the test request is moved back to the order list by the user.
2. When a test has been scheduled, the system moves it to the processing list and attempts to schedule other tests ordered for that sample.
3. When all tests for the current sample have been kitted, the System advances to the next sample on the sample carousel.

### E. KITTING A TEST

1. Once a test is scheduled, it is immediately kitted. (no tests are kitted until the scheduler ensures that the test can be transferred onto the process carousel immediately and processed within the timing requirements of the assay).
2. RV carousel is rotated clockwise until an RV is detected in pipette axis position.
3. Reagent pack carousel is rotated until reagent pack for test ordered is at the actuator position. The actuator opens the reagent cartridge caps and the reagent pack carousel is then rotated until reagent pack for test ordered is in the pipette axis position. After all pipetting steps have been completed, the reagent pack carousel is rotated back to the actuator position where the reagent cartridge caps are closed.
4. Sample carousel is rotated until sample cup (or primary tube) is in pipette axis position.
5. Pipette is always at HOME position (Pipette R-axis is parked over wash station and Pipette Z-axis is at the Z-clear position) when not in use.
6. Sample kitting.
   (a) Sample aspirate.
      (i) Syringe aspirates "X" µl of air at a rate of "X" µl/sec.
      (ii) Pipette R-axis is moved over sample cup.
      (iii) Pipette Z-axis is moved down to the Z-above position.
      (iv) Pipette Z-axis is moved down to the Z-LLS position.
      (v) LLS is enabled to ensure that no liquid is currently detected.
      (vi) Pipette Z-axis is moved down at constant speed until fluid is detected or until Z-Asp limit has been reached (it will be assumed that fluid is detected).
      (vii) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
      (viii) The following occur simultaneously until the total volume of sample required is aspirated:
         (1) Pipette Z-axis motor is moved down at a rate of "X" steps/sec.
         (2) Syringe aspirates "X" µl at a rate of "X" µl/sec.
         (3) LLS is checked to ensure probe still in liquid.
         (4) LLS is disabled.
         (5) Pipette Z-axis is moved up to Z-clear position.
         (6) Pipette R-axis is moved over the RV sample well.
         (7) Pipette Z-axis is moved down to the dispense position within the RV sample well.
         (8) Syringe dispenses "X" µl of sample at a rate of "X" µl/sec.
         (9) Pipette Z-axis is moved up to Z-clear position.
   (b) Probe post-wash.
      The probe is washed to ensure that it is free from contamination. It is to be understood that pipette activities in both kitting and process areas are generally followed with a probe post-wash to minimize carryover from one fluid aspirate to another. In some cases, pipette activities may be preceded with a probe prewash if necessary to guarantee the validity of the next fluid aspirate. For this assay description, it will be assumed that only a post-wash is used.
      (i) The inside of the probe is cleaned first.
         (1) Pipette R-axis is moved over waste area.
         (2) Pipette Z-axis is moved down to appropriate position within the waste area.
         (3) The wash valve is opened for the amount of time specified in the assay protocol.
         (4) Wash valve is closed.
      (ii) Pipette Z-axis is moved up to the Z-clear position.
      (iii) The outside of the probe is cleaned next.
         (1) Pipette R-axis is moved over wash cup.
         (2) Pipette Z-axis is moved down to wash position within the wash cup.
         (3) The wash valve is opened for the amount of time specified in the assay protocol.
         (4) Wash valve is closed.
         (5) Pipette is returned to "HOME" position.
7. Microparticle kitting.
   (a) Microparticle aspirate (microparticles are pipetted directly into the RV incubation well to save on volume, as this is the most costly MEIA reagent).
      (i) Syringe aspirates "X" µl of air at a rate of "X" µl/sec.
      (ii) Pipette R-Axis is moved over the microparticle reagent bottle in the Reagent Pack.
      (iii) Pipette Z-axis is moved down to the Z-above position.
      (iv) Pipette Z-axis is moved down to the Z-LLS position.
      (v) LLS is enabled to ensure no-liquid currently detected.
      (vi) Pipette Z-axis is moved down at constant speed until fluid is detected or until the Z-Asp limit is reached (it will be assumed that fluid is detected)
      (vii) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
      (viii) The following occur simultaneously until the total volume of microparticles required is aspirated:
         (1) Pipette Z-axis motor is moved down at a rate of "X" steps/sec.
         (2) Syringe aspirates "X" µl at a rate of "X" µl/sec.
         (3) LLS is checked to ensure probe still in liquid.
      (ix) LLS is disabled.
      (x) Pipette Z-axis is moved up to Z-clear position.
      (xi) Pipette R-axis is moved over the RV incubation well.
      (xii) Pipette Z-axis is moved down to the dispense position within the RV incubation well.
      (xiii) Syringe dispenses "X" µl of microparticles at a rate of "X" µl/sec. Pipette Z-axis is moved up to Z-clear position.
   (b) Probe post-wash.
      The probe is again washed to ensure that it is free from contamination as described in section 6 (Sample kitting).
8. Conjugate kitting.
   (a) Conjugate aspirate (conjugate, special wash fluid, and/or specimen diluent are pipetted into either RV reagent wells or RV predilution well, depending on volume requirements).
      (i) Syringe aspirates "X" µl of air at a rate of "X" µl/sec.
      (ii) Pipette R-Axis is moved over the conjugate reagent bottle in the Reagent Pack.
      (iii) Pipette Z-axis is moved down to the Z-above position.
      (iv) Pipette Z-axis is moved down to the Z-LLS position.
      (v) LLS is enabled to ensure no liquid currently detected.
      (vi) Pipette Z-axis is moved down at constant speed until fluid is detected or until the Z-Asp limit is reached (it will be assumed that fluid is detected).
      (vii) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
      (viii) The following occur simultaneously until the total volume of conjugate required is aspirated:
         (1) Pipette Z-axis motor is moved down at a rate of "x" steps/sec.
         (2) Syringe aspirates "X" µl at a rate of "X" µl/sec.
         (3) LLS is checked to ensure probe still in liquid.
      (ix) LLS is disabled.
      (x) Pipette Z-axis is moved up to Z-clear position.
      (xi) Pipette R-axis is moved over the RV reagent well.
      (xii) Pipette Z-axis is moved down to the dispense position within the RV reagent well.
      (xiii) Syringe dispenses "X" µl of conjugate at a rate of "X" µl/sec.
      (xiv) Pipette Z-axis is moved up to Z-clear position.
   (b) Probe post-wash.
      The probe is again washed to ensure that it is"free from contamination as described in section 6 (Sample kitting).
9. MEIA Buffer Kitting.
   (a) RV Carousel is rotated until RV buffer well is under the MEIA buffer dispenser at buffer kitting station.
   (b) "X" µl of MEIA buffer is dispensed into the buffer well at a rate of "X" µl/sec

### F. TRANSFERRING RV INTO PROCESS AREA

1. RV carousel is rotated to transfer station.
2. Process carousel is rotated so that the empty position is aligned with the transfer station.
3. Transfer mechanism 0-axis is rotated to sample entry area.
4. Transfer mechanism R-axis grabs the RV and pulls it into the transfer mechanism.
5. Transfer mechanism 0-axis is rotated so that RV is aligned with the empty position on the process carousel.
6. RV is loaded onto process carousel.

### PROCESS AREA

A. System waits for temperature equilibration time and evaporation window to expire.
B. FIRST PIPETTE ACTIVITY (microparticle/sample reaction)
   1. Incubation timer is set according to assay file specifications.
   2. MEIA buffer aspirate.
      (a) The process carousel is moved so that the RV is at the pipetting station.
      (b) Syringe aspirates "X" µl of air at a rate of "X" µl/sec.
      (c) Pipette R-axis is moved over the RV buffer well.
      (d) Pipette Z-axis is moved down to the Z-above position over the RV buffer well.
      (e) Pipette Z-axis is moved down to the Z-LLS position.
      (f) LLS is enabled to ensure no liquid currently detected.
      (g) Pipette Z-axis is moved down at constant speed until fluid is detected or until the Z-Asp limit is reached (it will be assumed that fluid is detected).
      (h) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (i) The following occur simultaneously until the total volume of MEIA buffer required is aspirated:
         (1) Pipette Z-axis motor is moved down at a rate of "X" steps/sec.
         (2) Syringe aspirates "X" µl at a rate of "X" µl/sec.
      (j) LLS is checked to ensure probe still in liquid.
      (k) LLS is disabled.
      (l) Pipette Z-axis is moved up to Z-above position.
   3. Sample aspirate
      (a) Pipette R-axis is moved over the RV sample well.
      (b) Pipette Z-axis is moved down to the Z-LLS position.
      (c) LLS is enabled to ensure no liquid currently detected.
      (d) Pipette Z-axis is moved down at constant speed until fluid is detected or until the Z-Asp limit is reached (it will be assumed that fluid is detected).
      (e) Based on the Z-height position at which fluid is detected and the Z-height/volume table, the system calculates the volume of fluid in the well and compares it to the volume specified in the pipetting description. If sufficient volume is present, the aspiration sequence is initiated (if sufficient volume is not present, the test is aborted and the test request moved to the exception list).
      (f) The following occur simultaneously until the total volume of sample required is aspirated:
         (1) Pipettor Z-axis motor is moved down at a rate of "X" steps/sec.
         (2) Syringe aspirates "X" µl at a rate of "X" µl/sec.
      (g) LLS is checked to ensure probe still in liquid.
      (h) LLS is disabled.
      (i) Pipette Z-axis is moved up to Z-above position.
   4. MEIA buffer and sample are added to microparticles in incubation well.
      (a) Pipette Z-axis is moved down to the dispense position within the RV incubation well.
      (b) Syringe dispenses "X" µl of MEIA buffer and sample at a rate of "X" µl/sec.
      (c) Pipette Z-axis is moved up to Z-clear position.
   5. Probe post-wash.
      The probe is again washed to ensure that it is free from contamination as described in section 6 (Sample kitting).
C. CARTRIDGE LOAD (This activity occurs when resources are not in use)
   1. Move the auxiliary carousel so that reserved position is under feeder.
   2. Cycle trap-door mechanism to load flashlight into carousel.
   3. Cycle shuttle mechanism to place another MEIA cartridge on trap door (for next tab load).
   4. Check incubation timer. When expires start next pipetting.
D. SECOND PIPETTE ACTIVITY (transfer of reaction mixture to matrix)
   1. Incubation timer is set according to assay file specifications.
   2. Buffer aspirate.
      (a) The process carousel is moved so that the RV is at the pipetting station.
      (b) Syringe aspirates "X" µl of air at a rate of "X" µl/sec.
      (c) Pipette R-axis is moved over the RV buffer well.
      (d) Pipette Z-axis is moved down to the Z-above position.
      (e) Pipette Z-axis is moved down to the Z-LLS position.
      (f) LLS is enabled to ensure no liquid currently detected.
      (g) Pipette Z-axis is moved down at constant speed until fluid is detected or until the Z-Asp limit is reached (it will be assumed that fluid is detected).
      (h) Based on the Z-height position at which fluid is detected and the Z-height/volume table, the system calculates the volume of fluid in the well and compares it to the volume specified in the pipetting description. If sufficient volume is present, the aspiration sequence is initiated (if sufficient volume is not present, the test is aborted and the test request moved to the exception list).
      (i) The following occur simultaneously until the total volume of buffer required is aspirated:
         (1) Pipette Z-axis motor is moved down at a rate of "X" steps/sec.
         (2) Syringe aspirates "X" µl at a rate of "X" µl/sec.
      (j) LLS is checked to ensure probe still in liquid.
      (k) LLS is disabled.
      (l) Pipette Z-axis is moved up to the Z-above position.
   3. Reaction mixture aspirate.
      (a) Pipette R-axis is moved over the RV incubation well.
      (b) Pipette Z-axis is moved down to the Z-LLS position.
      (c) LLS is enabled to ensure no liquid currently detected.
      (d) Pipette Z-axis is moved down at constant speed until fluid is detected or until the Z-Asp limit is reached (it will be assumed that fluid is detected).
      (e) Based on the Z-height position at which fluid is detected and the Z-height/volume table, the system calculates the volume of fluid in the well and compares it to the volume specified in the pipetting description. If sufficient volume is present, the aspiration sequence is initiated (if sufficient volume is not present, the test is aborted and the test request moved to the exception list).
      (f) The following occur simultaneously until the total volume of reaction mixture required is aspirated:
         (1) Pipette Z-axis motor is moved down at a rate of "X" steps/sec.
         (2) Syringe aspirates "X" µl at a rate of "X" µl/sec.
      (g) LLS is checked to ensure probe still in liquid.
      (h) LLS is disabled.
      (i) Pipette Z-axis is moved up to the Z-clear position.
   4. Reaction mixture dispense onto matrix.
      (a) The following are performed simultaneously and concurrently with the reaction mixture aspirate (above):
         (i) The auxiliary carousel is moved so that the cartridge is at the pipetting station.
         (ii) Pipette R-axis is moved over the MEIA cartridge (matrix) surface.
         (iii) Pipette Z-axis is moved down to the matrix dispense position.
         (iv) Syringe dispenses "X" µl of reaction mixture at a rate of "X" µl/sec.
         (v) System delays "X" seconds until reaction mixture has been absorbed by matrix.
   5. Buffer wash of matrix.
      (a) Syringe dispenses "X" µl of buffer at a rate of "X" µl/sec.
      (b) Pipette Z-axis is moved up to the Z-clear position.
   6. Probe post-wash.
      The probe is again washed to ensure that it is free from contamination as described in section 6 (Sample kitting).
   7. When incubation timer expires, next pipette activity begins.
E. THIRD PIPETTE ACTIVITY (conjugate addition)
   1. Incubation timer is set according to assay file specifications.
   2. Conjugate aspirate.
      (a) The process carousel is moved so that the RV is at the pipetting station.
      (b) Syringe aspirates "X" µl of air at a rate of "X" µl/sec.
      (c) Pipette R-axis is moved over the RV reagent 1 (conjugate) well.
      (d) Pipette Z-axis is moved down to the Z-above position.
      (e) LLS is enabled to ensure no liquid currently detected.
      (f) Pipette Z-axis is moved down at constant speed until fluid is detected or until the Z-Asp limit is reached (it will be assumed that fluid is detected).
      (g) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (h) The following occur simultaneously until the total volume of conjugate required is aspirated:
         (i) Pipette Z-axis motor is moved down at a rate of "X" steps/sec.
         (ii) Syringe aspirates "X" µl at a rate of "X" µl/sec.
      (i) LLS is checked to ensure probe still in liquid.
      (j) LLS is disabled.
      (k) Pipette Z-axis is moved up to the Z-clear position.
   3. Conjugate dispense (performed simultaneously).
      (a) The auxiliary carousel is moved so that the cartridge is at the pipetting station.
      (b) Pipette R-axis is moved over the cartridge (matrix) surface.
      (c) Pipette Z-axis is moved down to the matrix dispense position.
      (d) Syringe dispenses "X" µl of conjugate at a rate of "X" µl/sec.
      (e) Pipette Z-axis is moved up to the Z-clear position.
      (f) Wait "X" seconds until reaction mixture has been absorbed by matrix.
   4. Probe post-wash.
      The probe is again washed to ensure that it is free from contamination as described in section 6 (Sample kitting).
F. RV UNLOAD (This activity occurs when resources are not in use)
   1. The following are performed simultaneously:
      (a) Process carousel is rotated so that the empty position is at the transfer station.
      (b) Transfer mechanism 0-axis is moved to process carousel.
   2. RV is grabbed with the transfer mechanism R-axis and pulled into the transfer mechanism.
   3. Transfer mechanism 0-axis is rotated so that RV is aligned with the waste container.
   4. RV is pushed into the waste container.
   5. Check incubation timer. When expires start next activity.
G. PREREAD ACTIVITY PERIOD (allotted time 378). The MEIA optics system 361 simultaneously performs the following activities in response to the scheduler 256 and the optics control system 248 as described above in more detail:
   1. The auxiliary carousel 64 positions the cartridge 68 for a reading.
   2. The gain of the PMT 374 is set.
   3. The intensity of the lamp 364 is increased from the simmer state to the burn state.
H. MATRIX WASH
   1. Auxiliary carousel is rotated so that the cartridge is at the matrix wash station.
   2. The following steps are repeated until all the buffer specified in the assay file for cartridge wash has been dispensed.
      (a) "X" µl of heated MEIA buffer are dispensed in 50µl cycles at a rate of "X" µl/sec onto the matrix.
      (b) Wait "n" seconds.
I. MUP DISPENSE
   1. Auxiliary carousel is rotated so that the cartridge is at the MUP station.
   2. 50µl of heated MUP are dispensed at a rate of "X" µl/sec onto the matrix.
   3. Wait "n" seconds.
J. READ SEQUENCE PERIOD (allotted time 376). The MEIA optics system 361 then performs the following in response to the scheduler 256 and the optics control system 248 as described above:
   1. A predetermined number of sub-reads specified in the assay file specifications (usually 8) are sensed by the PMT 374.
   2. After each sub-read except the last one, the optics system 361 pauses during the dwell time.
   3. The OSP 254 converts the digitized analog signals from the PMT 374 to normalized readings by comparing the intensity at the detector 366 to the intensity of the lamp 364 ("normalized readings").
   4. The raw reads are converted to normalized reads (light intensity hitting detector/lamp intensity) by the optics microprocessor.
   5. A rate is calculated by the System from the normalized read vs time.
   6. For quantitative assays, the rate is fitted to a calibration curve to yield a concentration result.
   7. For qualitative assays, the sample rate is compared to an index or cutoff rate to determine if the sample is positive or negative (or reactive or nonreactive).
K. CARTRIDGE UNLOAD (This activity occurs when resources are not in use)
   1. Auxiliary carousel is rotated so that cartridge is at the ejector station.
   2. Ejector is cycled to place cartridge into waste container.

Schematic reaction sequences are presented in FIGS. 26, 27 and 28 which are typical of assays that can be handled by the automated immunoassay analytical system of the invention. In FIG. 26, a T4 assay, FPIA sequence 1420, is presented wherein Step 1, T4 bound by thyroxine binding protein (TBP) 1424, is reacted with T4 displacing agent 1426 to yield TBP 1428 plus unbound T4 (1430). In step 2, the T4 (1430) is added to T4 antibody 1432 which yields a reaction product 1434 (T4 antibody-T4 complex). In Step 3, the T4 antibody-T4 complex 1434 is treated with T4 tracer (fluorescent) 1436 which yields a fluorescent polarization measurable reaction product 1438.

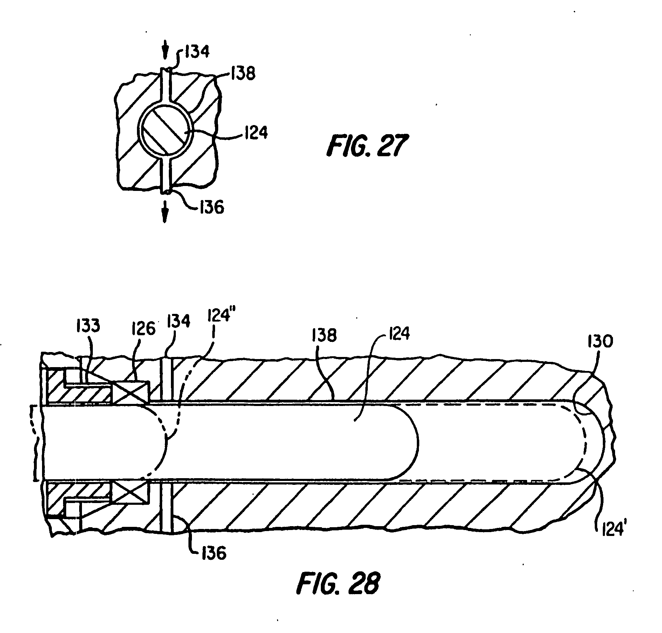

In FIG. 27, a schematic reaction sequence 1440 for a 1-step sandwich MEIA determination (ferritin) is presented. In Steps 1 and 2 an anti-ferritin alkaline phosphatase conjugate is mixed with ferritin sample 1444 and anti-ferritin microparticles 1446 to yield a ferritin antibody-antigen-antibody complex 1448. In step 3, the antibody-antigen-antibody complex 1448 is reacted with 4-methylumbelliferyl phosphate (MUP) 1450 which yields methylumbelliferone (MU) which is fluorescent. The rate of MU production is measured.

In FIG. 28, the schematic reaction sequence 1456 for a 2-step sandwich MEIA is provided for HTSH assay. Anti-hTSH specific microparticles 1458 are added to the HTSH sample 1460 which provides a reaction product HTSH antibody-antigen complex 1462. In Steps 2 through 4, the complex 1462 is combined with an anti-hTSH alkaline phosphatase 1464 yielding hTSH antibody-antigen-antibody complex 1466. In step 5, the complex 1466 is reacted with MUP 1450 to yield MU which is fluorescent. The rate of MU production is measured.

In accordance with the embodiments of the present invention, the automated immunoassay analytical system provides a method for performing a multitude of assays continuously and with random access being available to the operator. The utilization of carousel pipettor technology for kitting and pipetting operations at either the main carousel or the process carousel, depending on the scheduled test, provides scheduling flexibilities heretofore unachievable. The inventive system allows for a commonality of kitting and pipetting for either immuno precipitation or competitive immunoassay technologies utilizing a common main carousel, transfer station, first kitting and pipetting probe and process carousel as well as a second pipetting probe before separating into respective apparatus and process requirements. Also shared is the commonality of cabinetry disposal and supply materials as well as a common computer network for scheduling, testing, kitting and pipetting.

It will be seen that multiple assays can be performed with a minimum of operator input or handling on the system and the system can be utilized for other processes and assays which have not been directly discussed but will be readily apparent to one practiced in the art in view of the above invention disclosure and the claims. It will also be appreciated that although particular embodiments of the present invention have been disclosed, various changes and adaptations to the apparatus and methods can be made without departing from the teachings of the specification and scope of the invention as set out in the following claims.

## Claims

1. A method for simultaneously performing at least two assays, said assays comprising a plurality of activities, for a plurality of liquid samples on a continuous analytical system, said method comprising the steps of:
combining an aliquot of a first liquid sample with at least one reagent in a first reaction container to form a first assay reaction mixture for said first liquid sample;
combining an aliquot of a second liquid sample with at least one reagent in a second reaction container to form a second assay reaction mixture for said second liquid sample;
incubating said first and said second assay reaction mixtures at least one time;
performing activities associated with each assay other than said combining and said incubating on the first and second assay reaction mixtures to complete each assay, said other activities including analyzing the incubated assay reaction mixtures; and
scheduling the steps of said combining, said incubating, and said performing activities other than said combining and said incubating associated with each of the assays according to a predetermined protocol, said protocol specifying:
(a) what activities are to be performed for a given assay;
(b) an order in which said activities of (a) are to be performed;
(c) at least one incubation period between said activities of (a), said at least one incubation period comprising a nominal period of time for the performance of an incubating step between activities of (a) and a specified window of time to be added to or subtracted from the nominal incubation period for varying the duration of the nominal period between activities of (a) to optimize performance;
(d) how said activities of (a) are to be performed; and
(e) duration of said activities of (a).

2. A method according to claim 1, wherein the step of performing activities other than said combining and said incubating include those for a homogeneous assay.

3. A method according to claim 1, wherein the step of performing activities other than said combining and said incubating include those for a heterogeneous assay.

4. A method according to claim 1, wherein the step of performing activities other than said combining and said incubating include those for an immunoassay.

5. A method according to claim 1, wherein the step of performing activities other thar said combining and said incubating include those for two immunoassays.

6. A method according to claim 5, wherein the two immunoassays are the microparticle enzyme immunoassay and the fluorescent polarization immunoassay.

7. A method according to claim 1, wherein the step of scheduling according to the predetermined protocol comprises the step of using any one activity of (a) to initiate one or more incubating steps.

8. A method according to claim 1, wherein the step of scheduling according to the predetermined protocol comprises the step of following any one incubating step with only one activity of (a).

9. A method according to claim 1, wherein the step of scheduling according to the predetermined protocol comprises the step of completing said assay only with an activity of (a) preceded by an incubating step.

10. A method according to claim 9, wherein the incubating step is preceded by at least one activity of (a).

11. A method according to claim 10, wherein a second incubating step must immediately precede the activity of (a) preceding the first-mentioned incubating step.

12. A method according to claim 1, wherein the step of scheduling according to the predetermined protocol comprises the step of performing an activity of (a) during the period of an incubating step.

13. A method according to claim 1, wherein the step of scheduling according to the predetermined protocol comprises the step of sequencing the activities of (a) and incubating steps associated with each of the assays in a specific order.

14. A method according to claim 1, wherein any one of the activities includes events and the step of scheduling according to the predetermined protocol comprises the step of sequencing the events in a specific order.

15. A method according to claim 14, wherein the step of sequencing the events comprises the step of setting a nominal period of time for certain events.

16. A method according to claim 1, wherein the reaction containers are part of a reaction vessel and the vessels are transferred to a processing workstation.

17. A method according to claim 16, wherein the step of scheduling according to the predetermined protocol comprises the step of sequencing the transfer of vessels in a predetermined order.

18. A method according to claim 17, further comprising the step of interrupting the predetermined order of sequencing the transfer of vessels and scheduling a priority transfer of vessels for processing through the assays after completing assays in progress.

## Patentansprüche

1. Ein Verfahren zum gleichzeitigen Durchführen von mindestens zwei Assays, wobei die Assays eine Vielzahl von Aktivitäten umfassen, für eine Vielzahl von Flüssigproben in einem kontinuierlichen analytischen System, wobei das Verfahren folgende Schritte umfasst:
- Kombinieren einer Aliquote einer ersten Flüssigprobe mit mindestens einem Reagens in einem ersten Reaktionsbehälter, um eine erste Assay-Reaktionsmischung für die erste Flüssigprobe zu bilden;
- Kombinieren einer Aliquote einer zweiten Flüssigprobe mit mindestens einem Reagens in einem zweiten Reaktionsbehälter, um eine zweite Assay-Reaktionsmischung für die zweite Flüssigprobe zu bilden;
- zumindest einmaliges Inkubieren der ersten und der zweiten Assay-Reaktionsmischung;
- Durchführen von Aktivitäten im Zusammenhang mit jedem Assay außer dem Kombinieren und dem Inkubieren der ersten und der zweiten Assay-Reaktionsmischung, um jeden Assay abzuschließen, wobei die anderen Aktivitäten das Analysieren der inkubierten Assay-Reaktionsmischungen einschießen; und
- Planen der Schritte des Kombinierens, des Inkubierens und des Durchführens von anderen Aktivitäten als dem Kombinieren und dem Inkubieren im Zusammenhang mit jedem der Assays nach einem vordefinierten Protokoll, wobei das Protokoll Folgendes angibt:
(a) welche Aktivitäten für einen bestimmten Assay durchzuführen sind,
(b) eine Reihenfolge, in der die Aktivitäten von (a) durchzuführen sind,
(c) mindestens einen Inkubationszeitraum zwischen den Aktivitäten von (a), wobei der mindestens eine Inkubationszeitraum einen nominellen Zeitraum für die Ausführung eines Inkubationsschritts zwischen den Aktivitäten von (a) und ein angegebenes Zeitfenster, das zum nominellen Inkubationszeitraum zu addieren oder davon zu subtrahieren ist, zum Variieren der Dauer des nominellen Zeitraums zwischen den Aktivitäten von (a), um die Leistung zu optimieren, umfasst;
(d) wie die Aktivitäten von (a) durchzuführen sind und
(e) die Dauer der Aktivitäten von (a).

2. Ein Verfahren gemäß Anspruch 1, worin der Schritt der Durchführung von anderen Aktivitäten als dem Kombinieren und dem Inkubieren diejenigen für einen homogenen Assay einschließt.

3. Ein Verfahren gemäß Anspruch 1, worin der Schritt der Durchführung von anderen Aktivitäten als dem Kombinieren und dem Inkubieren diejenigen für einen heterogenen Assay einschließt.

4. Ein Verfahren gemäß Anspruch 1, worin der Schritt der Durchführung von anderen Aktivitäten als dem Kombinieren und dem Inkubieren diejenigen für einen Immunoassays einschließt.

5. Ein Verfahren gemäß Anspruch 1, worin der Schritt der Durchführung von anderen Aktivitäten als dem Kombinieren und dem Inkubieren diejenigen für zwei Immunoassays einschließt.

6. Ein Verfahren gemäß Anspruch 5, worin die beiden Immunoassays der Mikropartikel-Enzym-Immunoassay und der Fluoreszenz-Polarisation-Immunoassay sind.

7. Ein Verfahren gemäß Anspruch 1, worin der Schritt der Planung nach dem vordefinierten Protokoll den Schritt der Anwendung einer beliebigen Aktivität von (a) zur Einleitung eines oder mehrerer Inkubationsschritts(e) umfasst.

8. Ein Verfahren gemäß Anspruch 1, worin der Schritt der Planung nach dem vordefinierten Protokoll den Schritt der Bereitstellung einer einzigen Aktivität von (a) nach einem beliebigen Inkubationsschritt umfasst.

9. Ein Verfahren gemäß Anspruch 1, worin der Schritt der Planung nach dem vordefinierten Protokoll den Schritt des Abschließens des Assays mit einer einzigen Aktivität von (a), der ein Inkubationsschritt vorausgeht, umfasst.

10. Ein Verfahren gemäß Anspruch 9, worin dem Inkubationsschritt mindestens eine Aktivität von (a) vorausgeht.

11. Ein Verfahren gemäß Anspruch 10, worin ein zweiter Inkubationsschritt der Aktivität von (a), die dem zuerst erwähnten Inkubationsschritt vorausgeht, direkt vorausgehen muss.

12. Ein Verfahren gemäß Anspruch 1, worin der Schritt der Planung nach dem vordefinierten Protokoll den Schritt der Durchführung einer Aktivität von (a) während des Zeitraums eines Inkubationsschritts umfasst.

13. Ein Verfahren gemäß Anspruch 1, worin der Schritt der Planung nach dem vordefinierten Protokoll den Schritt der Sequenzierung der Aktivitäten von (a) und der Inkubationsschritte im Zusammenhang mit jedem der Assays in einer bestimmten Reihenfolge umfasst.

14. Ein Verfahren gemäß Anspruch 1, worin jede der Aktivitäten Ereignisse einschließt und der Schritt der Planung nach dem vordefinierten Protokoll den Schritt der Sequenzierung der Ereignisse in einer bestimmten Reihenfolge umfasst.

15. Ein Verfahren gemäß Anspruch 14, worin der Schritt der Sequenzierung der Ereignisse den Schritt der Festsetzung eines nominellen Zeitraums für bestimmte Ereignisse umfasst.

16. Ein Verfahren gemäß Anspruch 1, worin die Reaktionsbehälter Teil eines Reaktionsgefäßes sind und die Gefäße in eine Verarbeitungsstation überführt werden.

17. Ein Verfahren gemäß Anspruch 16, worin der Schritt der Planung nach dem vordefinierten Protokoll den Schritt der Sequenzierung der Überführung von Gefäßen in einer vordefinierten Reihenfolge umfasst.

18. Ein Verfahren gemäß Anspruch 17, das weiter den Schritt der Unterbrechung der vordefinierten Reihenfolge der Sequenzierung der Überführung von Gefäßen und die Planung einer Prioritäts-Überführung von Gefäßen für die Verarbeitung durch die Assays nach Abschluss der laufenden Assays umfasst.

## Revendications

1. Procédé pour réaliser simultanément au moins deux dosages, lesdits dosages comprenant une pluralité d'activités, pour une pluralité d'échantillons liquides sur un système analytique continu, ledit procédé comprenant les étapes de :
combinaison d'une portion aliquote d'un premier échantillon liquide avec au moins un réactif dans un premier réservoir réactionnel pour former un premier mélange réactionnel de dosage pour ledit premier échantillon liquide ;
combinaison d'une portion aliquote d'un second échantillon liquide avec au moins un réactif dans un second réservoir réactionnel pour former un second mélange réactionnel de dosage pour ledit second échantillon liquide ;
incubation desdits premier et second mélanges réactionnels de dosage au moins une fois ;
réalisation d'activités associées avec chaque dosage différentes de ladite combinaison et de ladite incubation sur les premier et second mélanges réactionnels de dosage pour réaliser chaque dosage, lesdites autres activités incluant l'analyse des mélanges réactionnels de dosage incubés ; et
planification des étapes de ladite combinaison, de ladite incubation et de ladite réalisation d'activités différentes de ladite combinaison et de ladite incubation associées avec chacun des dosages selon un protocole prédéterminé, ledit protocole spécifiant :
(a) quelles activités doivent être réalisées pour un dosage donné ;
(b) un ordre dans lequel lesdites activités de (a) doivent être réalisées ;
(c) au moins une période d'incubation entre lesdites activités de (a), ladite au moins une période d'incubation comprenant une période de temps nominale pour la réalisation d'une étape d'incubation entre les activités de (a) et une fenêtre de temps spécifiée destinée à être ajoutée à ou soustraite de la période d'incubation nominale pour modifier la durée de la période nominale entre les activités de (a) pour optimiser la réalisation ;
(d) comment lesdites activités de (a) doivent être réalisées ; et
(e) la durée desdites activités de (a).

2. Procédé selon la revendication 1, où l'étape de réalisation d'activités autres que ladite combinaison et ladite incubation inclut celles pour un dosage homogène.

3. Procédé selon la revendication 1, où l'étape de réalisation d'activités autres que ladite combinaison et ladite incubation inclut celles pour un dosage hétérogène.

4. Procédé selon la revendication 1, où l'étape de réalisation d'activités autres que ladite combinaison et ladite incubation inclut celles pour un immunodosage.

5. Procédé selon la revendication 1, où l'étape de réalisation d'activités autres que ladite combinaison et ladite incubation inclut celles pour deux immunodosages.

6. Procédé selon la revendication 5, où les deux immunodosages sont l'immunodosage enzymatique par microparticules et l'immunodosage par polarisation de fluorescence.

7. Procédé selon la revendication 1, où l'étape de planification selon le protocole prédéterminé comprend l'étape d'utilisation d'une activité de (a) quelconque pour initier une ou plusieurs étapes d'incubation.

8. Procédé selon la revendication 1, où l'étape de planification selon le protocole prédéterminé comprend l'étape consistant à faire suivre une étape d'incubation quelconque par seulement une activité de (a).

9. Procédé selon la revendication 1, où l'étape de planification selon le protocole prédéterminé comprend l'étape d'accomplissement dudit dosage seulement avec une activité de (a) précédée par une étape d'incubation.

10. Procédé selon la revendication 9, où l'étape d'incubation est précédée par au moins une activité de (a).

11. Procédé selon la revendication 10, où une seconde étape d'incubation doit précéder immédiatement l'activité de (a) précédant l'étape d'incubation mentionnée en premier lieu.

12. Procédé selon la revendication 1, où l'étape de planification selon le protocole prédéterminé comprend l'étape de réalisation d'une activité de (a) pendant la période d'une étape d'incubation.

13. Procédé selon la revendication 1, où l'étape de planification selon le protocole prédéterminé comprend l'étape de disposition en succession des activités de (a) et d'étapes d'incubation associées avec chacun des dosages dans un ordre spécifique.

14. Procédé selon la revendication 1, où l'une quelconque des activités inclut des événements et l'étape de planification selon le protocole prédéterminé comprend l'étape de disposition en succession des événements dans un ordre spécifique.

15. Procédé selon la revendication 14, où l'étape de disposition en succession des événements comprend l'étape de fixation d'une période de temps nominale pour certains événements.

16. Procédé selon la revendication 1, où les réservoirs réactionnels font partie d'un récipient réactionnel et les récipients sont transférés à un poste de travail de traitement.

17. Procédé selon la revendication 16, où l'étape de planification selon le protocole prédéterminé comprend l'étape de disposition en succession du transfert de récipients dans un ordre prédéterminé.

18. Procédé selon la revendication 17, comprenant en outre l'étape d'interruption de l'ordre prédéterminé de disposition en succession du transfert de récipients et de planification d'un transfert de priorité de récipients pour le traitement dans les dosages après l'accomplissement de dosages en cours.
